(19) Europäisches Patentamt / European Patent Office / Office européen des brevets

(11) **EP 4 555 107 B1**

(12) **EUROPEAN PATENT SPECIFICATION**

(45) Date of publication and mention
of the grant of the patent:
**11.03.2026 Bulletin 2026/11**

(21) Application number: **23754487.9**

(22) Date of filing: **14.07.2023**

(51) International Patent Classification (IPC):
***C12Q 1/6886*** (2018.01)

(52) Cooperative Patent Classification (CPC):
**C12Q 1/6886;** C12Q 2600/106

(86) International application number:
**PCT/US2023/027750**

(87) International publication number:
**WO 2024/015561 (18.01.2024 Gazette 2024/03)**

(54) **TECHNIQUES FOR DETECTING HOMOLOGOUS RECOMBINATION DEFICIENCY (HRD)**

VERFAHREN ZUM NACHWEIS DES MANGELS AN HOMOLOGER REKOMBINATION (HRD)

TECHNIQUES DE DÉTECTION D'UNE DÉFICIENCE DE RECOMBINAISON HOMOLOGUE (HRD)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **15.07.2022 US 202263389640 P**

(43) Date of publication of application:
**21.05.2025 Bulletin 2025/21**

(73) Proprietor: **BostonGene Corporation
Waltham, MA 02453 (US)**

(72) Inventors:
• **KOTLOV, Nikita
Waltham, MA 02453 (US)**
• **GURYLEVA, Mariia
St. Petersburg, 199106 (RU)**
• **SHEVKOPLIAS, Aleksei
Yerevan, 0070 (AM)**
• **MELNIKOVA, Aleksandra
Yerevan, 0070 (AM)**
• **BAGAEV, Alexander
Waltham, MA 02453 (US)**

(74) Representative: **Mewburn Ellis LLP
Aurora Building
Counterslip
Bristol BS1 6BX (GB)**

(56) References cited:
**WO-A1-2016/135478**

• **WANG SHIXIANG ET AL: "Copy number signature analysis tool and its application in prostate cancer reveals distinct mutational processes and clinical outcomes", PLOS GENETICS, vol. 17, no. 5, 4 May 2021 (2021-05-04), pages e1009557, XP055931432, DOI: 10.1371/journal.pgen.1009557**
• **FRANCH-EXPÓSITO SEBASTIÀ ET AL: "CNApp, a tool for the quantification of copy number alterations and integrative analysis revealing clinical implications", ELIFE, vol. 9, 15 January 2020 (2020-01-15), XP093063755, Retrieved from the Internet <URL:https://cdn.elifesciences.org/articles/50267/elife-50267-v2.xml> DOI: 10.7554/eLife.50267**
• **DONGJU CHEN ET AL: "GSA: an independent development algorithm for calling copy number and detecting homologous recombination deficiency (HRD) from target capture sequencing", BMC BIOINFORMATICS, BIOMED CENTRAL LTD, LONDON, UK, vol. 22, no. 1, 23 November 2021 (2021-11-23), pages 1 - 19, XP021299178, DOI: 10.1186/S12859-021-04487-9**
• **AHMEDIBRAHIMSAMIR KHALIL ET AL: "Hierarchical discovery of large-scale and focal copy number alterations in low-coverage cancer genomes", BMC BIOINFORMATICS, BIOMED CENTRAL LTD, LONDON, UK, vol. 21, no. 1, 16 April 2020 (2020-04-16), pages 1 - 22, XP021275370, DOI: 10.1186/S12859-020-3480-3**

• THORSSON VÉSTEINN ET AL: "The Immune Landscape of Cancer", IMMUNITY, CELL PRESS, AMSTERDAM, NL, vol. 48, no. 4, 5 April 2018 (2018-04-05), pages 812, XP085382263, ISSN: 1074-7613, DOI: 10.1016/J.IMMUNI.2018.03.023

**Description**

RELATED APPLICATIONS

This application claims the benefit under 35 U.S.C. §119(e) of the filing date of U.S. provisional application serial number 63/389,640, filed July 15, 2022, entitled "TECHNIQUES FOR DETECTING HOMOLOGOUS RECOMBINATION DEFICIENCY (HRD)", Attorney Docket No. B1462.70047US00.

BACKGROUND

Homologous recombination is a mechanism by which nucleotide sequences are exchanged between two similar or identical molecules of deoxyribose nucleic acid (DNA). Homologous recombination plays an important role in maintaining the integrity of the genome by repairing DNA double-stranded breaks. The repair is mediated by many different proteins, including those in the Poly ADP-ribose polymerase (PARP) family. For example, PARP-1 plays an important role in detecting and signaling DNA damage and facilitating repair.

Homologous recombination deficiency (HRD) is a phenotype that is characterized by a defective double-stranded DNA repair mechanism due to alterations in the homologous recombination (HR) pathway. Absent the ability to accurately repair deleterious genetic mutations, cells are at an increased risk of becoming cancerous.

WO 2016/135478 A1 relates to chromosomal instability, and in particular to methods for scoring chromosomal instabilities.

Wang et al: "Copy number signature analysis tool and its application in prostate cancer reveals distinct mutational processes and clinical outcomes", PLOS GENETICS, vol. 17, no. 5, 4 May 2021, discusses a bioinformatics tool, "sigminer", that has been constructed for copy number signature extraction, analysis and visualization.

SUMMARY

The invention is defined by the claims.

Some aspects provide for a method for determining whether a biological sample obtained from a subject includes cells having homologous recombination deficiency (HRD), the method comprising: using at least one computer hardware processor to perform: obtaining data about segments of the subject's genome, the data including, for each of at least some of the segments, a respective copy number and a respective length; identifying a first subset of the segments, the first subset including segments associated with at least one chromosome arm of the genome, wherein each of the segments of the first subset has a common copy number, wherein the segments include a first segment, and wherein identifying the first subset of the segments comprises, for the first segment: determining whether a length of the first segment is greater than or equal to a first threshold length, wherein the first threshold length is between 30% and 70% of a length of the chromosome arm associated with the first segment; determining whether a copy number of the first segment equals ploidy of tumor cells in the biological sample; and including the first segment in the first subset when it is determined that (i) the length of the first segment is greater than or equal to the first threshold length, and that (ii) the copy number of the first segment equals the ploidy of tumor cells in the biological sample; identifying a second subset of the segments, wherein each of the segments of the second subset has (i) a respective copy number different from the common copy number and (ii) a respective length that is between 1 megabase and 30% of a length of the chromosome arm associated with the segment; determining a proportion of a number of segments in the second subset to a number of chromosome arms of the at least one chromosome arm associated with the segments included in the first subset; determining, based on the determined proportion, whether the biological sample includes cells having HRD, wherein determining, based on the determined proportion, whether the biological sample includes cells having HRD comprises processing the determined proportion with a statistical model trained to predict an output indicating whether the biological sample includes cells having HRD. The method may may further comprise identifying a therapy for the subject based on a result of determining whether the biological sample includes cells having HRD.

Some aspects provide for a system, comprising: at least one computer hardware processor; and at least one non-transitory computer-readable storage medium storing processor-executable instructions that, when executed by the at least one computer hardware processor, cause the at least one computer hardware processor to perform a method for determining whether a biological sample obtained from a subject includes cells having homologous recombination deficiency (HRD), the method comprising: obtaining data about segments of the subject's genome, the data including, for each of at least some of the segments, a respective copy number and a respective length; identifying a first subset of the segments, the first subset including segments associated with at least one chromosome arm of the genome, wherein each of the segments of the first subset has a common copy number, wherein the segments include a first segment, and wherein identifying the first subset of the segments comprises, for the first segment: determining whether a length of the first segment is greater than or equal to a first threshold length, wherein the first threshold length is between 30% and 70% of a length of the chromosome arm associated with the first segment; determining whether a copy number of the first segment equals ploidy of tumor cells in the biological sample; and including the first segment in the first subset when it is determined that (i) the length of the first segment is greater than or equal to the first threshold length, and that (ii) the copy number of the first segment equals the ploidy of tumor cells in the biological sample; identifying a second subset of the segments, wherein each of the segments of the second subset has (i) a respective copy number different from the common copy number and (ii) a respective length that that is between 1 megabase and 30% of a length of the chromosome arm associated with the segment; determining a proportion of a number of segments in the second subset to a number of chromosome arms of the at least one chromosome arm associated with the segments included in the first subset; determining, based on the determined proportion, whether the biological sample includes cells having HRD, wherein determining, based on the determined proportion, whether the biological sample includes cells having HRD comprises processing the determined proportion with a statistical model trained to predict an output indicating whether the biological sample includes cells having HRD. The method may further comprise identifying a therapy for the subject based on a result of determining whether the biological sample includes cells having HRD.

Some aspects provide for at least one non-transitory computer-readable storage medium storing processor-executable instructions that, when executed by at least one computer hardware processor, cause the at least one computer hardware processor to perform a method for determining whether a biological sample obtained from a subject includes cells having homologous recombination deficiency (HRD), the method comprising: obtaining data about segments of the subject's genome, the data including, for each of at least some of the segments, a respective copy number and a respective length; identifying a first subset of the segments, the first subset including segments associated with at least one chromosome arm of the genome, wherein each of the segments of the first subset has a common copy number, wherein the segments include a first segment, and wherein identifying the first subset of the segments comprises, for the first segment: determining whether a length of the first segment is greater than or equal to a first threshold length, wherein the first threshold length is between 30% and 70% of a length of the chromosome arm associated with the first segment; determining whether a copy number of the first segment equals ploidy of tumor cells in the biological sample; and including the first segment in the first subset when it is determined that (i) the length of the first segment is greater than or equal to the first threshold length, and that (ii) the copy number of the first segment equals the ploidy of tumor cells in the biological sample; identifying a second subset of the segments, wherein each of the segments of the second subset has (i) a respective copy number different from the common copy number and (ii) a respective length that that is between 1 megabase and 30% of a length of the chromosome arm associated with the segment; determining a proportion of a number of segments in the second subset to a number of chromosome arms of the at least one chromosome arm associated with the segments included in the first subset; determining, based on the determined proportion, whether the biological sample includes cells having HRD, wherein determining, based on the determined proportion, whether the biological sample includes cells having HRD comprises processing the determined proportion with a statistical model trained to predict an output indicating whether the biological sample includes cells having HRD. The method may further comprise identifying a therapy for the subject based on a result of determining whether the biological sample includes cells having HRD.

Some aspects provide for a method for identifying whether a subject is likely to be sensitive to treatment with PolyADPribose polymerase inhibitor (PARPi) therapy or a platinum-based chemotherapy, the method comprising: using at least one computer hardware processor to perform: obtaining data about segments of the subject's genome, the data including, for each of at least some of the segments, a respective copy number and a respective length; identifying a first subset of the segments, the first subset including segments associated with at least one chromosome arm of the genome, wherein each of the segments of the first subset has a common copy number, wherein the segments include a first segment, and wherein identifying the first subset of the segments comprises, for the first segment: determining whether a length of the first segment is greater than or equal to a first threshold length, wherein the first threshold length is between 30% and 70% of a length of the chromosome arm associated with the first segment; determining whether a copy number of the first segment equals ploidy of tumor cells in the biological sample; and including the first segment in the first subset when it is determined that (i) the length of the first segment is greater than or equal to the first threshold length, and that (ii) the copy number of the first segment equals the ploidy of tumor cells in the biological sample; identifying a second subset of the segments, wherein each of the segments of the second subset has (i) a respective copy number different from the common copy number and (ii) a respective length that that is between 1 megabase and 30% of a length of the chromosome arm associated with the segment; determining a proportion of a number of segments in the second subset to a number of chromosome arms of the at least one chromosome arm associated with the segments included in the first subset; determining, based on the determined proportion, whether the biological sample includes cells having HRD, wherein determining, based on the determined proportion, whether the biological sample includes cells having HRD comprises processing the determined proportion with a statistical model trained to predict an output indicating whether the biological sample includes cells having HRD; and when it is determined that the biological sample includes cells having HRD, determining that the subject is likely to be sensitive to treatment with the PARPi therapy or the platinum-based chemotherapy.

Embodiments of any of the above aspects may have one or more of the following optional features.

In some embodiments, identifying the therapy for the subject comprises: when the output indicates that the biological sample includes cells having HRD, identifying a polyADPribose polymerase inhibitor (PARPi) therapy or a platinum-based chemotherapy for the subject.

In some embodiments, the first threshold length is at least 40% of a length of a chromosome arm of the at least one chromosome arm of the genome.

In some embodiments, the segments include one or more other segments, and identifying the first subset of the segments further comprises: determining whether a sum of the length of the first segment and lengths of the one or more other segments is greater than or equal to a second threshold length; and including the first segment and the one or more other segments in the first subset when it is determined that the sum is greater than or equal to the second threshold length.

In some embodiments, the second threshold length is at least 50% of a length of a chromosome arm of the at least one chromosome arm.

In some embodiments, the one or more other segments consist of three or fewer segments.

In some embodiments, identifying the second subset of the segments comprises, for a particular segment: determining whether a copy number of the particular segment differs from the common copy number; determining whether a length of the particular segment satisfies the predetermined length criterion; and including the particular segment in the second subset of segments when it is determined that (i) the copy number of the particular segment differs from the common copy number and (ii) that the length of the particular segment satisfies the predetermined length criterion.

In some embodiments, determining whether the length of the particular segment satisfies the predetermined length criterion comprises determining whether the length is in a predetermined range.

In some embodiments, the predetermined length criterion for each particular segment of the second subset depends on a length of a chromosome arm associated with the particular segment.

In some embodiments, the output indicating whether the biological sample includes cells having HRD is a probability that the biological sample includes cells having HRD.

In some embodiments, the output indicating whether the biological sample includes cells having HRD is a score indicative of whether the biological sample includes cells having HRD. In some embodiments, a score greater than or equal to a threshold value indicates that the biological sample includes cells having HRD.

In some embodiments, the statistical model is a generalized linear model. In some embodiments, the statistical model is a regression model. In some embodiments, the generalized linear model is a logistic regression model.

In some embodiments, the statistical model is trained to predict the output using training data comprising, for each of a plurality of subjects with a known HRD status, data about segments of the subject's genome.

In some embodiments, the subject has, is suspected of having, or is at risk of having cancer of a first cancer type, processing the determined proportion with the statistical model comprises processing the determined proportion with a first statistical model trained to predict whether cells of the first cancer type have HRD.

In some embodiments, the method further comprises: using a second statistical model different from the first statistical model to determine whether a second biological sample obtained from a second subject includes cells having HRD, wherein the second subject has, is suspected of having, or is at risk of having cancer of a second cancer type different from the first cancer type, and wherein the second statistical model is trained to predict whether cells of the second cancer type have HRD.

In some embodiments, the first cancer type is breast cancer and wherein the second cancer type is ovarian cancer.

In some embodiments, processing the determined proportion with the statistical model comprises processing the determined proportion with a statistical model trained to predict whether breast cancer cells have HRD.

In some embodiments, processing the determined proportion with the statistical model comprises processing the determined proportion with a statistical model trained to predict whether ovarian cancer cells have HRD.

Some embodiments further comprise: determining ploidy of tumor cells in the biological sample, wherein processing the determined proportion with the statistical model further comprises processing the determined ploidy with the statistical model to obtain the output.

In some embodiments, the subject has, is suspected of having, or is at risk of having breast cancer.

Some embodiments further comprise: obtaining sequencing data, the sequencing data having been previously obtained by sequencing the biological sample from the subject; and processing the sequencing data to obtain the data about the segments of the subject's genome.

In some embodiments, obtaining the sequencing data comprises obtaining sequencing data for at least a chromosome of the subject's genome.

In some embodiments, obtaining the sequencing data comprises obtaining sequencing data for the subject's whole genome.

The at least some of the segments comprise segments having (i) respective copy numbers different from the common copy number and (ii) respective lengths that satisfy predetermined length criteria, and the second subset consists of the segments of the at least some of the segments having (i) the respective copy numbers different from the common copy number and (ii) the respective lengths that satisfy the predetermined length criteria.

In some embodiments, the subject has, is suspected of having or is at risk of having breast cancer, ovarian cancer, pancreatic cancer, prostate cancer, bladder cancer, colorectal cancer, or lymphoma.

BRIEF DESCRIPTION OF DRAWINGS

The accompanying drawings are not intended to be drawn to scale. In the drawings, each identical or nearly identical component that is illustrated in various figures is represented by a like numeral. For purposes of clarity, not every component may be labeled in every drawing. In the drawings:

FIG. 1A is a diagram depicting an illustrative technique 100 for identifying a therapy for a subject based on determining whether cells in a biological sample obtained from the subject have homologous recombination deficiency (HRD), according to some embodiments of the technology described herein.

FIG. 1B is a diagram depicting an illustrative technique 120 for determining whether cells in a biological sample obtained from a subject have HRD, according to some embodiments of the technology described herein.

FIG. 2 is a block diagram of an example system 200 for determining whether cells in a biological sample obtained from a subject have HRD and for identifying a therapy for the subject, according to some embodiments of the technology described herein.

FIG. 3A is a flowchart of an illustrative process 300 for determining whether a biological sample obtained from a subject includes cells having HRD, according to some embodiments of the technology described herein.

FIG. 3B is a flowchart of an illustrative process 340 for identifying a first subset of segments of a subject's genome, according to some embodiments of the technology described herein.

FIG. 3C is a flowchart of an illustrative process 360 for identifying a second subset of segments of a subject's genome, according to some embodiments of the technology described herein.

FIG. 4 is an example of determining a proportion of a number of segments of the subject's genome to a number of chromosome arms, according to some embodiments of the technology described herein.

FIG. 5 shows an example of chromosome segments that are used for determining a proportion of a number of segments to a number of chromosome arms, according to some embodiments of the technology described herein.

FIG. 6A is a diagram depicting an example process for training a generalized linear model to predict whether breast cancer samples include cells having HRD, according to some embodiments of the technology described herein.

FIG. 6B is a diagram depicting example, candidate predictors of HRD, according to some embodiments of the technology described herein.

FIGs. 7A-7C are violin plots showing that the proportion of a number of segments to a number of chromosome arms, determined according to some embodiments of the technology described herein, outperformed genome-wide loss of heterozygosity (gwLOH) and ploidy as predictors of HRD.

FIG. 8 shows that the proportion of a number of segments to a number of chromosome arms, determined according to embodiments of the technology described herein, outperformed gwLOH and ploidy as predictors of HRD.

FIG. 9A is a coefficient plot showing results of a regression analysis performed using, as predictors of HRD, aneuploidy, gwLOH, and the proportion of the number of segments to the number of chromosome arms, determined according to embodiments of technology described herein.

FIG. 9B shows that sample ploidy influences the proportion of the number of segments to the number chromosome arms determined according to embodiments of the technology described herein.

FIGs. 10A-10B show results of determining whether breast cancer samples include cells having HRD, according to some embodiments of the technology described herein.

FIGs. 11A-11B show boxplots showing that determining an HRD status according to embodiments of the technology described herein outperforms determining an HRD status using existing genomic instability score (GIS) techniques.

FIG. 12 is a receiver operating characteristic (ROC) curve showing that determining an HRD status according to embodiments of the technology described herein outperforms determining an HRD status using existing GIS techniques.

FIG. 13 shows a Pearson correlation between HRD scores, determined according to embodiments of the technology described herein, and GIS scores, determined according to the existing GIS techniques.

FIG. 14 shows that, despite the absence of mutated homologous recombination genes, many subjects having breast cancer were determined to be HRD-positive according to embodiments of the technology described herein, suggesting that these subjects may respond well to certain therapies.

FIGs. 15A-15B show results of determining whether ovarian cancer samples include cells having HRD, according to some embodiments of the technology described herein.

FIG. 16A is a plot confirming that subjects who responded well to platinum-based compounds were determined to be HRD-positive according to embodiments of the technology described herein.

FIG. 16B is a plot confirming that the subjects who had a longer overall survival rate when treated with platinum-based compounds were determined to be HRD-positive according to embodiments of the technology described herein.

FIG. 17 is a schematic diagram of an illustrative computing device with which aspects described herein may be implemented.

DETAILED DESCRIPTION

The inventors have developed techniques for determining whether a biological sample obtained from a subject includes cells having homologous recombination deficiency (HRD). Determining whether a biological sample includes cells having HRD includes processing data about segments of the subject's genome using a statistical model. The data about the segments of the subject's genome includes a proportion of a number of segments of the subjects genome to a number of chromosome arms. The proportion is the proportion of a number of segments in a second subset of segments to number of chromosome arms associated with segments in a first subset of segments. Each of the segments in the first subset is associated with a respective chromosome arm and has a common copy number. Each of the segments in the second subset has a respective copy number different from the common copy number of segments in the first subset and a respective length that satisfies a predetermined length criterion (e.g., a segment length within a predetermined range). The proportion of the number of segments to the number of chromosome arms is used to determine whether a biological sample includes cells having HRD. The proportion is provided as input to a statistical model trained to predict whether a biological sample includes cells having HRD.

In some embodiments, the techniques described herein are used to identify a therapy to be administered to a subject. Subjects having HRD have been shown to be responsive to certain therapies such as platinum-based therapeutic agents and poly ADP ribose polymerase (PARP) inhibitors, for example. Such therapies, in some embodiments, may be identified for a subject determined to have cells having HRD. For example, such therapies may be recommended to a healthcare provider, who may then administer the identified therapy or therapies to the subject.

As described above, HRD refers to a cellular condition where the ability to repair DNA damage is impaired. In normal cells, homologous recombination repairs DNA double-stranded breaks by using an undamaged copy of the DNA molecule as a template to accurately restore the broken strand. This is crucial in maintaining genomic stability and integrity. In cells with HRD, this repair process is compromised, leading to an increased susceptibility to genetic alterations and an accumulation of DNA damage, which in turn leads to an increased susceptibility to cancer. In particular, HRD is commonly associated with breast, ovarian, pancreatic, colorectal, bladder, and prostate cancer, among others. The techniques described herein may be applied to detecting HRD in samples obtained from patients having been diagnosed as having one of these

The inventors have appreciated that it is challenging to detect HRD in cells due to the various factors that cause HRD. First, HRD can be caused by mutations of genes that play a role in homologous recombination. For example, BRCA1, BRCA2, PALB2, and BARD1 are genes that are important to homologous recombination. Mutations of these genes impair a cell's ability to perform the functions necessary for repairing DNA double-stranded breaks through homologous recombination. When HRD is caused by genetic mutations, genetic testing (e.g., DNA sequencing, gene panel testing, etc.) may be used to help identify the presence of such mutations for determining whether a subject has HRD. HRD can also be caused by epigenetic modifications that affect the expression of genes involved in HRD. Epigenetic modifications are chemical modifications that occur on DNA or proteins associated with DNA, without changing the underlying DNA itself. Detecting epigenetic modifications can be challenging due to their dynamic and complex nature. Even if detected, it is challenging to determine how the epigenetic modifications, alone or in combination, impact the expression of genes involved in homologous recombination. In addition to genetic mutations and epigenetic modifications, other factors such as post-translational modifications and cross-pathway interactions further affect HRD status. Due to the multitude and variety of factors that cause HRD, it is challenging to accurately and reliably detect HRD.

Existing techniques for detecting HRD include determining values for multiple genomic factors, which are used to determine whether cells have HRD. Such factors include, for example, loss of heterozygosity (LOH), telomeric allelic imbalance (TAI), and large-scale state transitions (LST). LOH refers to a type of genetic abnormality in diploid organisms in which one copy of an entire gene and its surrounding chromosomal region are lost. TAI refers to a number of regions with allelic imbalance. LST refers to chromosome breaks in adjacent segments of DNA at least 10 Mb. LST is used, by existing techniques, to represent genomic instability, which is an indicator of HRD.

The inventors have recognized several challenges associated with detecting HRD using existing techniques. First, due to the strict and rigid criteria used to identify LSTs according to the existing techniques, the resulting LST score does not accurately represent genomic instability, and thus is not an accurate indicator of HRD. As described above, LSTs are defined as breaks in adjacent segments of DNA of at least 10 Mb. This criterion excludes breaks between segments which are smaller than 10 Mb. However, such segments may greatly contribute to genomic instability. For example, such segments may represent copy number alterations (CNAs) of any size, including less than 10 Mb, that impact one or more genes and regulatory elements along the genome. By excluding information representing these segments (e.g., excluding breaks between these segments), the LST score does not accurately represent instability of the genome, and thus is not an accurate indicator of HRD.

Second, because the existing techniques require the determination of multiple different genomic factors, they are computationally complex and inefficient. As described above, some existing techniques require the determination of LOH, TAI, and LST. Determining each factor requires processing genomic data, the size of which may be on the order of millions to billions of base pairs. Such processing is time consuming and computationally complex.

Accordingly, the inventors have developed techniques for determining whether a biological sample includes cells having HRD that address the above-described challenges of existing techniques. The techniques developed by the inventors include processing data about segments of a subject's genome to identify: (a) a first subset of the segments, each of which has a common copy number and is associated with a respective chromosome arm, and (b) a second subset of the segments, each of which has (i) a respective copy number different from the common copy number and (ii) a respective length that satisfies a predetermined length criterion. For example, a segment in the second subset may represent a CNA having a length that satisfies a predetermined length criterion, such as a length included in a predetermined range. The predetermined length criterion may be based on the length of the chromosome arm associated with the particular segment, thereby providing for a flexible threshold that accounts for the variable lengths of different chromosome arms, as well as the variable lengths of the segments relative to the chromosome arms with which they are associated.

The identified subsets of segments are used to determine a proportion of a number of segments included in the second subset to a number of chromosome arms associated with segments included in the first subset. The proportion is used to determine whether a biological sample includes cells having HRD. A statistical model is used to process the proportion to obtain an output indicating whether the biological sample includes cells having HRD.

The techniques developed by the inventors improve upon existing techniques for detecting HRD because they are more accurate and less computationally complex than the existing techniques. First, the techniques developed by the inventors are an improvement to conventional techniques for determining an HRD status for a subject because they more accurately capture genomic instability than the measures relied upon by the existing techniques. In particular, by using criteria to identify segments of the subject's genome (e.g., criteria that account for a range of segment lengths, criteria that accounts for the length of the chromosome arms with which the segments are associated, etc.), the techniques developed by the inventors capture information about segments (e.g., CNAs) of varying length, including segments with lengths less than 10 megabases (Mb), which contribute to genomic instability. For example, the techniques developed by the inventors account for segments (e.g., the second subset of segments), each of which has a length in a predetermined range of lengths such as a length between 3 Mb and 10% of the length of the chromosome arm. Because they are based upon a more accurate measure of genomic instability, the techniques developed by the inventors can be used to more accurately predict HRD. Furthermore, the more accurate prediction of HRD can be used to more accurately predict whether a subject will respond positively to certain therapies such as, for example, platinum-based therapeutic agent and PARP inhibitors. Accordingly, the techniques developed by the inventors are an improvement to conventional techniques for detecting HRD and for identifying a therapy for a subject based on an HRD status determined for the subject.

Second, the techniques developed by the inventors are an improvement to computer technology because they are less computationally complex and more efficient than conventional techniques for determining whether a biological sample includes cells having HRD. In particular, because the techniques developed by inventors can be used to detect HRD using a single genomic factor (e.g., the determined proportion) the techniques developed by the inventors are less computationally complex and more efficient than the conventional techniques, which rely on the computation of multiple different genomic factors.

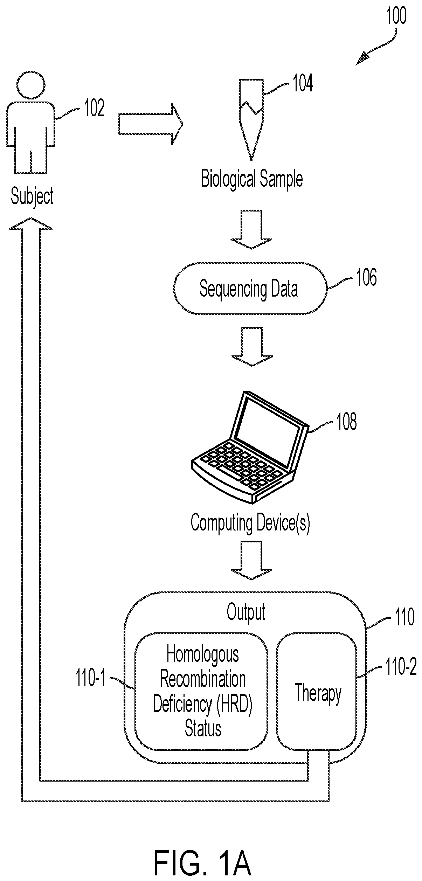

Accordingly, some embodiments provide for computer-implemented techniques for determining whether a biological sample obtained from a subject includes cells having homologous recombination deficiency (HRD). The techniques include: (a) obtaining data about segments (e.g., by processing sequencing data, by using segmentation software, etc.) of the subject's genome (e.g., some or all of the genome), the data including, for each of at least some of the segments, a respective copy number and a respective length; (b) identifying a first subset of the segments, the first subset including segments associated with (e.g., map to) at least one chromosome arm of the genome, wherein each of the segments of the first subset has a common copy number (e.g., the same copy number); (c) identifying a second subset of the segments, wherein each of the segments of the second subset has (i) a respective copy number different from the common copy number and (ii) a respective length that satisfies a predetermined length criterion (e.g., the respective length falls within a predetermined range of lengths); (d) determining a proportion of a number of segments in the second subset to a number of chromosome arms of the at least one chromosome arm associated with the segments included in the first subset; (e) determining, based on the determined proportion (e.g., by processing the determined proportion using a statistical model), whether the biological sample includes cells having HRD; and identifying a therapy (e.g., therapy 110-2 shown in FIGs. 1A-1B) for the subject based on a result of determining whether the biological sample includes cells having HRD (e.g., HRD status 110-1 shown in FIGs. 1A-1B).

Identifying the therapy for the subject may include identifying a polyADPribose polymerase inhibitor (PARPi) therapy (e.g., veliparib, fluzoparib, talazoparib, olaparib, rucaparib, niraparib, etc.) or a platinum-based chemotherapy (e.g., cisplatin, carboplatin, oxaliplatin, etc.) for the subject when the output indicates that the biological sample includes cells having HRD.

The segments include a first segment, and identifying the first subset of the segments includes, for the first segment: (a) determining whether a length of the first segment is greater than or equal to a first threshold length (e.g., between 20% and 100% of the length of the chromosome arm, between 25% and 75% of the length of the chromosome arm, between 30% and 70% of the length of the chromosome arm, between 35% and 65% of the length of the chromosome arm, or any other suitable length, etc.); (b) determining whether a copy number of the first segment equals ploidy of cells in the biological sample (e.g., tumor cells) in the biological sample; and (c) including the first segment in the first subset when it is determined that (i) the length of the first segment is greater than or equal to the first threshold length and that (ii) the copy number of the first segment equals the ploidy of cells in the biological sample. For example, the first threshold length may be a length of at least 40% of the length of the chromosome arm associated with the first segment. According to the claims, the first threshold length is between 30% and 70% of a length of the chromosome arm associated with the first segment.

In some embodiments, the segments include one or more other segments (e.g., in addition to the first segment), and identifying the first subset of the segments further includes: determining whether a sum of the length of the first segment and lengths of the one or more other segments is greater than or equal to a second threshold length (e.g., between 20% and 100% of the length of the chromosome arm, between 25% and 75% of the length of the chromosome arm, between 30% and 70% of the length of the chromosome arm, between 35% and 65% of the length of the chromosome arm, or any other suitable length, etc.); and including the first segment and the one or more other segments in the first subset when it is determined that the sum is greater than or equal to the second threshold length. For example, the second threshold length may be a length of at least 50% of the length of the chromosome arm associated with the first segment and the one or more segments. The one or more segments may consist of fewer than or equal to a threshold number of segments (e.g., between 1 and 10 segments, between 2 and 6 segments, etc.). For example, the one or more segments may consist of three or fewer segments.

Identifying the second subset of the segments includes, for a particular segment: determining whether a copy number of the particular segment differs from the common copy number; determining whether a length of the particular segment satisfies the predetermined length criterion; and including the particular segment in the second subset of segments when it is determined that (i) the copy number of the particular segment differs from the common copy number and (ii) that the length of the particular segment satisfies the predetermined length criterion. In some embodiments, the predetermined length criterion for each particular segment of the second subset depends on a length of the chromosome arm associated with the particular segment. According to the claims, the predetermined length criterion is that the respective length is between 1 Mb and 30% of a length of the chromosome arm associated with the segment.

In some embodiments, determining whether the length of the particular segment satisfies the predetermined length criterion may include determining whether the length is in a predetermined range (within a predetermined range between 1 Mb and 30% of the length of the chromosome arm associated with the segment, e.g., between 2 Mb and 20% of the length of the chromosome arm associated with the segment, between 3 Mb and 10% of the length of the chromosome arm associated with the segment, 4 Mb and 5% of the length of the chromosome, etc.). For example, the predetermined range may be a range between at least 3 megabases and at least 10% of a length of a chromosome arm of the at least one chromosome arm associated with the segments included in the first subset.

Determining, based on the determined proportion, whether the biological sample includes cells having HRD includes processing the determined proportion with a statistical model trained to predict an output (e.g., a probability, a likelihood, and/or any other score which may be numeric or categorical) indicating whether the biological sample includes cells having HRD. For example, the statistical model may include a generalized linear model such as a logistic regression model. In some embodiments, the output indicating whether the biological sample includes cells having HRD is a probability or a likelihood that the biological sample includes HRD. In some embodiments, the output is a score (e.g., a numerical score) indicative of whether the biological sample includes cells having HRD. In some embodiments, when the score is greater than or equal to a threshold value, this may indicate that the biological sample includes cells having HRD.

In some embodiments, the statistical model is trained to predict the output using training data comprising, for each of a plurality of subjects with a known HRD status (e.g., HRD-positive or HRD-negative), data about segments of the subject's genome (e.g., a proportion of a number of segments to a number of chromosome arms determined for each subject). For example, the plurality of subjects may include at least 100 subjects, at least 300 subjects, at least 500 subjects, at least 700 subjects, at least 900 subjects, at least 1,000 subjects, at least 1,100 subjects, at least 1,300 subjects, at least 1,500 subjects, at least 2,000 subjects, at least 3,000 subjects, at least 5,000 subjects, at most 50,000 subjects, at most 30,000 subjects, at most 10,000 subjects, at most 8,000 subjects, at most 6,000 subjects, at most 4,000 subjects, at most 2,000 subjects, between 100 and 50,000 subjects, between 500 and 10,000 subjects, between 900 and 1,300 subjects, or any other suitable number of subjects.

In some embodiments, the subject has, is suspected of having, or is at risk of having cancer of a first cancer type (e.g., breast cancer, ovarian cancer, pancreatic cancer, colorectal cancer, prostate cancer, bladder cancer, lymphoma, etc.) and processing the determined proportion with the statistical model includes processing the proportion with a first statistical model (e.g., a first logistic regression model) trained to predict whether cells of the first cancer type have HRD. In some embodiments, the techniques further include using a second statistical model (e.g., a second logistic regression model) different from the first statistical model (e.g., trained using different training data) to determine whether a second biological sample obtained from a second subject includes cells having HRD. The second subject may have, be suspected of having, or is at risk of having cancer of a second cancer type (e.g., breast cancer, ovarian cancer, pancreatic cancer, colorectal cancer, prostate cancer, bladder cancer, lymphoma, etc.) different from the first cancer type. The second statistical model may be trained to predict whether cells of the second cancer type have HRD. For example, the first cancer type may be breast cancer and the second cancer type may be ovarian cancer.

In some embodiments, the subject has, is suspected of having, or is at risk of having breast cancer, ovarian cancer, pancreatic cancer, prostate cancer, bladder cancer, colorectal cancer, or lymphoma. In some embodiments, processing the determined proportion with the statistical model includes processing the determined proportion with a statistical model trained to predict whether breast cancer cells have HRD. In some embodiments, processing the determined proportion with the statistical model includes processing the determined proportion with a statistical model trained to predict whether ovarian cancer cells have HRD. In some embodiments, processing the determined proportion with the statistical model includes processing the determined proportion with a statistical model trained to predict whether pancreatic cancer cells have HRD. In some embodiments, processing the determined proportion with the statistical model includes processing the determined proportion with a statistical model trained to predict whether colorectal cancer cells have HRD. In some embodiments, processing the determined proportion with the statistical model includes processing the determined proportion with a statistical model trained to predict whether prostate cancer cells have HRD. In some embodiments, processing the determined proportion with the statistical model includes processing the determined proportion with a statistical model trained to predict whether bladder cancer cells have HRD. In some embodiments, processing the determined proportion with the statistical model includes processing the determined proportion with a statistical model trained to predict whether lymphoma cells have HRD.

In some embodiments, the techniques further include determining ploidy of cells (e.g., tumor cells) in the biological sample (e.g., based on sequencing data and/or segment data). Determining whether the biological sample includes cells having HRD may be based on both the determined proportion and the determined ploidy. For example, ploidy may be used to determine whether a biological sample from a subject having, suspected of having, or at risk of having breast cancer includes cells having HRD.

The techniques may further include obtaining (e.g., from a data store, from a sequencing platform, from a user, etc.) sequencing data having been previously obtained by sequencing the biological sample from the subject (e.g., using a sequencing platform); and processing the sequencing data (e.g., determining a copy number profile, deriving segment data from the copy number profile, using segmentation software configured to determine a copy number profile and derive segment data, etc.) to obtain the data about the segments of the subject's genome. Obtaining the sequencing data may include obtaining sequencing data for at least a chromosome of the subject's genome, for the subject's whole genome, or for any other suitable portion of the subject's genome, as aspects of the technology described herein are not limited in this respect.

The at least some of the segments comprise segments having (i) respective copy numbers different from the common copy number and (ii) respective lengths that satisfy predetermined length criteria (e.g., the respective length falls within a predetermined range of lengths). In some embodiments, the second subset consists of the segments of the at least some of the segments having (i) the respective copy numbers different from the common copy number and (ii) the respective lengths that satisfy the predetermined length criteria.

Following below are descriptions of various concepts related to, and embodiments of, techniques for determining whether a biological sample includes cells having HRD. It should be appreciated that various aspects described herein may be implemented in any of numerous ways, as the techniques are not limited to any particular manner of implementation. Examples of details of implementations are provided herein solely for illustrative purposes. Furthermore, the techniques disclosed herein may be used individually or in any suitable combination, as aspects of the technology described herein are not limited to the use of any particular technique or combination of techniques.

FIG. 1A is a diagram depicting an illustrative technique 100 for identifying a therapy for a subject based on determining whether cells in a biological sample obtained from the subject have homologous recombination deficiency (HRD). Technique 100 includes obtaining an output 110 indicative of a homologous recombination deficiency (HRD) status 110-1 for a biological sample 104 from subject 102 using computing device 108. The computing device 108 may be configured to process sequencing data 106 obtained from the biological sample 104 to determine whether the biological sample 104 includes cells having HRD (e.g., HRD status 110-1). Based on the determined HRD status 110-1, technique 100 further includes identifying a therapy 110-2 to be administered to the subject 102.

In some embodiments, aspects of the illustrated technique 100 may be implemented in a clinical or laboratory setting. For example, aspects of the illustrated technique 100 may be implemented on a computing device 108 that is located within a clinical or laboratory setting. In some embodiments, the computing device 108 may directly obtain sequencing data 106 from a sequencing platform co-located with the computing device 108 within the clinical or laboratory setting. For example, the computing device may be included within the sequencing platform. In some embodiments, the computing device 108 may indirectly obtain the sequencing data 106 from a sequencing platform that is located externally from or co-located with the computing device 108 within the clinical or laboratory setting. For example, computing device 108 may obtain the sequencing data via at least one communication network, such as the Internet or any other suitable communication network(s), as aspects of the technology described herein are not limited in this respect.

In some embodiments, aspects of the illustrated technique 100 may be implemented in a setting that is located externally from a clinical or laboratory setting. In this case, the computing device 108 may indirectly obtain sequencing data 106 that is generated from a sequencing platform located within or externally to a clinical or laboratory setting. For example, the sequencing data 106 may be provided to the computing device 108 via at least one communication network, such as the Internet or any other suitable communication network(s), as aspects of the technology described herein are not limited in this respect.

As shown in FIG. 1A, the technique 100 involves obtaining a biological sample 104 from a subject 102. In some embodiments of any aspect of the technology described herein, the subject has, is suspected of having, or is at risk of having cancer. In some embodiments of any aspect of the technology described herein, the subject may have, be suspected of having, or be at risk of having breast cancer, ovarian cancer, pancreatic cancer, colorectal cancer, prostate cancer, bladder cancer, lymphoma, or any other type of cancer associated with HRD, as aspects of the technology described herein are not limited in this respect. The biological sample 104 may have been obtained by performing a biopsy or by obtaining a blood sample, salivary sample, or any other suitable biological sample from the subject. The biological sample 104 may be include diseased tissue (e.g., cancerous) and/or healthy tissue. In some embodiments, the origin or preparation methods of the biological sample may have included any of the examples described herein including with respect to the "Biological Samples" section.

Technique 100 further includes, in some embodiments, obtaining sequencing data 106 from biological sample 104. For example, the sequencing data 106 may be obtained using a sequencing platform such as a next generation sequencing platform (e.g., Illumina®, Roche®, Ion Torrent®, etc.), or any high-throughput or massively parallel sequencing platform. In some embodiments, these methods may be automated, in some embodiments, there may be manual intervention. In some embodiments, the sequencing data may be the result of non-next generation sequencing (e.g., Sanger sequencing).

The sequencing data 106 may include DNA sequencing data, DNA exome sequencing data (e.g., from whole exome sequencing (WES), DNA genome sequencing data (e.g., from whole genome sequencing (WGS)), gene sequencing data, bias-corrected gene sequencing data, or any other suitable type of sequencing data comprising data obtained from a sequencing platform and/or comprising data derived from data obtained from a sequencing platform. In some embodiments, the origin, type, or preparation methods of the sequencing data may include any of the embodiments described herein including in the section "Sequencing Data."

In some embodiments, computing device 108 is used to process the sequencing data 106. The computing device 108 may be operated by a user such as a doctor, clinician, researcher, subject, and/or any other suitable entity. For example, the user may provide the sequencing data 106 as input to the computing device 108 (e.g., by uploading a file), provide user input specifying processing or other methods to be performed using the sequencing data 106 and/or provide input specifying one or more clinical features associated with the subject 102 and/or the biological sample 104.

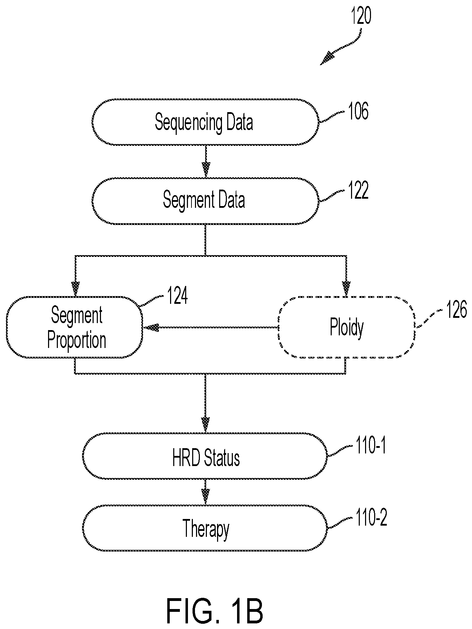

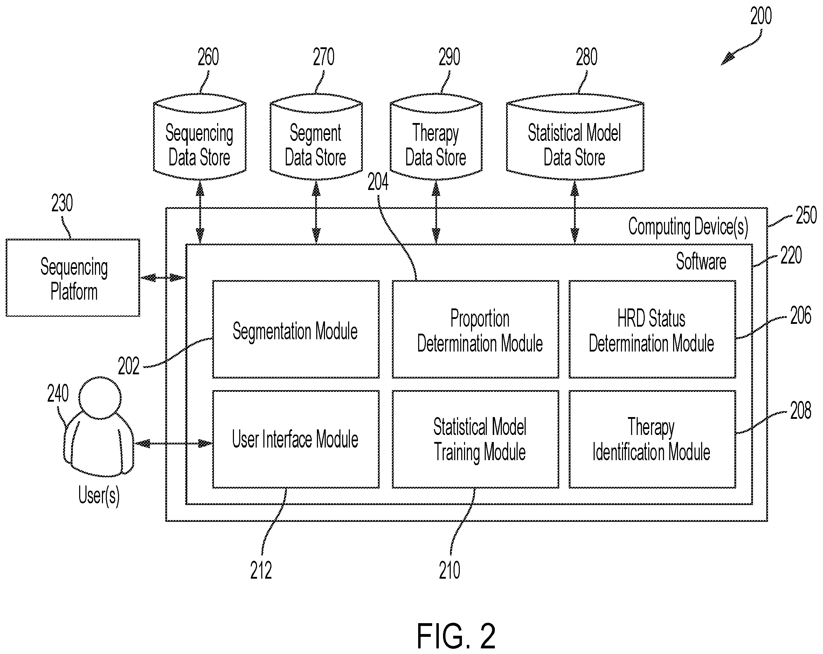

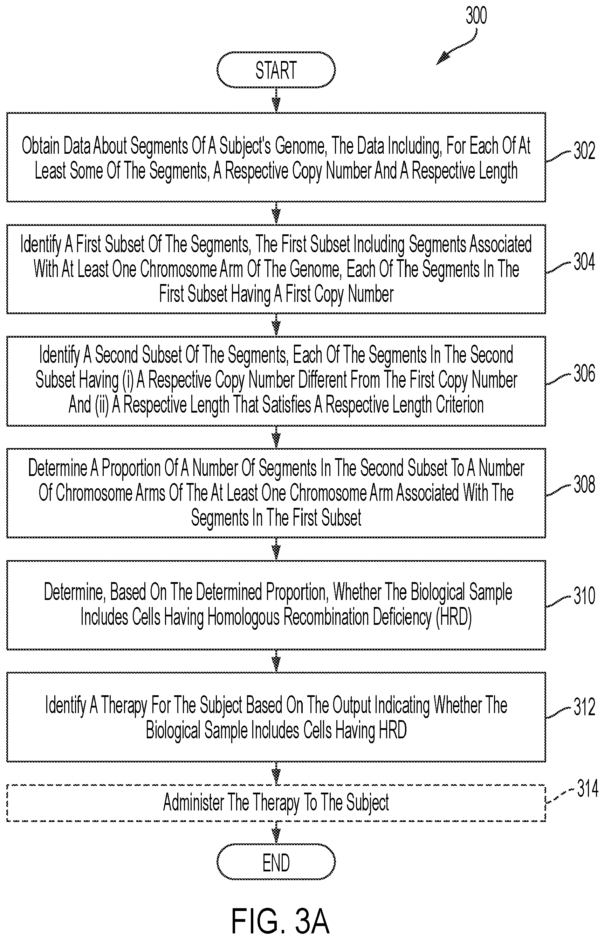

In some embodiments, software on the computing device 108 may be used to determine the HRD status 110-1 of the biological sample 104. An example of computing device 108 and such software is described herein including at least with respect to FIG. 2 (e.g., computing device 250 and software 220). In some embodiments, software on the computing device 108 may be configured to process at least some (e.g., all) of the sequencing data 106 to determine the HRD status of the biological sample 104. This includes: (a) obtaining data about segments of the subject's DNA (e.g., by processing sequencing data 106); (b) identifying a first subset of the obtained segments ("arm-level segments") that includes one or more segments associated with at least one chromosome arm; (c) identifying a second subset of the segments ("long-focal segments"); (d) determining a proportion of the number of segments in the second subset to the number of chromosome arms associated with segments in the first subset; and (e) determining, based on the determined proportion, whether the biological sample includes cells having HRD. Example techniques for determining whether a biological sample includes cells having HRD are described herein including at least with respect to FIGS. 1B and 3A-3C.

In some embodiments, software on the computing device 108 may use the HRD status 110-1 determined for the biological sample 104 to generate a recommendation for treating the subject 102 from which the biological sample 104 was obtained. For example, the HRD status may be used to predict how a subject 102 will respond to a particular therapy or therapies (e.g., platinum-based therapeutic agents, poly ADP ribose polymerase (PARP) inhibitors, etc.). Accordingly, in some embodiments, the software on the computing device 108 may generate a recommendation for administering a particular therapy for the subject when the subject is predicted to respond positively to that particular therapy. For example, when the biological sample 104 is determined to include cells having HRD, the software may be configured to recommend a platinum-based therapeutic agent and/or a PARP inhibitor for the subject 102.

In some embodiments, computing device 108 includes one or multiple computing devices. In some embodiments, when the computing device 108 includes multiple computing devices, each of the computing devices may be used to perform the same process or processes. For example, each of the multiple computing devices may include software used to implement the one or more of the processes 300, 340, and 360, described herein including at least with respect to FIGS. 3A-3C. In some embodiments, when the computing device 108 includes multiple computing devices, the computing devices may be used to perform different processes or different aspects of a process. For example, one computing device may include software used to process the sequencing data 106 to generate segment data, while a different computing device may be used to process segment data to determine an HRD status for a biological sample.

In some embodiments, when the computing device 108 includes multiple computing devices, the multiple computing devices may be configured to communicate via at least one communication network, such as the Internet or any other suitable communication network(s), as aspects of the technology described herein are not limited in this respect. For example, one computing device may be configured to process sequencing data to obtain segment data, and then provide the segment data to one or more other computing devices via the communication network.

In some embodiments, the computing device is configured to generate an output 110 indicative of (a) the HRD status 110-1 determined by computing device 108 and/or (b) a recommendation for administering a particular therapy 110-2 (e.g., a platinum-based therapeutic agent, PARP inhibitor, etc.) to the subject 102 from which the biological sample 104 was obtained. The therapy may include any suitable therapy including one or more of the therapies described herein with respect to the "Therapies" section.

In some embodiments, the output 110 is stored (e.g., in memory), displayed via a user interface, transmitted to one or more other devices, or otherwise processed using any suitable techniques, as aspects of the technology are not limited in this respect. For example, the output 110 may be displayed using a graphical user interface (GUI) of a computing device (e.g., computing device 108).

In some embodiments, the output 110 may be in the form of a report, such as a report including an indication of the HRD status 110-1 and/or a recommendation for prognosis and/or treatment (e.g., therapy 110-2). The generated report can provide a summary of information, so that a clinician can identify an HRD status of the subject 102 or suitable therapy. The report as described herein may be a paper report, an electronic record, or a report in any format that is deemed suitable in the art. The report may be shown and/or stored on a computing device known in the art (e.g., handheld device, desktop computer, smart device, website, etc.). The report may be shown and/or stored on any device that is suitable as understood by a skilled person in the art.

In some embodiments, methods disclosed herein can be used for commercial diagnostic purposes. For example, the generated report may include, but is not limited to, information concerning sequencing data, clinical and pathologic factors, subject's prognostic analysis, predicted response to the treatment, HRD status, and/or other information. In some embodiments, the methods and reports may include database management for the keeping of the generated reports. For instance, the methods as disclosed herein can create a record in a database for the subject (e.g., subject 1, subject 2, etc.) and populate the specific record with data for the subject. In some embodiments, the generated report can be provided to the subject and/or to the clinicians. In some embodiments, a network connection can be established to a server computer that includes the data and report for receiving or outputting. In some embodiments, the receiving and outputting of the date or report can be requested from the server computer.

In some embodiments, technique 100 further includes a step for administering the therapy 110-2 identified as part of the output 110 of computing device 108. The therapy may be administered according to embodiments described herein including with respect to the "Therapies" section.

FIG. 1B is a diagram depicting an illustrative technique 120 for determining whether cells in a biological sample obtained from a subject have HRD, according to some embodiments of the technology described herein. The illustrative technique 120 includes: (a) processing sequencing data 106 to obtain segment data 122; (b) processing the segment data to determine values for segment proportion 124 and (optionally) ploidy 126; and (c) determining HRD status 110-1 based on the segment proportion 124 and (optionally) ploidy 126. In some embodiments, the determined HRD status 110-1 is used to identify the therapy 110-2 for the subject 102.

In some embodiments, technique 120 is implemented using computing device 108. For example, computing device 108 may be configured to receive sequencing data 106 as input and to provide the HRD status 110-1 and/or therapy 110-2 as output. Additionally, or alternatively, the computing device 108 may be configured to receive segment data 122 as input. For example, the computing device 108 may be configured to receive segment data 122 previously-obtained by processing the sequencing data 106.

In some embodiments, the segment data 122 includes data derived from the sequencing data 106. For example, in some embodiments, the segment data 122 identifies one or more segments of the genome. A "segment" of the genome refers to a portion of the genome that has a continuous copy number state. The segment may map to a position in a position in a genome. For example, the segment may map to a chromosome and/or chromosome arm. In some embodiments, segment may be identified using coordinates of positions in a genomic region. For example, the coordinates may include coordinates of endpoints of the segment. As another example, the coordinates may include a starting point and a length of the segment. A coordinate of an endpoint of a segment may represent the position of the endpoint relative to the sequence of the chromosome and/or chromosome arm to which the segment maps. Such a coordinate may indicate a number of bases relative to the start point of the sequence represented by the chromosome and/or chromosome arm. In some embodiments, the segment data 122 additionally, or alternatively, includes data about the identified segments. For example, the segment data 122 may identify the copy number of a particular segment. The segment data 122 may identify a length of a particular segment. Additionally, or alternatively, in some embodiments, the segment data 122 includes data about the biological sample 104. For example, in some embodiments, the segment data 122 includes ploidy of at least some cells (e.g., tumor cells) in the biological sample 104.

In some embodiments, segment data 122 is obtained by processing sequencing data 106. In one non-limiting example, processing sequencing data to obtain segment data may include one or more of the following acts: (a) partitioning a reference genome into bins, (b) mapping sequence reads to the reference genome, (b) determining a number of sequence reads mapped to each bin to obtain a copy number profile, and (e) segmenting the copy number profile. Example segmentation techniques are described by Kendall, J. and Krasnitz, A. ("Computational methods for DNA copy-number analysis of tumors." Cancer Genomics and Proteomics: Methods and Protocols (2014): 243-259).

In some embodiments, the reference genome is partitioned into any suitable number of bins. For example, the reference genome may be portioned into a number of bins between 5,000 and 100,000 bins, between 10,000 and 70,000 bins, between 15,000 and 40,000 bins, between 18,000 and 25,000 bins, at least 5,000 bins, at least 10,000 bins, at least 15,000 bins, at least 18,000 bins, at least 20,000 bins, at least 30,000 bins, at least 40,000 bins, at least 50,000 bins, at most 200,000 bins, at most 100,000 bins, at most 70,000 bins, at most 40,000 bins, at most 25,000 bins, at most 20,000 bins, or any other suitable number of bins. In some embodiments, the genome is partitioned such that each bin includes the same number of unique k-mers. Accordingly, the size of each bin may vary depending on the relative genomic positions of the unique k-mers. For example, a bin may include bases between adjacent k-mers, and the number of bases between adjacent k-mers may vary.

In some embodiments, the sequencing data 106 includes sequence reads (e.g., DNA sequence reads), and the sequence reads are mapped to the reference genome. The sequence reads may be mapped to the reference genome using any suitable sequence alignment techniques, as aspects of the technology described herein are not limited to any particular sequence alignment technique. In some embodiments, the positions of the bins (e.g., into which the reference genome was portioned) and the mapped positions of the sequence reads are used to determine the number of sequence reads that map to the different bins. In some embodiments, segments of the genome are obtained by estimating the underlying piece-wise copy number profile from bin-count data. Additionally, or alternatively, in some embodiments, the segment data 122 may be obtained using segmentation software. Any segmentation software capable of segmenting genomic sequences may be used. For example, Sequenza software may be used. Sequenza software and techniques used by it to segment genomic sequences are described in: Favero F., et al. ("Sequenza: allele-specific copy number and mutation profiles from tumor sequencing data." Annals of Oncology 26.1 (2015): 64-70). Additionally, or alternatively, ABSOLUTE software may be used. ABSOLUTE software and techniques used by it to segment genomic sequences are described in: Carter, S., et al. ("Absolute quantification of somatic DNA alterations in human cancer." Nat Biotechnol. 30.5 (2012): 413-421). Additionally, or alternatively, absCN-seq software may be used. absCN-seq software and techniques used by it to segment genomic sequences are described in Bao, L., ("AbsCN-seq: a statistical method to estimate tumor purity, ploidy and absolute copy numbers from next-generation sequencing data." Bioinformatics. 30.8 (2014): 1056-1063). Additionally, or alternatively, the ModelSegments tool of the GATK software may be used. GATK software is described by Van der Auwera GA & O'Connor BD. (Genomics in the Cloud: Using Docker, GATK, and WDL in Terra (1st Edition). O'Reilly Media. (2020)).

The segment data 122 is used to determine segment proportion 124. Determining the segment proportion 124 includes (a) identifying a first subset of the segments 122 ("arm-level segments") associated with chromosome arms (e.g., the chromosome arms to which the segments map), (b) identifying a second subset of the segments 122 ("long-focal segments"), and (c) determining the segment proportion 124 based on the identified long-focal segments and the chromosome arms associated with the arm-level segments 122.

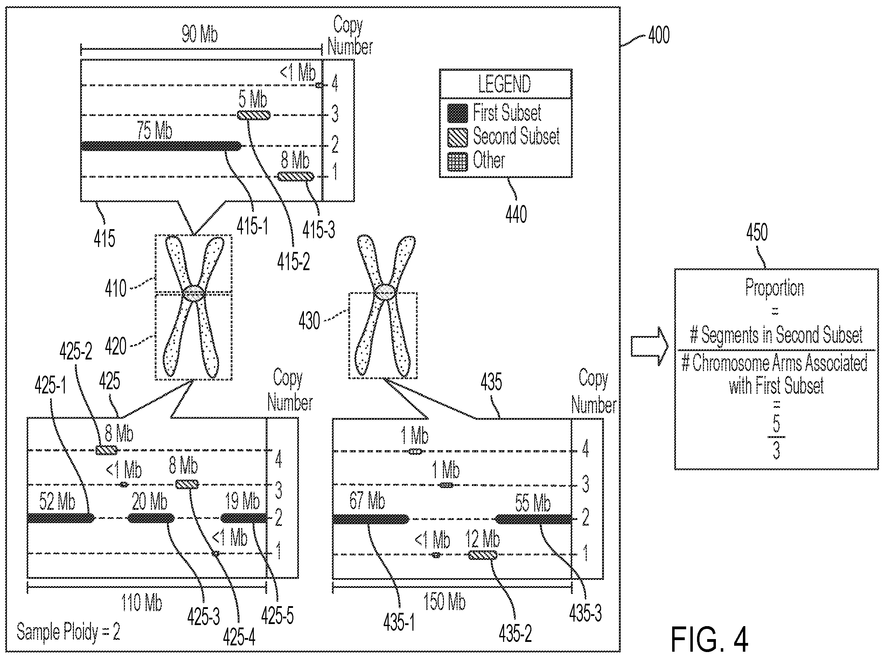

An "arm-level" segment refers to a segment that meets at least one of the following criteria. First, a segment may be identified as an arm-level segment if it: (a) has a length that is greater than or equal to a first threshold length, and (b) has a copy number that is equal to ploidy of the biological sample. In some embodiments, the first threshold length depends on a length of a chromosome arm associated with the segment. A segment is associated with a particular chromosome arm if it maps to that particular chromosome arm. For example, the first threshold length may be between 20% and 100% of the length of the chromosome arm, between 25% and 75% of the length of the chromosome arm, between 30% and 70% of the length of the chromosome arm, between 35% and 65% of the length of the chromosome arm, or any other suitable length. Additionally, or alternatively, the first threshold length may be at least 20% of the length of the chromosome arm, at least 25% of the length of the chromosome arm, at least 30% of the length of the chromosome arm, at least 35% of the length of the chromosome arm, at least 40% of the length of the chromosome arm, at least 60% of the length of the chromosome arm, or any other suitable length. Additionally, or alternatively, the first threshold length may be at most 100% of the length of the chromosome arm, at most 95% of the length of the chromosome arm, at most 90% of the length of the chromosome arm, at most 80% of the length of the chromosome arm, at most 75% of the length of the chromosome arm, at most 70% of the length of the chromosome arm, or any other suitable length. It should be appreciated that any of the above-listed upper boundaries may be listed with any of the above-listed lower boundaries. Second, a segment may be identified as an arm-level segment if (a) it is one of a threshold number or fewer than a threshold number of segments, and (b) the sum of the lengths of the segments that satisfy criterion (a) is greater than or equal to a second threshold length. For example, the threshold number of segments may be between 1 and 10 segments, between 2 and 6 segments, or any other suitable number of segments. Additionally, or alternatively, the threshold number of segments may be at most 8 segments, at most 7 segments, at most 6 segments, at most 5 segments, at most 4 segments, at most 3 segments, at most 2 segments, or any other suitable number of segments. Additionally, or alternatively, the threshold number of segments may be at least one segment, at least 2 segments, or any other suitable number of segments. It should be appreciated that any of the above-listed upper boundaries may be listed with any of the above-listed lower boundaries. In some embodiments, the second threshold length depends on the length of the chromosome arm associated with the segment. For example, the second threshold length may be between 20% and 100% of the length of the chromosome arm, between 25% and 75% of the length of the chromosome arm, between 30% and 70% of the length of the chromosome arm, between 35% and 65% of the length of the chromosome arm, or any other suitable length. Additionally, or alternatively, the second threshold length may be at least 20% of the length of the chromosome arm, at least 25% of the length of the chromosome arm, at least 30% of the length of the chromosome arm, at least 35% of the length of the chromosome arm, at least 40% of the length of the chromosome arm, at least 60% of the length of the chromosome arm, or any other suitable length. Additionally, or alternatively, the second threshold length may be at most 100% of the length of the chromosome arm, at most 95% of the length of the chromosome arm, at most 90% of the length of the chromosome arm, at most 80% of the length of the chromosome arm, at most 75% of the length of the chromosome arm, at most 70% of the length of the chromosome arm, or any other suitable length. It should be appreciated that any of the above-listed upper boundaries may be listed with any of the above-listed lower boundaries. The second threshold length may be the same as or different from the first threshold length. Examples of identifying arm-level segments are described herein including at least with respect to FIGs. 3A-3B, and 4.

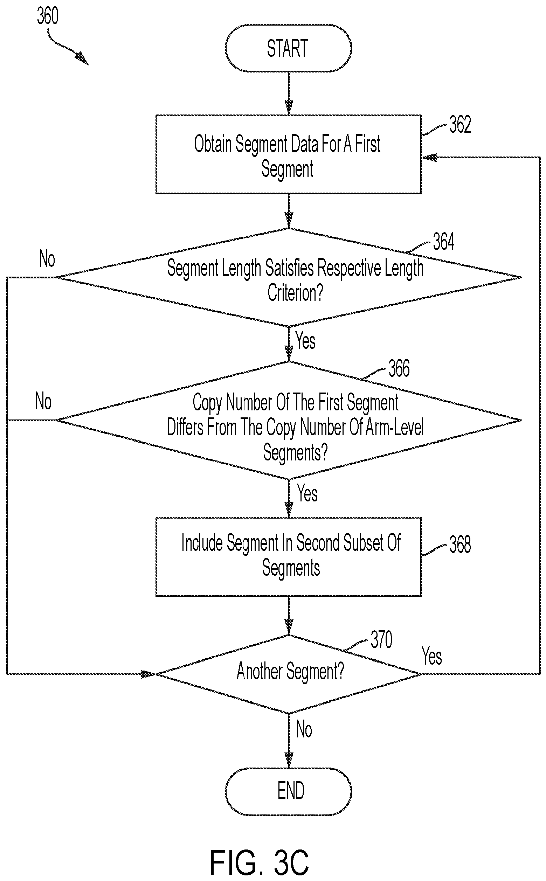

A "long-focal" segment refers to a segment that meets the following criteria. A segment may be identified as a long-focal segment if (a) the segment has a copy number that differs from a copy number of the identified arm-level segments, and (b) the segment has a length that satisfies a predetermined length criterion. In some embodiments, the length criterion depends on a length of a chromosome arm associated with the segment (i.e., the chromosome arm that the segment maps to). In some embodiments, the length criterion is a predetermined range, and a segment satisfies the length criterion if a length of the segment falls within the predetermined range. For example, the predetermined range may be a range between 1 Mb and 30% of the length of the chromosome arm associated with the segment, between 2 Mb and 20% of the length of the chromosome arm associated with the segment, between 3 Mb and 10% of the length of the chromosome arm associated with the segment, 4 Mb and 5% of the length of the chromosome arm, or any other suitable predetermined range. Additionally, or alternatively, in some embodiments, the length criterion is a threshold length of at least 1 Mb, at least 2 Mb, at least 3 Mb, at least 4 Mb, at least 5% of the length of the chromosome arm, at least 10% of the length of the chromosome arm, at least 20% of the length of the chromosome arm, at least 30% of the length of the chromosome arm, or any other suitable threshold length. Additionally, or alternatively, the length criterion may be a threshold length of at most 30% of the length of the chromosome arm, at most 20% of the length of the chromosome arm, at most 10% of the length of the chromosome arm, at most 5% of the length of the chromosome arm, or any other suitable threshold length. It should be appreciated that any of the above-listed upper boundaries may be listed with any of the above-listed lower boundaries. Examples of identifying long-focal segments are described herein including at least with respect to FIGs. 3A, 3C, and 4.

In some embodiments, segment proportion 124 is determined based on the identified arm-level and long-focal segments. For example, in some embodiments, the segment proportion 124 is a proportion of the number of identified long-focal segments (i.e., number of segments included in the first subset if segments) to the number of chromosome arms associated with the identified arm-level segments (i.e., segments included in the second subset).

In some embodiments, ploidy 126 of at least some cells of the biological sample 104 is also obtained from the segment data 122. For example, in some embodiments, ploidy is estimated for tumor cells in the biological sample. In some embodiments, ploidy 126 is estimated based on results of processing sequencing data to obtain segment data 122. For example, as described herein, in some embodiments, sequencing data is processed to obtain segments of the genome. In some embodiments, a copy number may be determined for each of one or more of the segments. In some embodiments, ploidy 126 is estimated by averaging the copy numbers determined for each of the one or more segments. For example, segments and their respective copy numbers may be obtained for at least some of the genome (e.g., the whole genome) of at least some cells (e.g., tumor cells) of the biological sample. Ploidy may be estimated for those cells by determining the average of the copy numbers obtained for the segments. Additionally, or alternatively, in some embodiments, ploidy may be estimated using the total and minor allele counts obtained for at least some of the segments. Such a technique may be used when the majority of segments appear amplified relative to the anticipated ploidy. For example, in the standard diploid, the majority of alleles are anticipated to have a total copy number of 2, with the number of minor alleles being 1 or 0. When the majority of segments appear amplified relative to these anticipated values, a ploidy estimation algorithm may account for the total and minor allele counts, as well as the minimization of copy number alteration events. For example, segmentation software such as Sequenza software, ABSOLUTE software, absCN-seq software, and/or the ModelSegments tool of the Genome Analysis Toolkit (GATK) software may be used to implement such techniques to estimate ploidy.

In some embodiments, ploidy 126 is used to identify arm-level segments. For example, as described herein, the copy number of a segment may be compared to ploidy of the biological sample as part of the techniques for identifying arm-level segments.

In some embodiments, segment proportion 124 and (optionally) ploidy 126 are used to determine HRD status 110-1 that indicates whether the biological sample includes cells having HRD. In some embodiments, determining the HRD status 110-1 includes determining a score. The score may be the segment proportion 124 itself or a combination (e.g., linear or non-linear) of the segment proportion 124 and ploidy 126. When the score is a combination of predictors (e.g., combination of segment proportion 124 and ploidy 126), coefficients may be determined for each of the predictors and used to determine a weighted sum of the predictors. For example, coefficients may be estimated by performing a regression analysis on training data. In some embodiments, the regression analysis is performed using data that includes, for each of a plurality of training subjects, values for each of the predictors and a known HRD status for the subject. In some embodiments, the score is compared to a threshold to determine the HRD status 110-1. For example, if the score is greater than or equal to the threshold, then the biological sample is determined to include cells having HRD. The threshold may be determined based on results of performing the regression analysis used to estimate coefficients (e.g., for segment proportion 124 and/or ploidy 126). For example, performance metrics (e.g., F1 score, positive predictive value, negative predictive value, etc.) used for evaluating the performance of the regression analysis in distinguishing between HRD positive and/or HRD negative subjects may be used to determine the threshold.

A statistical model is used to determine the HRD status 110-1 based on segment proportion 124, (optionally) ploidy 126, and (optionally) any other suitable predictor(s). The statistical model may include any suitable statistical model such as, for example, a generalized linear model (e.g., a linear regression model, a logistic regression model, a probit regression model, etc.). It should be appreciated that, in some embodiments, the statistical model may not be a generalized linear model and may be a different type of statistical model such as, for example, a random forest regression model, a neural network, a support vector machine, a Gaussian mixture model, a hierarchical Bayesian model, and/or any other suitable statistical model, as aspects of the technology described herein are not limited to using generalized linear models for the prediction of HRD status 110-1. Techniques for processing one or more predictors using a statistical model are described herein including at least with respect to act 310 of process 300 shown in FIG. 3A.

In some embodiments, the HRD status 110-1 is used to identify therapy 110-2 to be administered to the subject 102. For example, subjects having HRD have been shown to be responsive to certain therapies such as, for example, platinum-based therapeutic agents and PARP inhibitors. If the HRD status 110-1 indicates that the subject 102 has HRD, then a platinum-based therapeutic agent and/or PARP inhibitor may be identified as therapy 110-2. The therapy may be identified and/or administered according to embodiments described herein including with respect to the "Therapies" section.

FIG. 2 is a block diagram of an example system 200 for determining whether a biological sample obtained from a subject includes cells having HRD and for identifying a therapy for the subject, according to some embodiments of the technology described herein. System 200 includes computing device(s) 250 configured to have software 220 execute thereon to perform various functions in connection with determining the HRD status of a biological sample and/or identifying a therapy for a subject based on an HRD status of a biological sample. In some embodiments, software 220 includes a plurality of modules. A module may include processor-executable instructions that, when executed by at least one computer hardware processor, cause the at least one computer hardware processor to perform the function(s) of the module. Such modules are sometimes referred to herein as "software modules," each of which includes processor executable instructions configured to perform one or more processes, such as the processes described herein including at least with respect to FIGs. 3A-3C.

The computing device(s) 250 may be operated by one or more user(s) 240. For example, the user(s) 240 may include one or more individuals who are treating and/or studying subject(s) (e.g., doctors, clinicians, researchers, etc.). Additionally, or alternatively, the user(s) 240 may include the subjects(s) for which the HRD status is being determined. In some embodiments, the user(s) 240 may provide, as input to the computing device 250 (e.g., by uploading one or more files, by interacting with a user interface of the computing device(s) 250, etc.), sequencing data and/or segment data obtained for biological sample(s) (e.g., obtained or previously-obtained from subject(s)). Additionally, or alternatively, the user(s) 240 may provide user input specifying processing or other methods to be performed on the sequencing data and/or segment data. Additionally, or alternatively, the user(s) 240 may access results of processing the sequencing and/or segment data. For example, the user(s) 240 may access the HRD status determined for a subject and/or a recommendation for administering therapy to the subject.

As shown in FIG. 2, software 220 includes multiple software modules for determining an HRD status for a biological sample and/or identifying a therapy for a subject. Such software modules include a segmentation module 202, a proportion determination module 204, an HRD status determination module 206, and a therapy identification module 208.

In some embodiments, the segmentation module 202 obtains sequencing data (e.g., sequencing data 106 shown in FIGs. 1A-1B) from sequencing platform 230, the user(s) 240 (e.g., by the user uploading the sequencing data), and/or the sequencing data store 260.

In some embodiments, the segmentation module 202 is configured to generate segment data (e.g., segment data 122 shown in FIG. 1B) for the biological sample. For example, the segmentation module 202 may be configured to process sequencing data, such as DNA sequence reads, to obtain segment data. In some embodiments, processing sequence data to obtain segment data includes using the DNA sequence reads to determine a copy number profile for at least a portion (e.g., the whole) genome, and deriving the segment data from the copy number profile. Additionally, or alternatively, in some embodiments, the segmentation module 202 may use segmentation software such as, for example, Sequenza software, ABSOLUTE software, absCN-seq software, and/or the ModelSegments tool of the Genome Analysis Toolkit (GATK) software. Techniques for generating segment data are described herein including at least with respect to FIG. 1B and act 302 of process 300 shown in FIG. 3A.

In some embodiments, the segment data generated by the segmentation module 202 may include any suitable segment data, as aspects of the technology described herein are not limited in this respect. For example, the segmentation module 202 may be configured to generate data identifying a genomic position of the segments (e.g., coordinates for endpoints of segments), data identifying a chromosome arm associated with each segment, data indicative of lengths of the segments, copy numbers for the segments, ploidy of at least some cells (e.g., tumor cells) in the biological sample, and/or any other suitable segment data, as aspects of the technology described herein are not limited in this respect.

In some embodiments, the proportion determination module 204 obtains segment data (e.g., segment data 122 shown in FIG. 1B) from segment data store 270, the user(s) 240 (e.g., by the user uploading the segment data), and/or the segmentation module 202.

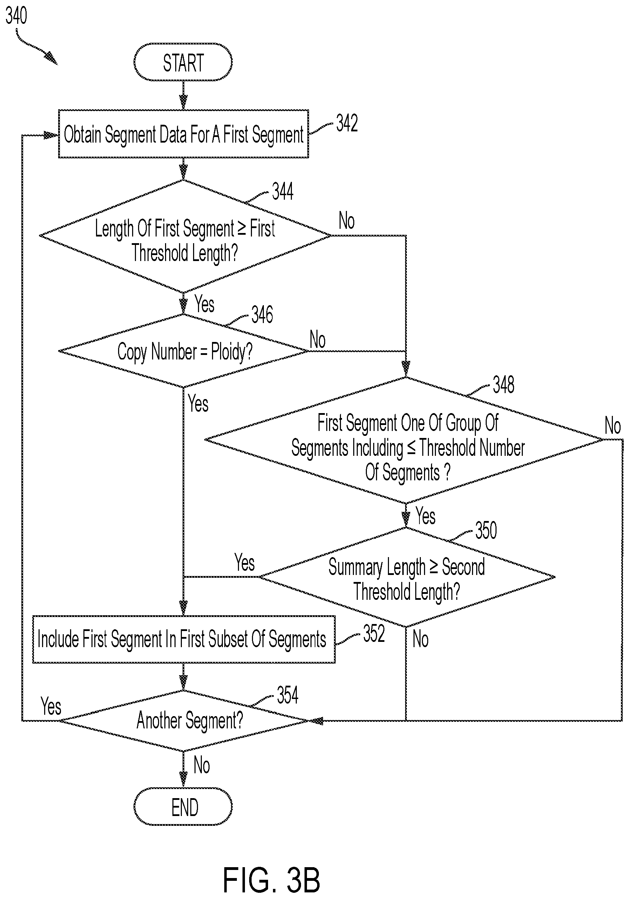

In some embodiments, the proportion determination module 204 is configured to identify different subsets of the segments for which the segment data was obtained. For example, in some embodiments, the proportion determination module 204 is configured to identify a first subset of segments. The first subset of segments may consist of arm-level segments. In some embodiments, identifying the first subset of segments includes, for a particular segment, (a) comparing data obtained for the particular segment to one or more criteria and (b) including the segment in the first subset if the data satisfies the criteria. For example, this may include comparing a copy number of the segment to ploidy of at least some cells (e.g., tumor cells) the biological sample. Additionally, or alternatively, this may include comparing a length of the particular segment to a threshold length and/or predetermined range of lengths. Additionally, or alternatively, this may include (a) determining whether the segment is one of fewer than or equal to a threshold number of segments (e.g., between 1 and 10 segments, between 2 and 6 segments, 2 segments, 3 segments, 4 segments, 5 segments, 6 segments, 7 segments, 8 segments, etc.), and (b) comparing the sum of the lengths of the segment(s) satisfying criterion (a) to a threshold length and/or predetermined range of lengths (e.g., between 25% and 75% of the length of the chromosome arm, between 30% and 70% of the length of the chromosome arm, between 35% and 65% of the length of the chromosome arm, 30% of the length of the chromosome arm, 40% of the length of the chromosome arm, 50% of the length of the chromosome arm, 60% of the length of the chromosome arm, 70% of the length of the chromosome arm, etc.). Techniques for identifying a first subset of segments (i.e., arm-level segments) are described herein including at least with respect to FIG. 1B, act 304 of process 300 shown in FIG. 3A, process 340 shown in FIG. 3B, and the example shown in FIG. 4.

In some embodiments, the proportion determination module 204 is further configured to identify a second subset of segments. The second subset of segments may consist of long-focal segments. In some embodiments, identifying the second subset of segments includes, for a particular segment, (a) comparing data obtained for the particular segment to one or more criteria, and (b) including the segment in the second subset if the data satisfies the criteria. For example, this may include comparing a copy number of the segment to a copy number of segments included in the first subset of segments (e.g., a copy number of the arm-level segments). Additionally, or alternatively, this may include comparing a length of the segment to a threshold length and/or predetermined range of lengths. Techniques for identifying a second subset of segments (i.e., long-focal segments) are described herein including at least with respect to FIG. 1B, act 306 of process 300 shown in FIG. 3A, process 360 shown in FIG. 3C, and the example shown in FIG. 4.

In some embodiments, the proportion determination module 204 is further configured to determine a proportion (e.g., segment proportion 124 shown in FIG. 1B) based on the identified first and second subsets of segments. In some embodiments, determining the proportion includes determining a proportion of the number of segments included in the second subset to a number of chromosome arms associated with segments in the first subset. In some embodiments, the proportion determination module 204 provides the determined proportion to the HRD status determination module 206 for use in determining an HRD status of the biological sample. Techniques for determining the proportion are described herein including at least with respect to FIGs. 1A-1B, act 308 of process 300 shown in FIG. 3A, and the example shown in FIG. 4.

In some embodiments, the HRD status determination module 206 obtains the proportion from proportion determination module 204 and uses the proportion to determine whether the biological sample includes cells having HRD. In some embodiments, the HRD status determination module 206 is configured to determine the HRD status using any suitable techniques, as aspects of the technology described herein are not limited in this respect. For example, the HRD status determination module 206 may obtain one or more trained statistic models from the statistical model data store 280 and process the proportion using the obtained statistical model(s) to obtain a prediction of whether the biological sample includes cells having HRD. Additionally, or alternatively, the HRD status determination module 206 may compare the proportion to a threshold and determine the HRD status based on results of the comparison.

Additionally, or alternatively, in some embodiments, the HRD status determination module 206 obtains segment data from segment data store 270, the user(s) 240 (e.g., by the user uploading the segment data), and/or the segmentation module 202 and processes the segment data (e.g., using the obtained statistical model(s)) to obtain the prediction of whether the biological sample includes cells having HRD. For example, the segment data may indicate ploidy of at least some cells (e.g., the tumor cells) the biological sample, and the HRD status determination module 206 may process the ploidy to determine the HRD status. Techniques for determining whether cells of a biological sample have HRD are described herein including at least with respect to FIG. 1B and act 310 of process 300 shown in FIG. 3A.

In some embodiments, the therapy identification module 208 obtains the HRD status from HRD status determination module and therapy data from therapy data store 290 and/or user(s) 240 (e.g., by uploading the therapy data) and uses the HRD status and therapy data to identify a therapy to be administered to the subject. For example, the therapy data may identify therapies that have been shown to be effective in treating HRD-positive subjects (i.e., at least some of the subject's cells having HRD). If the obtained HRD status indicates that the subject is HRD positive, then the therapy identification module 208 may use the therapy data to identify a therapy shown to be effective in treating HRD-positive subjects. Additionally, or alternatively, the therapy data may identify therapies that have been shown to be effective in treating HRD-negative subjects. If the obtained HRD status indicates that the subject is HRD-negative, then the therapy identification module 208 may use the therapy data to identify a therapy shown to be effective in treating HRD-negative subjects. Techniques for identifying a therapy are described herein including at least with respect to FIGs. 1A-1B, act 312 of process 300 shown in FIG. 3A, and in the section "Therapies".

In some embodiments, sequencing data is obtained from sequencing platform 230. For example, segmentation module 202 may obtain (either pull or be provided) the sequencing data from the sequencing platform 230. The sequencing platform 230 may be of any suitable type such as, for example, any of the sequencing platforms described herein including at least with respect to FIG. 1A and with respect to the section "Sequencing Data".

In some embodiments, the segmentation module 202, the proportion determination module 204, the HRD status determination module 206, and/or the therapy identification module 208 obtain data via user interface module 212 and/or one or more other interface modules (not shown). For example, the modules may obtain sequencing data, segment data, and/or therapy data from the interface module(s). The data may be provided by a communication network (not shown), such as Internet or any other suitable network, as aspects of the technology described herein are not limited in this respect.

As shown in FIG. 2, system 200 also includes sequencing data store 260, segment data store 270, therapy data store 290, and statistical model data store 280. In some embodiments, software 220 obtains data from sequencing data store 260, segment data store 270, therapy data store 290, statistical model data store 280, and/or user(s) 240 (e.g., by uploading data). In some embodiments, the software 220 further includes statistical model training module 210 for training one or more statistical models (e.g., stored in statistical model data store 280).

In some embodiments, sequencing data is obtained from the sequencing data store 260. The sequencing data store 260 may be of any suitable type (e.g., database system, multi-file, flat file, etc.) and may store sequencing data in any suitable way in any suitable format, as aspects of the technology described herein are not limited in this respect. The sequencing data store 260 may be part of or external to computing device(s) 250.

In some embodiments, the sequencing data store 260 stores sequencing data obtained for a biological sample, as described herein including at least with respect to FIG. 1A. In some embodiments, the stored sequencing data may have been previously uploaded by a user (e.g., user(s) 240) and/or from one or more public data stores and/or studies. In some embodiments, a portion of the sequencing data may be processed by the segmentation module 202 to generate segment data for the biological sample.

In some embodiments, segment data is obtained from the segment data store 270. The segment data store 270 may be of any suitable type (e.g., database system, multi-file, flat file, etc.) and may store segment data in any suitable way in any suitable format, as aspects of the technology described herein are not limited in this respect. The segment data store 270 may be part of or external to computing device(s) 250.

In some embodiments, the segment data store 270 stores segment data obtained for a biological sample, as described herein including at least with respect to FIG. 1B. For example, the segment data may have been previously-obtained by processing sequencing data obtained for the biological sample. In some embodiments, the stored segment data may have been previously uploaded by a user (e.g., user(s) 240), uploaded from one or more public data stores and/or studies, and/or uploaded from the segmentation module 202. In some embodiments, a portion of the segment data may be processed by the proportion determination module 204 to identify arm-level and long-focal segments. In some embodiments, at least a portion of the segment data may be processed by the HRD status determination module to determine whether the biological sample includes cells having HRD. For example, ploidy of at least some cells (e.g., tumor cells) the biological sample may be included in the segment data and used by the HRD status determination module 206 to determine the HRD status.

In some embodiments, therapy data is obtained from the therapy data store 290. The therapy data store 290 may be of any suitable type (e.g., database system, multi-file, flat file, etc.) and may store therapy data in any suitable way in any suitable format, as aspects of the technology described herein are not limited in this respect. The therapy data store 290 may be part of or external to computing device(s) 250.

In some embodiments, the therapy data store 290 stores therapy data identifying candidate therapies for treating subject(s). For example, the therapy data may identify therapies that have been shown to effectively treat subjects having HRD and/or therapies shown to effectively treat subjects who do not have HRD. In some embodiments, the therapy data may have been previously uploaded by a user (e.g., user(s) 240) and/or from one or more public data stores and/or studies. In some embodiments, at least some of the therapy data may be processed by the therapy identification module 208 to identify a therapy for a subject based on an HRD status determined for the subject.

In some embodiments, the statistical model training module 210 may be configured to train one or more statistical models to perform various tasks. For example, the statistical model training module 210 may be configured to train one or more statistical models to predict whether a biological sample includes cells having HRD. In some embodiments, the statistical model training module 210 may be configured to train the one or more statistical models using training data. For example, the statistical model training module 210 may obtain the training data from sequencing data store 260, segment data store 270, sequencing platform 230, segmentation module 202, proportion determination module 204, and/or user(s) 240 (e.g., by uploading a file including the training data, by entering the data via a user interface, etc.).

In some embodiments, the statistical model training module 210 may provide statistical model(s) to statistical model data store 280 for storage therein. The statistical model data store 280 may be of any suitable type (e.g., database system, multi-file, flat file, etc.) and may store statistical model data in any suitable way and in any suitable format, as aspects of the technology described herein are not limited in this respect. The statistical model data store 280 may be part of or external to computing device(s) 250.

User interface module 212 may be configured to generate a graphical user interface (GUI), a text-based user interface, and/or any other suitable type of interface through which a user may provide input and view information generated by software 220. For example, in some embodiments, the user interface module 212 may be a webpage or web application accessible through an Internet browser. In some embodiments, the user interface module 212 may generate a graphical user interface (GUI) of an app executing on the user's mobile device. In some embodiments, the user interface module 212 may generate a number of selectable elements through which a user may interact. For example, the user interface module 212 may generate dropdown lists, checkboxes, text fields, or any other suitable element.

In some embodiments, the user interface module 212 is configured to generate a GUI including one or more results of processing the sequencing and/or segment data. For example, the GUI may include an indication of the HRD status determined for the biological sample by the HRD status determination module 206. Additionally, or alternatively, the GUI may include an indication of the therapy identified for the subject by the therapy identification module 208. For example, the GUI may include a prompt to a healthcare professional (e.g., user(s) 240) to administer, to the subject, the therapy identified by the therapy identification module 208.

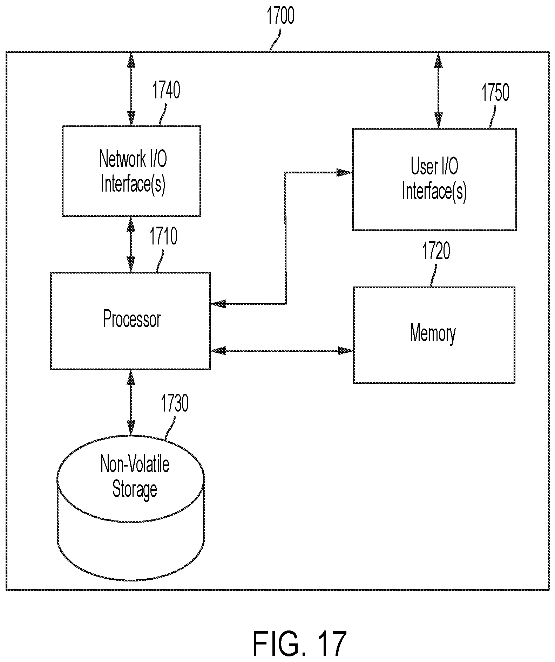

**FIG. 3A** is a flowchart of an illustrative process 300 for determining whether a biological sample obtained from a subject includes cells having HRD, according to some embodiments of the technology described herein. One or more acts of process 300 may be performed automatically by any suitable computing device(s). For example, the act(s) may be performed by a laptop computer, a desktop computer, one or more servers, in a cloud computing environment, computing device 1700 as described herein with respect to FIG. 17, and/or in any other suitable way.

At act 302, data is obtained about segments of a subject's genome. In some embodiments, the segment data was previously-obtained. Thus, in some embodiments, obtaining the segment data may include accessing the data (e.g., from a memory, over a network, via a file being provided via an appropriate interface, etc.). For example, the segment data may be obtained from a data store, such as segment data store 270 shown in FIG. 2, and/or from user(s) (e.g., user(s) 240 shown in FIG. 2) providing a file including the segment data via an appropriate interface, such as user interface module 212 shown in FIG. 2.

In additional or alternative embodiments, obtaining the segment data includes generating the segment data. In some embodiments, generating the segment data includes processing sequence data to obtain the segment data. For example, this may include using DNA sequence reads to determine a copy number profile for at least a portion (e.g., the whole) genome, and deriving the segment data from the copy number profile. Additionally, or alternatively, in some embodiments, the segment data may be obtained using segmentation software such as, for example, Sequenza software, ABSOLUTE software, absCN-seq software, and/or the ModelSegments tool of the Genome Analysis Toolkit (GATK) software. Techniques for generating segment data are described herein including at least with respect to FIG. 1B.

In some embodiments, segment data is obtained for some or all of the subject's genome. For example, segment data may be obtained for the subject's entire genome or at least one chromosome of the subject's genome. Additionally, or alternatively, segment data may be obtained for between 10% and 100% of the subject's genome, between 25% and 95% of the subject's genome, between 45% and 85% of the subject's genome, or any other suitable amount of the subject's genome. Additionally, or alternatively, segment data may be obtained for at least 95% of the subject's genome, at least 90% of the subject's genome, at least 85% of the subject's genome, at least 80% of the subject's genome, at least 75% of the subject's genome, at least 70% of the subject's genome, at least 65% of the subject's genome, at least 60% of the subject's genome, at least 55% of the subject's genome, at least 50% of the subject's genome, at least 45% of the subject's genome, at least 40% of the subject's genome, at least 30% of the subject's genome, at least 25% of the subject's genome, at least 20% of the subject's genome, at least 15% of the subject's genome, at least 10% of the subject's genome, or any other suitable amount of the subject's genome. Additionally, or alternatively, segment data may be obtained for at most 100% of the subject's genome, at most 95% of the subject's genome, at most 85% of the subject's genome, or any other suitable amount of the subject's genome, as aspects of the technology described herein are not limited in this respect. It should be appreciated that any of the above-listed upper boundaries may be listed with any of the above-listed lower boundaries.

In some embodiments, the segment data includes data about each of at least some (all or less than all) of the segments identified for the genome (e.g., derived from a copy number profile, output by segmentation software, etc.). For example, the segment data may not include data about segments that have been filtered out due to quality control reasons, mappability reasons (e.g., the inability of sequence reads to map to particular region(s) of the genome), and/or noise. In some embodiments, the segment data includes data indicative of a respective length for each of at least some of the segments. The data indicative of length may specify the length itself, may specify coordinates of endpoints of the segment from which the length can be inferred, or may include any other suitable data indicative of a length of a segment, as aspects of the technology described herein are not limited in this respect. For example, the segment data may specify, for a particular segment, the number of base pairs included in the segment. Additionally, or alternatively, in some embodiments, the segment data includes data indicative of a respective copy number for each of at least some of the segments. The copy number of a segment refers to the number of copies of that particular segment in the genome of at least some cells of the biological sample. For example, the copy number of a segment may refer to the number of copies of that particular segment in the genome of tumor cells in the biological sample. Additionally, or alternatively, the copy number of a segment may refer to the number of copies of the particular segment in the genome of normal cells in the biological sample. Additionally, or alternatively, in some embodiments, the segment data includes data identifying a respective chromosome arm associated with each of at least some of the segments. A segment is associated with a particular chromosome arm if it maps to the particular chromosome arm. In some embodiments, the data identifying the chromosome arm may include a number used to identify the chromosome, as well as an indication of whether the segment is a segment that maps to the p or q arm of the chromosome. Additionally, or alternatively, in some embodiments, the segment data includes data indicative of ploidy of at least some cells (e.g., tumor cells) the biological sample.

At act 304, process 300 includes identifying a first subset of the segments (i.e., arm-level segments). For example, the first subset of segments may be identified from among the segments for which segment data was obtained at act 302. In some embodiments, identifying the first subset of segments includes, for each particular segment of at least some (e.g., all of the segments, (a) comparing data obtained for the particular segment to one or more criteria and (b) including the segment in the first subset if the one or more criteria are satisfied. This includes comparing a copy number of the segment to ploidy of the biological sample. Additionally, or alternatively, this includes comparing a length of the particular segment to a threshold length and/or predetermined range of lengths. Additionally, or alternatively, this may include (a) determining whether the segment is one of fewer than or equal to a threshold number of segments, and (b) comparing the sum of the lengths of the segment(a) satisfying criterion (a) to a threshold length and/or predetermined range of lengths. Techniques for identifying a first subset of segments are described herein including at least with respect to FIG. 1B, process 340 in FIG. 3B and in the example shown in FIG. 4.

At act 306, a second subset of the segments of the subject's genome is identified (i.e., long-focal segments). For example, the second subset of segments may be identified from among the segments for which segment data was obtained at act 302. Alternatively, the second subset of segments may be identified among the segments remaining after the first subset of segments is identified at act 304. Identifying the second subset of segments includes, for each particular segment of at least some (e.g., all) of the segments, (a) comparing data obtained for the particular segment to one or more criteria, and (b) including the segment in the second subset if the data satisfies the criteria. This includes comparing a copy number of the segment to a copy number of segments included in the first subset of segments (e.g., a copy number of the arm-level segments). Additionally, or alternatively, this includes comparing a length of the segment to a threshold length and/or predetermined range. Techniques for identifying a second subset of segments are described herein including at least with respect to FIG. 1B, process 360 shown in FIG. 3C, and the example shown in FIG. 4.

At act 308, a proportion is determined based on the second subset of segments and the chromosome arms associated with segments in the first subset of segments. Determining the proportion includes (a) determining the number of segments included in the second subset of segments identified at act 306, and (b) determining the number of chromosome arms associated with segments included in the first subset identified at act 304. The proportion is the proportion of the number of segments included in the second subset to the number of chromosome arms associated with segments in the first subset. An example of determining a proportion of segments to chromosome arms is described herein including at least with respect to FIG. 4.

At act 310, the proportion determined at act 308 is used to determine whether a biological sample includes cells having HRD. In some embodiments, the proportion may be determined using any suitable techniques for determining whether the biological sample includes cells having HRD, as aspects of the technology described herein are not limited in this respect. For example, in some embodiments, the proportion or a value derived therefrom may be used to determine a score. For example, the score may be the proportion itself or the proportion in combination with one or more other predictors (e.g., ploidy). When the score is a combination of predictors (e.g., combination of the determined proportion and ploidy), coefficients may be determined for each of the predictors and used to determine a weighted sum of the predictors. For example, coefficients may be estimated by performing a regression analysis. The regression analysis may be performed using data that includes, for each of a plurality of training subjects, values for each of the predictors and a known HRD status for the subject. In some embodiments, the score is compared to a threshold to determine the HRD status. For example, if the score is greater than or equal to the threshold, then the biological sample is determined to include cells having HRD. The threshold may be determined based on results of performing a regression analysis (e.g., the regression analysis used to estimate the coefficient(s)). For example, performance metrics (e.g., F1 score, positive predictive value, negative predictive value, etc.) used for evaluating the performance of the regression analysis in distinguishing between HRD positive and/or HRD negative subjects may be used to determine the threshold.

The proportion is processed using a statistical model to obtain an output indicating whether the biological sample includes cells having HRD. The statistical model may include any suitable statistical model used to predict whether a biological sample includes cells having HRD such as, for example, a generalized linear model (e.g., a linear regression model, a logistic regression model, a probit regression model, etc.). It should be appreciated that, in some embodiments, the statistical model may not be a generalized linear model and may be a different type of statistical model such as, for example, a random forest regression model, a neural network, a support vector machine, a Gaussian mixture model, a hierarchical Bayesian model, and/or any other suitable statistical model, as aspects of the technology described herein are not limited to using generalized linear models for the prediction of HRD status.

In some embodiments, the statistical model is a regression model and has a regression variable for the proportion determined at act 308. In some embodiments, the statistical model includes a coefficient for the proportion regression value. In some embodiments, the coefficient is estimated using (a) proportions determined for training biological samples, (b) (optionally) values obtained for one or more other regression variables, and (c) information indicating which of the training biological samples included cells having HRD and/or which of the training biological samples did not include cells having HRD.

In some embodiments, the statistical model (optionally) has one or more other regression variables for one or more other predictors (not shown). For example, the statistical model may include regression variables for ploidy of the biological sample, genome-wide loss-of-heterozygosity (gwLOH), aneuploidy, and/or any other suitable predictor. In some embodiments, the statistical model is regularized. For example, regularization techniques may be used when the statistical model includes more than one predictor. The statistical model may be regularized using any suitable regularization techniques such as, for example, L1 and/or L2 regularization.

In some embodiments, training the statistical model includes determining whether or not to include a regression variable for a particular predictor. This may be done in any suitable way and, for example, may be done by iteratively adding regression variables for respective predictors to the statistical model. In some embodiments, iteratively adding regression variables includes: identifying a candidate predictor (e.g., proportion, ploidy, gwLOH, aneuploidy, etc.); augmenting a current statistical model with a regression variable for the candidate predictor to obtain an augmented statistical model; evaluating performance of the augmented statistical model (e.g., by calculating the area under a receiver operating characteristic (ROC) curve statistic or in any other suitable way); and determining to add the regression variable for the candidate predictor to the current statistical model based on results of evaluating the performance (e.g., improved performance relative to the performance of the current model). For example, the performance of the statistical model may be evaluated using predictive accuracy, sensitivity, specificity, precision, F1 score, area under the receiver operating characteristic (ROC) curve, and/or any other suitable metric for evaluating the performance of a statistical model, as aspects of the technology described herein are not limited in this respect.

In some embodiments, the statistical model is one of multiple statistical models, each of which is trained to predict HRD statuses for subjects having, suspected of having, or at risk of having a respective cancer type. As a nonlimiting example, one such statical model may be trained to predict an HRD status for subjects having, suspected of having, or at risk of having breast cancer, while a different statistical model may be trained to predict an HRD status for subjects having, suspected of having, or at risk of having ovarian cancer. The statistical model may be trained for any suitable type of cancer, as aspects of the technology are not limited in this respect. Nonlimiting examples of such cancer types include breast cancer, ovarian cancer, pancreatic cancer, prostate cancer, bladder cancer, and colorectal cancer.

In some embodiments, statistical models trained for different types of cancers may have regression variable(s) for different predictor(s). As one nonlimiting example, a statistical model trained to predict an HRD status for a subject having, suspected of having, or at risk of having ovarian cancer may include a regression variable for the proportion determined at act 308, while a statistical model trained to predict an HRD status for a subject having, suspected of having, or at risk of having breast cancer may include regression variables for both the proportion determined at act 308, as well as ploidy of the biological sample obtained from the subject.

In some embodiments, cancer type-specific data is used estimate coefficients for a statistical model trained to predict an HRD status for a subject having a particular type of cancer. For example, the coefficients may be estimated using (a) proportions determined for biological samples having the particular type of cancer and (b) (optionally) values for other regression variables obtained for the biological samples having the particular type of cancer, and (d) information indicating which of the biological samples included cells having HRD and/or which of the biological samples did not include cells having HRD.

In some embodiments, a statistical model is trained to predict HRD statuses for subjects having, suspected of having, or at risk of having breast cancer. In some embodiments, the statistical model may be trained using breast cancer-specific training data. The breast cancer-specific training data may include or consist of data derived from patients diagnosed with breast cancer. In some embodiments, the breast cancer-specific training data may include (a) proportions determined for breast cancer samples (e.g., samples from patients diagnosed with breast cancer), (b) (optionally) values for other regression variables (e.g., ploidy) obtained for the breast cancer samples, and (d) information indicating which of the breast cancer samples include cells having HRD and/or which of the breast cancer samples do not include cells having HRD. In some embodiments, the statistical model is trained exclusively using the breast cancer-specific training data. In other embodiments, the statistical model is trained using breast cancer-specific training data in addition to training data for one or more other cancer types (e.g., data derived from patients diagnosed with the one or more other cancer types). For example, the training data may include the data for one or more other cancer types to allow for the possibility that the training data is aggregated across different cancer types.

In some embodiments, a statistical model is trained to predict HRD statuses for subjects having, suspected of having, or at risk of having ovarian cancer. In some embodiments, the statistical model may be trained using ovarian cancer-specific training data. The ovarian cancer-specific training data may include or consist of data derived from patients diagnosed with ovarian cancer. In some embodiments, the ovarian cancer-specific training data may include (a) proportions determined for ovarian cancer samples (e.g., samples from patients diagnosed with ovarian cancer), (b) (optionally) values for other regression variables (e.g., ploidy) obtained for the ovarian cancer samples, and (d) information indicating which of the ovarian cancer samples include cells having HRD and/or which of the ovarian cancer samples do not include cells having HRD. In some embodiments, the statistical model is trained exclusively using the ovarian cancer-specific training data. In other embodiments, the statistical model is trained using ovarian cancer-specific training data in addition to training data for one or more other cancer types (e.g., data derived from patients diagnosed with the one or more other cancer types). For example, the training data may include the data for one or more other cancer types to allow for the possibility that the training data is aggregated across different cancer types.

In some embodiments, a statistical model is trained to predict HRD statuses for subjects having, suspected of having, or at risk of having pancreatic cancer. In some embodiments, the statistical model may be trained using pancreatic cancer-specific training data. The pancreatic cancer-specific training data may include or consist of data derived from patients diagnosed with pancreatic cancer. In some embodiments, the pancreatic cancer-specific training data may include (a) proportions determined for pancreatic cancer samples (e.g., samples from patients diagnosed with pancreatic cancer), (b) (optionally) values for other regression variables (e.g., ploidy) obtained for the pancreatic cancer samples, and (d) information indicating which of the pancreatic cancer samples include cells having HRD and/or which of the pancreatic cancer samples do not include cells having HRD. In some embodiments, the statistical model is trained exclusively using the pancreatic cancer-specific training data. In other embodiments, the statistical model is trained using pancreatic cancer-specific training data in addition to training data for one or more other cancer types (e.g., data derived from patients diagnosed with the one or more other cancer types). For example, the training data may include the data for one or more other cancer types to allow for the possibility that the training data is aggregated across different cancer types.

In some embodiments, a statistical model is trained to predict HRD statuses for subjects having, suspected of having, or at risk of having prostate cancer. In some embodiments, the statistical model may be trained using prostate cancer-specific training data. The prostate cancer-specific training data may include or consist of data derived from patients diagnosed with prostate cancer. In some embodiments, the prostate cancer-specific training data may include (a) proportions determined for prostate cancer samples (e.g., samples from patients diagnosed with prostate cancer), (b) (optionally) values for other regression variables (e.g., ploidy) obtained for the prostate cancer samples, and (d) information indicating which of the prostate cancer samples include cells having HRD and/or which of the prostate cancer samples do not include cells having HRD. In some embodiments, the statistical model is trained exclusively using the prostate cancer-specific training data. In other embodiments, the statistical model is trained using prostate cancer-specific training data in addition to training data for one or more other cancer types (e.g., data derived from patients diagnosed with the one or more other cancer types). For example, the training data may include the data for one or more other cancer types to allow for the possibility that the training data is aggregated across different cancer types.

In some embodiments, a statistical model is trained to predict HRD statuses for subjects having, suspected of having, or at risk of having bladder cancer. In some embodiments, the statistical model may be trained using bladder cancer-specific training data. The bladder cancer-specific training data may include or consist of data derived from patients diagnosed with bladder cancer. In some embodiments, the bladder cancer-specific training data may include (a) proportions determined for bladder cancer samples (e.g., samples from patients diagnosed with bladder cancer), (b) (optionally) values for other regression variables (e.g., ploidy) obtained for the bladder cancer samples, and (d) information indicating which of the bladder cancer samples include cells having HRD and/or which of the bladder cancer samples do not include cells having HRD. In some embodiments, the statistical model is trained exclusively using the bladder cancer-specific training data. In other embodiments, the statistical model is trained using bladder cancer-specific training data in addition to training data for one or more other cancer types (e.g., data derived from patients diagnosed with the one or more other cancer types). For example, the training data may include the data for one or more other cancer types to allow for the possibility that the training data is aggregated across different cancer types.

In some embodiments, a statistical model is trained to predict HRD statuses for subjects having, suspected of having, or at risk of having colorectal cancer. In some embodiments, the statistical model may be trained using colorectal cancer-specific training data. The colorectal cancer-specific training data may include or consist of data derived from patients diagnosed with colorectal cancer. In some embodiments, the colorectal cancer-specific training data may include (a) proportions determined for colorectal cancer samples (e.g., samples from patients diagnosed with colorectal cancer), (b) (optionally) values for other regression variables (e.g., ploidy) obtained for the colorectal cancer samples, and (d) information indicating which of the colorectal cancer samples include cells having HRD and/or which of the colorectal cancer samples do not include cells having HRD. In some embodiments, the statistical model is trained exclusively using the colorectal cancer-specific training data. In other embodiments, the statistical model is trained using colorectal cancer-specific training data in addition to training data for one or more other cancer types (e.g., data derived from patients diagnosed with the one or more other cancer types). For example, the training data may include the data for one or more other cancer types to allow for the possibility that the training data is aggregated across different cancer types.

The output of the statistical model is indicative of whether the biological sample includes cells having HRD (i.e., the HRD status for the subject). For example, the output may be a probability that the biological sample includes cells having HRD. Additionally, or alternatively, the output may be a score indicative of whether the biological sample includes cells having HRD. For example, if the score is greater than or equal to a threshold, this may indicate that the biological sample includes cells having HRD. If the output indicates that the biological sample includes cells having HRD, then the subject is determined to be HRD-positive. If the output indicates that the biological sample does not include cells having HRD, then the subject is determined to be HRD-negative.

At act 312, a therapy is identified for the subject based on the output indicating whether the biological sample includes cells having HRD. For example, HRD-positive subjects have been shown to be responsive to certain therapies such as, for example, platinum-based therapeutic agents and PARP inhibitors. If the output indicates that the biological sample includes cells having HRD, then a platinum-based therapeutic agent and/or PARP inhibitor may be identified as therapy for the subject. The therapy may be identified according to embodiments described herein including with respect to the "Therapies" section.

At act 314, the therapy is optionally administered to the subject. For example, the therapy may be administered by a healthcare provider treating the subject. The therapy may be administered according to embodiments described herein including with respect to the "Therapies" section.

It should be appreciated that process 300 may include one or more additional or alternative acts not shown in FIG. 3A. For example, process 300 may include an act for obtaining sequencing data from which the segment data is obtained at act 302. Additionally, or alternatively, process 300 may include only a subset of the acts shown in FIG. 3A. For example, process 300 may include all of the acts 302-314, or only some of the acts including acts 302-312; 302-310; 308-314; 308-312; 308-312, or any other suitable combination of acts.

FIG. 3B is a flowchart of an illustrative process 340 for identifying a first subset of segments of a subject's genome, according to some embodiments of the technology described herein. One or more acts of process 340 may be performed automatically by any suitable computing device(s). For example, the act(s) may be performed by a laptop computer, a desktop computer, one or more servers, in a cloud computing environment, computing device 1700 as described herein with respect to FIG. 17, and/or in any other suitable way.

At act 342, segment data is obtained for a first segment. In some embodiments, the segment data includes at least some of the segment data obtained at act 302 of process 300 shown in FIG. 3A. The segment data obtained for the first segment may include at least (a) a length of the first segment, (b) a copy number of the first segment, and (c) a chromosome arm associated with the first segment. Additionally, the segment data obtained at act 342 may include ploidy of at least some cells (e.g., tumor cells) in the biological sample.

At act 344, the length of the first segment is compared to a first threshold length to determine whether the length is greater than or equal to the first threshold length. In some embodiments, the first threshold length depends on the chromosome arm associated with the segment. For example, the first threshold length may be between 20% and 100% of the length of the chromosome arm, between 25% and 75% of the length of the chromosome arm, between 30% and 70% of the length of the chromosome arm, between 35% and 65% of the length of the chromosome arm, or any other suitable length. Additionally, or alternatively, the first threshold length may be at least 20% of the length of the chromosome arm, at least 25% of the length of the chromosome arm, at least 30% of the length of the chromosome arm, at least 35% of the length of the chromosome arm, at least 40% of the length of the chromosome arm, at least 60% of the length of the chromosome arm, or any other suitable length. As another example, the first threshold length may be at most 100% of the length of the chromosome arm, at most 95% of the length of the chromosome arm, at most 90% of the length of the chromosome arm, at most 80% of the length of the chromosome arm, at most 75% of the length of the chromosome arm, at most 70% of the length of the chromosome arm, or any other suitable length. It should be appreciated that any of the above-listed upper boundaries may be listed with any of the above-listed lower boundaries. As one example, the first threshold length may be 40% of the length of the chromosome arm associated with the first segment.

If the length of the first segment is determined to be equal to or greater than the first threshold length at act 344, then process 340 proceeds to act 346, else process 340 proceeds to act 348.

At act 346, the copy number of the first segment is compared to ploidy of cells (e.g., tumor cells) of the biological sample to determine whether the copy number of the first segment equals ploidy of the biological sample. If it is determined that the copy number of the first segment equals ploidy of the biological sample, then process 340 proceeds to act 352, else process 340 proceeds to act 348.

At act 348, process 340 includes determining whether the first segment is one of a group of segments including fewer than or equal to a threshold number of segments. In some embodiments, the threshold number of segments includes any suitable number of segments such as, a number of segments between 1 and 10 segments, between 2 and 6 segments, or any other suitable number of segments. Additionally, or alternatively, the threshold number of segments may be at most 8 segments, at most 7 segments, at most 6 segments, at most 5 segments, at most 4 segments, at most 3 segments, at most 2 segments, or any other suitable number of segments. Additionally, or alternatively, the threshold number of segments may be at least one segment, at least 2 segments, or any other suitable number of segments. It should be appreciated that any of the above-listed upper boundaries may be listed with any of the above-listed lower boundaries. As one example, the threshold number of segments may be 4 segments. In some embodiments, the group of segments may be identified from among segments associated with the same chromosome arm with which the first segment is associated. In some embodiments, identifying the group of segments includes identifying one segment or identifying multiple segments having a common copy number (e.g., the same copy number as one another).

If, at act 348, the first segment is determined to be one of a group of segments including the threshold number of segments or fewer than the threshold number of segments, then process 340 proceeds to act 350, else process 340 proceeds to act 354.

At act 350, the sum of the lengths of the segments included in the group of segments (i.e., the summary length of the group of segments) is compared to a second threshold length to determine whether the summary length is greater than or equal to the second threshold length. In some embodiments, the second length threshold depends on a length of the chromosome arm with which the first segment is associated. For example, the second threshold length may be between 20% and 100% of the length of the chromosome arm, between 25% and 75% of the length of the chromosome arm, between 30% and 70% of the length of the chromosome arm, between 35% and 65% of the length of the chromosome arm, or any other suitable length. Additionally, or alternatively, the second threshold length may be at least 20% of the length of the chromosome arm, at least 25% of the length of the chromosome arm, at least 30% of the length of the chromosome arm, at least 35% of the length of the chromosome arm, at least 40% of the length of the chromosome arm, at least 60% of the length of the chromosome arm, or any other suitable length. Additionally, or alternatively, the second threshold length may be at most 100% of the length of the chromosome arm, at most 95% of the length of the chromosome arm, at most 90% of the length of the chromosome arm, at most 80% of the length of the chromosome arm, at most 75% of the length of the chromosome arm, at most 70% of the length of the chromosome arm, or any other suitable length. It should be appreciated that any of the above-listed upper boundaries may be listed with any of the above-listed lower boundaries. The second length threshold may be the same as or different from the first length threshold. As one example, the first threshold length may be 50% of the length of the chromosome arm associated with the first segment.

If the summary length is determined to be greater than or equal to the second threshold length, then process 340 proceeds to act 352, else process 340 proceeds to act 354.

At act 352, the first segment is included in the first subset of segments. As shown in FIG. 3B, the first segment is included in the first subset of segments if (a) it satisfies the criteria at both act 344 and act 346, or (b) it satisfies the criteria at both act 348 and 350.

In some embodiments, one or more acts of process 340 may be repeated for each of multiple segments. For example, process 340 may be repeated for each of at least some segments for which segment data was obtained at act 302 of process 300 shown in FIG. 3A. At act 354, process 340 includes determining whether there is another segment (e.g.,

a second segment). If it is determined that there is another segment, then process 340 returns to act 342, which is repeated for the next segment, along with one or more of acts 344, 346, 348, 350, and 352.

It should be appreciated that one or more of act 344, act 346, act 348, and act 350 may be performed in any order. For example, act 346 may precede act 344. Additionally, or alternatively, act 348 and act 350 may precede one or both of act 344 and act 346. Alternatively, two or more of act 344, act 346, act 348, and act 350 may be performed in parallel with one another. For example, acts 344, 346, and 348 may be performed in parallel.

Furthermore, it should be appreciated that one or more of acts 344, 346, 348, and 350 may be excluded from process 340 depending on result(s) of performing one or more of the acts. For example, if (a) at act 344, it is determined that the length of the first segment is greater than or equal to the first threshold length, and (b) at act 346, it is determined that the copy number of the first segment equals ploidy of at least some cells (e.g., tumor cells) of the biological sample, then act 348 and act 350 may be excluded from process 340. Alternatively, if act 348 and act 350 are performed prior to one or both of acts 344 and 346, and it is determined that the first segment is one of less than or equal to a threshold number of segments having a summary length greater than or equal to the second threshold length, then one or both of acts 344 and 346 may be excluded from process 340.

FIG. 3C is a flowchart of a process 360 for identifying a second subset of segments of a subject's genome, according to some embodiments of the technology described herein. One or more acts of process 360 may be performed automatically by any suitable computing device(s). For example, the act(s) may be performed by a laptop computer, a desktop computer, one or more servers, in a cloud computing environment, computing device 1700 as described herein with respect to FIG. 17, and/or in any other suitable way.

At act 362, segment data is obtained for a first segment. In some embodiments, the segment data includes at least some of the segment data obtained at act 302 of process 300 shown in FIG. 3A. The segment data obtained for the first segment may include at least (a) a length of the first segment, (b) a copy number of the first segment, and (c) a chromosome arm associated with the first segment.

At act 364, a length of the first segment is compared to a predetermined length criterion to determine whether the length satisfies the predetermined length criterion. In some embodiments, the length criterion depends on the chromosome arm associated with the first segment. In some embodiments, the length criterion is a predetermined range, and the first segment satisfies the length criterion if a length of the segment falls within the predetermined range. For example, the predetermined range may be a range between 1 Mb and 30% of the length of the chromosome arm associated with the segment, between 2 Mb and 20% of the length of the chromosome arm associated with the segment, between 3 Mb and 10% of the length of the chromosome arm associated with the segment, 4 Mb and 5% of the length of the chromosome arm, or any other suitable predetermined range. Additionally, or alternatively, in some embodiments, the length criterion is a threshold length of at least 1 Mb, at least 2 Mb, at least 3 Mb, at least 4 Mb, at least 5% of the length of the chromosome arm, at least 10% of the length of the chromosome arm, at least 20% of the length of the chromosome arm, at least 30% of the length of the chromosome arm, or any other suitable threshold length. Additionally, or alternatively, the length criterion may be a threshold length of at most 30% of the length of the chromosome arm, at most 20% of the length of the chromosome arm, at most 10% of the length of the chromosome arm, at most 5% of the length of the chromosome arm, or any other suitable threshold length. It should be appreciated that any of the above-listed upper boundaries may be listed with any of the above-listed lower boundaries. If it is determined that the length of the first segment satisfies the predetermined length criterion at act 364, then process 360 proceeds to act 366, else process 340 proceeds to act 370.

At act 366, the copy number of the first segment is compared to a copy number of arm-level segments of the chromosome arm with which the first segment is associated to determine whether the copy number of the first segment differs from the copy number of the arm-level segments. In some embodiments, arm-level segments of the chromosome arm associated with the first segment may include the segments identified as belonging to the first subset of segments (e.g., using process 340 shown in FIG. 3B) that are associated with the same chromosome arm with which the first segment is associated. In some embodiments, the copy number of the arm-level segments of the chromosome arm is included in segment data obtained for the arm-level segments (e.g., the segment data obtained at act 302 of process 300 in FIG. 3A and the segment data obtained at act 342 of process 340 shown in FIG. 3B).

If it is determined that the copy number of the first segment differs from that of the arm-level segments, then process 360 proceeds to act 368, else process 360 proceeds to act 370.

At act 368, the first segment is included in the second subset of segments. As shown in FIG. 3C, the first segment is included in the second subset of segments if it satisfies both the criteria at act 364 and the criteria at act 366.

In some embodiments, one or more acts of process 360 may be repeated for each of multiple segments. For example, process 360 may be repeated for each of at least some segments for which segment data was obtained at act 302 of process 300 shown in FIG. 3A. At act 370, process 360 includes determining whether there is another segment (e.g., a second segment). If it is determined that there is another segment, then process 360 returns to act 362, which is repeated for the next segment, along with one or more of acts 364, 366, and 368.

It should be appreciated that act 364 and act 366 may be performed in any order. For example, act 364 may be performed prior to act 366, or act 366 may be performed prior to act 364. Alternatively, act 364 and act 366 may be performed in parallel with one another.

Furthermore, it should be appreciated that one of act 364 and act 366 may be excluded from process 360 depending on result(s) of performing one or more of the acts. For example, if act 364 is performed prior to act 366, and it is determined, at act 364, that the length of the first segment does not satisfy the predetermined length criteria, then act 366 may be excluded from process 360. Alternatively, if act 366 is performed prior to act 364, and it is determined, at act 366, that the copy number of the first segment equals the copy number of the arm-level segments, then act 364 may be excluded from process 360.

**FIG. 4** is an example of determining a proportion of a number of segments of the subject's genome to a number of chromosome arms, according to some embodiments of the technology described herein.

As shown in FIG. 4, segment data 400 is used to determine proportion 450. The segment data 400 includes segment data 415 for segments of chromosome arm 410, segment data 425 for segments of chromosome arm 420, and segment data 435 for segments of chromosome arm 430.

The segment data 415, segment data 425, and segment data 435 is used to identify a first subset of segments (i.e., arm-level segments) and a second subset of segments (i.e., long-focal segments). As indicated in legend 440, segments identified for inclusion in the first subset of segments include segment 415-1, segment 425-1, segment 425-3, segment 425-5, segment 435-1, and segment 435-3. Segments identified for inclusion in the second subset of segments include segment 415-2, segment 415-3, segment 425-2, segment 425-4, and segment 435-2. Other segments, which not identified for inclusion in either of the subsets, are shaded in grey.

Process 340 shown in FIG. 3B may be used to identify the first subset of segments. For the purpose of this example, the following criteria may be used to identify the first subset. First, a segment is included in the first subset of segments if (a) the length of the segment is greater than or equal to 40% of the length of the chromosome arm with which it is associated (e.g., first threshold length), and (b) the copy number of the segment is equal to ploidy of the tumor cells of the biological sample. Alternatively, a segment is included in the first subset of segments if (a) it is one of a group of segments having fewer than or equal to 4 segments (e.g., threshold number of segments), and (b) the sum of the lengths of segments (i.e., summary length of the segments) included in the group of segments is greater than or equal to 50% of the length of the chromosome arm with which the segment is associated (e.g., second threshold length).

First, consider segment data 415. The segment 415-1 was identified for inclusion in the first subset. The segment 415-1 has a length (75 Mb) that is greater than 40% of 90 Mb (the length of chromosome arm 410) and has a copy number (2) equal to ploidy of the tumor cells of the biological sample. Additionally, the segment 415-1 is a single segment (e.g., less than or equal to 4 segments) with a summary length (75 Mb) greater than 50% of the length of the chromosome arm 410. Therefore, the segment 415-1 is identified for inclusion in the first subset of segments.

Second, consider segment data 425. The segment 425-1, the segment 425-3, and the segment 425-5 were each identified for inclusion in the first subset. The segment 425-1 has a length (52 Mb) that is greater than 40% of the length of chromosome arm 420 (110 Mb) and a copy number (2) equal to ploidy of the tumor cells of the biological sample. Therefore, it is identified for inclusion in the first subset of segments. The segments 425-3 and 425-5, having lengths 20 Mb and 19 Mb respectively, do not have lengths that are greater than or equal to 40% of the length of the chromosome arm 420. However, the three segments (e.g., segments 425-1, 425-3, and 425-5) may be considered as a whole, since three is fewer than the threshold of four segments, and all three segments have the same copy number. The summary length of the three segments is 91 Mb, which is greater than 50% of the length of the chromosome arm 420. Therefore, all three of the segments 425-1, 425-3, and 425-5 are identified for inclusion in the first subset.

Third, consider segment data 435. The segment 435-1 and the segment 435-3 were identified for inclusion in the first subset. The segment 435-1 has a length (67 Mb) that is greater than 40% of the length of the chromosome arm 430 (150Mb) and a copy number (2) that is equal to ploidy of the tumor cells of the biological sample. Therefore, it is identified for inclusion in the first subset of segments. The segment 435-3 does not have a length (55 Mb) that is greater than or equal to 40% of the length of the chromosome arm 430. However, the two segments (having lengths 67 Mb and 55 Mb) may be considered as a whole, since two is fewer than the threshold of four segments, and the two segments have the same copy number. The summary length of the two segments is 122 Mb, which is greater than 50% of the length of the chromosome arm 430. Therefore, both segments 435-1 and 435-3 are identified for inclusion in the first subset.

Process 360 shown in FIG. 3C may be used to identify the second subset of segments. For the purpose of this example, the following criteria may be used. A segment is included in the second subset of segments if (a) it has a length between 3 Mb and 10% the length of the chromosome arm with which it is associated (e.g., predetermined length criterion), and (b) it has a copy number that differs from the arm-level segments of the chromosome arm with which it is associated.

First, consider segment data 415. Segment 415-2 and segment 415-3 were identified for inclusion in the second subset of segments. The segment 415-2 has a length (5 Mb) that is between 3 Mb and 9 Mb (10% of the length of chromosome arm 410) and has a copy number (3) that differs from the copy number (2) of the arm-level segments (shaded in black in segment data 415). The segment 415-3 has a length (8 Mb) that is between 3 Mb and 9 Mb and has a copy number (1) that differs from the copy number of the arm-level segments, which is 2. Therefore, both segment 415-2 and segment 415-3 are identified for inclusion in the second subset of segments.

Second, consider segment data 425. Segment 425-2 and segment 425-4 were identified for inclusion in the second subset of segments. Both segments have a length between 3 Mb and 11 Mb (10% of the length of chromosome arm 425) and both have copy numbers (4 and 3) that differ from the copy number (2) of the arm-level segments (shaded in black in segment data 425). Therefore, both segment 425-2 and segment 425-4 are identified for inclusion in the second subset of segments.

Third, consider segment data 435. The segment 435-2 was identified for inclusion in the second subset of segments. The segment 435-2 has a length between 3 Mb and 15 Mb (10% of the length of chromosome arm 430) and a copy number (1) that differs from the copy number (2) of the arm-level segments (shaded in black in segment data 435). Therefore, the segment 435-2 is identified for inclusion in the first subset of segments.

In the example of FIG. 4, the first subset of segments and the second subset of segments are used to determine the proportion 450, which is the proportion of the number of segments included in the second subset of segments to the number of chromosome arms associated with segments included in the first subset of segments. In this example, 5 segments were identified for inclusion in the second subset of segments (2 segments from segment data 415, 2 segments from segment data 425, and 1 segment from segment data 435), and segments associated with three chromosome arms (e.g., chromosome arm 410, chromosome arm 420, and chromosome arm 430) were identified for inclusion in the first subset of segments. Therefore, proportion 450 is 5/3.

*Example: Comparison to Existing Techniques*

This example compares aspects of the techniques described herein to existing techniques for determining whether a biological sample includes cells having HRD.

Aspects of the techniques described herein include predicting an HRD status of cells in a biological sample based on the proportion of a number of chromosome segments in a second subset (long-focal segments) to a number of chromosome arms associated with segments included in a first subset (arm-level segments). Examples of techniques for determining this proportion are described herein including at least with respect to FIGs. 1B, 3A-3C, and 4. In this and the following examples, this proportion will be referred to as the "long-focal total copy number alterations (LF-tCNA)" proportion.

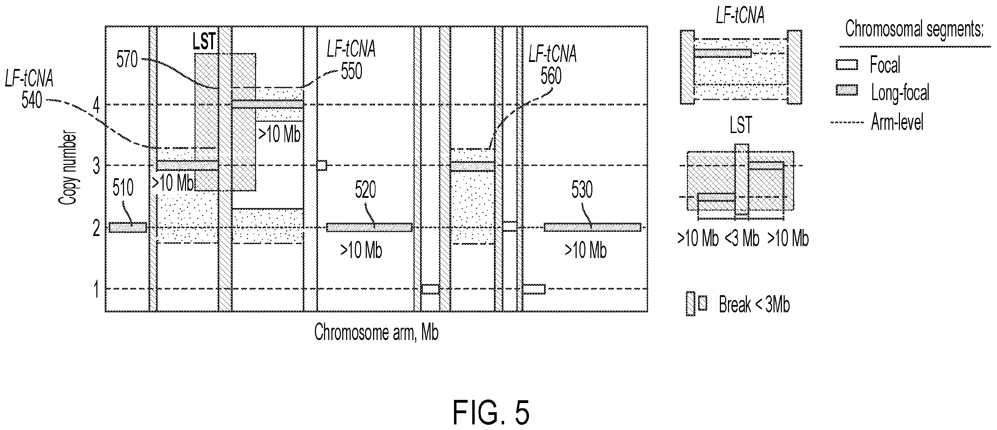

FIG. 5 shows an example of chromosome segments that are used for determining the LF-tCNA proportion. As shown, the segments include arm-level segments 510, 520, 530 of the chromosome arm (e.g., first subset of segments). The arm-level segments 510, 520, 530 have a copy number of 2. The segments also include long-focal segments 540, 550, 560 (e.g., second subset of segments) which have (a) a copy number different from the copy number of the arm-level segments 510, 520, 530, and (b) a length of at least 3 Mb or at least 10% of the length of the chromosome arm. In some embodiments, the number of segments 540, 550, 560 (e.g., three segments) and the number of chromosome arms (e.g., one chromosome arm) is used to determine the LF-tCNA proportion.

In contrast, conventional techniques determine an HRD status for cells based on a large-scale transitions (LST) score. The LST score is defined as the number of breakpoints between chromosomal segments longer than 10 Mb, after filtering out regions shorter than 3 Mb. FIG. 5 shows an example of one such breakpoint. As shown, breakpoint 570 is between chromosomal segment 540 and segment 550, each of which is longer than 10 Mb.

The criteria for identifying breakpoints for determining the LST score is stricter than the criteria for identifying chromosome segments for determining the LF-tCNA proportion. As a result, the existing techniques are less sensitive to the genomic instability resulting from HRD, which causes gene amplification and/or loss-of-heterozygosity. This is highlighted in the example of FIG. 5, in which the techniques described herein detect three instances of gene amplification, whereas the conventional techniques detect a single breakpoint. As a result the LF-tCNA proportion, determined according to the methods described herein, is a more sensitive detector of HRD than conventional LST-based techniques.

*Example: Detecting HRD in Samples Obtained from Breast Cancer Patients*

This example describes use of the techniques described herein as part of a system for determining whether biological samples obtained from breast cancer patients include cells having HRD.

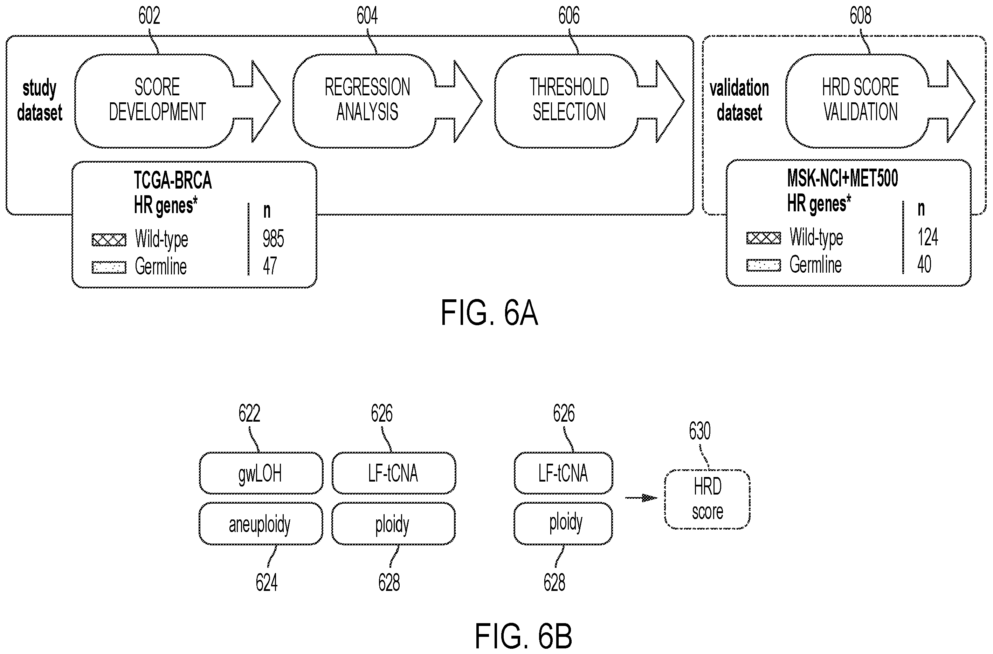

FIG. 6A is a diagram depicting an example process for training a generalized linear model to predict whether the breast cancer samples include cells having HRD. As shown, the example process includes a score development stage 602, a regression analysis stage 604, a threshold selection stage 606, and a validation stage 608. Stages 602, 604, and 606 were performed using a study data set including samples obtained from the TCGA-BRCA cohort. Stage 608 was performed using a validation data set including samples obtained from the MET500 and MSK-NCI cohorts. Both the study data set and validation data set included wild-type and germline samples. The wild-type samples included breast cancer samples with non-mutated homologous recombination genes (e.g., BRCA1, BRCA2, PALB2, and BARD1). The germline breast cancer samples included breast cancer samples with mutated homologous recombination genes and are therefore labeled as being HRD-positive.

At the score development stage 602, four candidate predictors of HRD were identified. The four predictors are shown in **FIG. 6B** and include: genome-wide loss of heterozygosity (gwLOH) 622, aneuploidy 624, LF-tCNA proportion 626, and ploidy 628. gwLOH 622 refers to the percentage of LOH regions across the whole genome and was calculated as the sum of the length of segments with a minor allele (i.e., number of copies of the least frequent allele) equal to zero, normalized to the total length of sample segments. Aneuploidy 624 refers to the average number of segments (regardless of size) with a different total copy number than a total copy number of chromosome arm-level segments. Ploidy 628 refers to the number of sets of chromosomes in cells of an organism. As described above, the LF-tCNA proportion 626 refers to the proportion of a number of long-focal chromosome segments to a number of chromosome arms associated with arm-level chromosome segments.

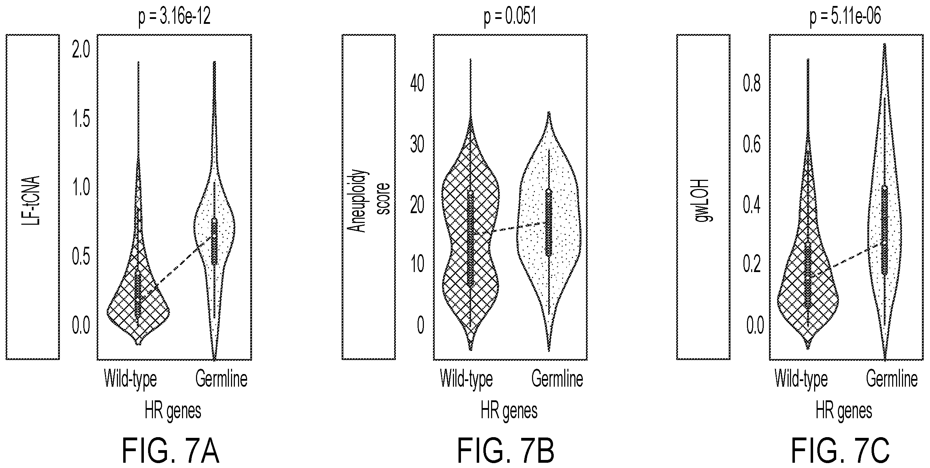

Values for the predictors were determined for samples in the study data set. **FIGS. 7A-7C** are violin plots showing the distribution of values determined for the predictors, separated by HRD status (i.e., germline versus wild-type). FIG. 7A shows the distribution of the LF-tCNA proportions determined for the wild-type samples as compared to the LF-tCNA proportions determined for the germline samples. FIG. 7B shows the distribution of aneuploidy scores determined for the wild-type samples as compared to the distribution of the aneuploidy scores determined for the germline samples. FIG. 7C shows the distribution of gwLOH values determined for the wild-type samples as compared to the distribution of gwLOH values determined for the germline samples.

As shown in the plots of FIGs. 7A-7C, the LF-tCNA proportion better distinguishes the wild-type samples from the germline samples compared to the aneuploidy score and gwLOH predictors. This is evidenced by the p-values measured for the distributions; the p-value (p = 3.16e-12) measured between the wild-type and germline distributions of LF-tCNA proportions is lower than both the p-value (p = 0.051) measured between the wild-type and germline distributions of aneuploidy scores and the p-value (p = 5.11e-6) measured between the wild-type and germline distributions of gwLOH values.

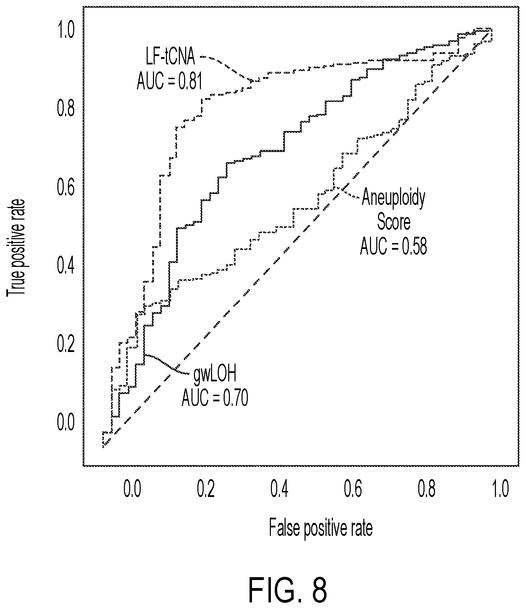

The receiver operating characteristic (ROC) curve of **FIG. 8** confirms that the LF-tCNA proportion outperformed the aneuploidy score and gwLOH as a predictor of HRD. As shown, the area under curve (AUC) for LF-tCNA proportion (AUC = 0.81) is greater than the AUC for gwLOH (AUC = 0.70) and aneuploidy score (AUC = 0.58), indicating higher predictive accuracy.

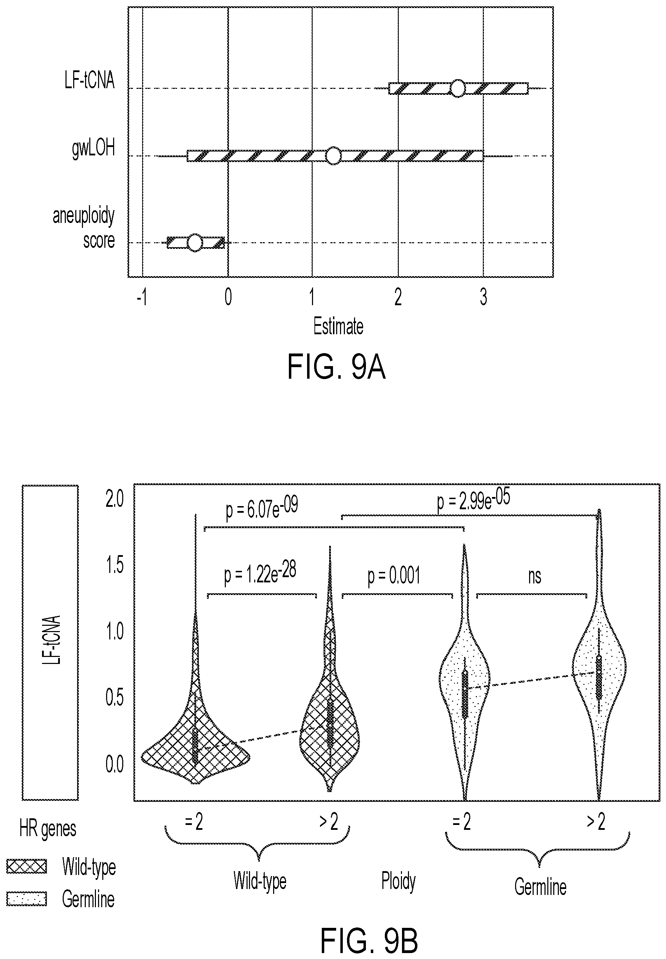

Returning to FIGs. 6A and 6B, at the regression analysis stage 604, an initial multivariate logistic regression analysis was performed with genome-wide loss of heterozygosity (gwLOH) 622, aneuploidy 624, and LF-tCNA proportion 626 as predictors. **FIG. 9A** is a coefficient plot showing results of the regression analysis. Based on the results of the regression analysis, LF-tCNA was selected as a separator between HRD-positive and HRD-negative breast cancer samples, since it resulted in the highest coefficient estimate, with a relatively low standard deviation, as compared to gwLOH and aneuploidy.

Additional analysis was conducted to determine the effect of ploidy on the LF-tCNA score. Results of this analysis are shown in the violin plots of **FIG. 9B,** which demonstrate the distribution of LF-tCNA proportions based on sample ploidy and sample type (e.g., samples with wild-type HR genes or samples with germline mutated genes). As can be inferred from the p-value (p = 1.22e-28), there is a significant difference between the distribution determined for the wild-type samples with a ploidy=2 and the distribution determined for the wild-type samples with a ploidy greater than 2, indicating that ploidy has an influence over LF-tCNA proportion. Based on these results, ploidy was also selected as a separator between HRD-positive and HRD-negative breast cancer samples.

Table 1 shows the coefficients of the regression variables in the logistic regression model.

Table 1: Example coefficients of the logistic regression model.

| Coefficient | Estimate | Standard Error | z-value | p-value |
|---|---|---|---|---|
| Intercept | -3.5088 | 0.5796 | -6.054 | -1.41e-09 |
| LF-tCNA | 2.7096 | 0.4964 | 5.459 | 4.79e-08 |
| Ploidy | -0.3684 | 0.2059 | -1.789 | 0.0735 |

In this example, the coefficients of the regression model, the LF-tCNA proportions for breast cancer samples in the study dataset, and the ploidy of samples in the study dataset were used to calculate an HRD score for each sample. For example, based on the coefficient shown in Table 1 the HRD score may be calculated using Equation 1

$$HRD\ Score = -3.5088 + (2.7096 * LFtCNA\ proportion) - (0.3684 * ploidy) \qquad \text{(Equation 1)}$$

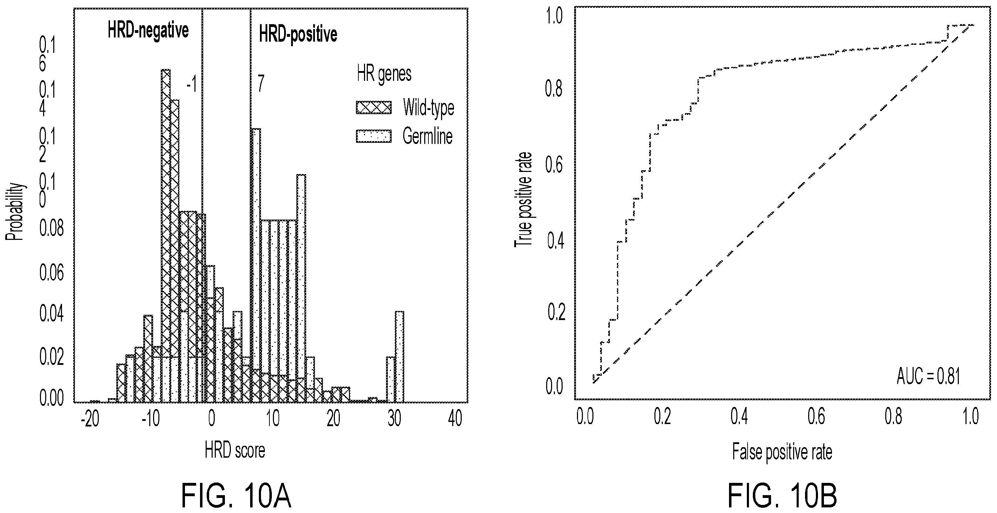

Returning to FIG. 6A, after the regression analysis stage 604, thresholds were selected during the threshold selection stage 606. In this example, the thresholds were selected based on positive predictive values and negative predictive values to separate HRD-positive (i.e., include cells having HRD), HRD-negative (i.e., they do not include cells having HRD), and HRD-ambiguous samples. In **FIG. 10A,** which shows the probability histogram of HRD scores determined for the breast cancer samples in the study dataset, dashed lines mark the selected thresholds. Samples with an HRD score greater than 10 are classified as HRD-positive, samples with an HRD score lesser than -1 are classified as HRD-negative, and samples with an HRD score between -1 and 7 are classified as HRD-ambiguous.

The receiver ROC curve of **FIG. 10B** shows the performance of the logistic regression model in classifying the breast cancer samples as HRD-positive, HRD-negative, and HRD- ambiguous. One challenge of assessing the performance of the logistic regression model is the availability of data. While some of the breast cancer samples are confirmed to be HRD-positive due to germline mutations of the homologous recombination genes, there are many factors that influence whether a sample is HRD-positive. As a result, breast cancer cells may be HRD-positive, despite not having germline mutated homologous recombination genes. In the training data, such samples may be mislabeled as being HRD-negative due to the lack of mutated homologous recombination genes. Therefore, even if the logistic regression model correctly classifies these samples as being HRD-positive, the output will be identified as a false positive due to the incorrect ground truth label. Accordingly, it is possible that the logistic regression model has an even higher performance than that shown in FIG. 10B.

Returning to FIG. 6A, after the threshold selection stage 606, the logistic regression model was validated during HRD score validation stage 608 using breast cancer samples from the MSK-NCI and MET500 cohorts. Similar to the study dataset, the validation cohorts include both samples with cells having wildtype homologous recombination genes, as well as samples with cells having germline mutated homologous recombination genes.

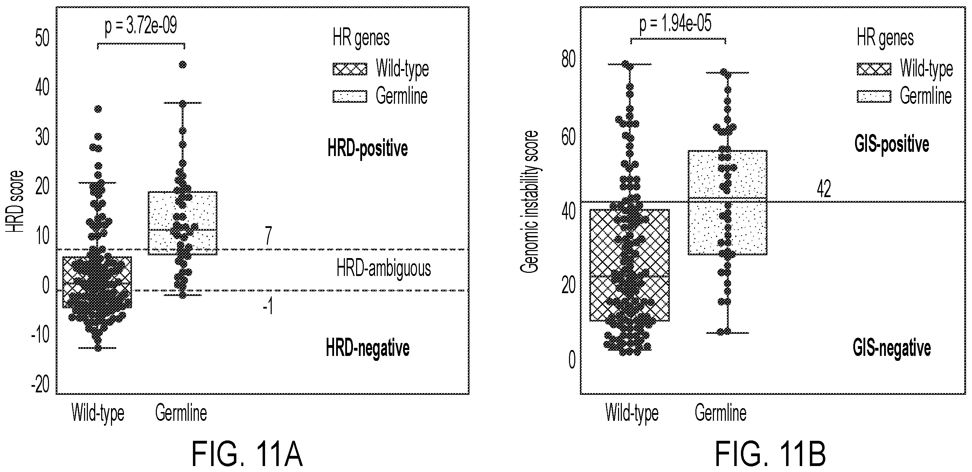

**FIG. 11A** shows boxplots showing the performance of the logistic regression model in classifying the wildtype and germline samples as HRD-positive, HRD-negative, and HRD-ambiguous. As shown, the logistic regression model accurately classifies the majority of the germline samples as HRD-positive. As explained above, it is probable that at least some of the wildtype samples are HRD-positive despite not having germline mutated homologous recombination genes. This helps to explain why many of the wildtype samples are classified as HRD-ambiguous, as opposed to HRD-negative.

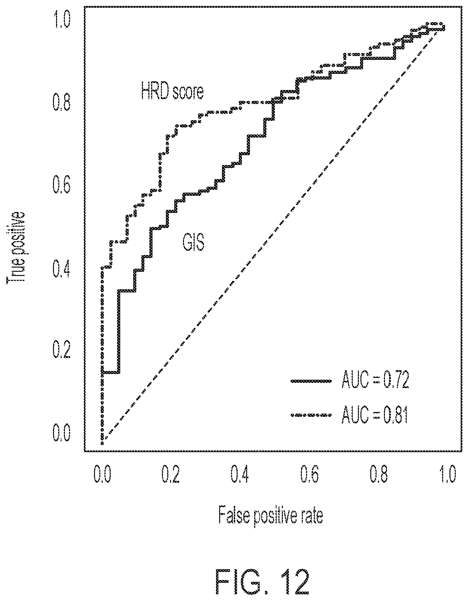

Additionally, as shown in FIGS. 11A-11C, the logistic regression model performance was compared to the performance of genomic instability score (GIS), an existing technique for determining whether a sample includes cells having HRD. GIS is based on three different factors: loss of heterozygosity (LOH), telomeric-allelic imbalance (TAI), and LST. First, the techniques developed by the inventors for predicting the HRD status of breast cancer samples are less complex than GIS, given that they involve determining values for only two variables (e.g., LF-tCNA and ploidy) as opposed to determining values for the three variables serving as the basis for GIS. Second, the techniques developed by the inventors outperformed the GIS techniques in predicting whether the breast cancer samples in the validation dataset included cells having HRD. As shown in **FIG. 11B** the GIS technique predicted that only about half of the germline mutated breast cancer samples were GIS-positive (i.e., included cells having HRD), and that most of the wildtype breast cancer samples were GIS-negative (i.e., did not include cells having HRD). As explained above, the majority of germline samples should be identified as GIS-positive, since they are confirmed to include cells having mutated homologous recombination genes. The ROC curves in **FIG. 12** also shows that the techniques developed by the inventors (AUC = 0.81) more accurately predicted HRD in the breast cancer samples compared to the GIS techniques (AUC = 0.72).

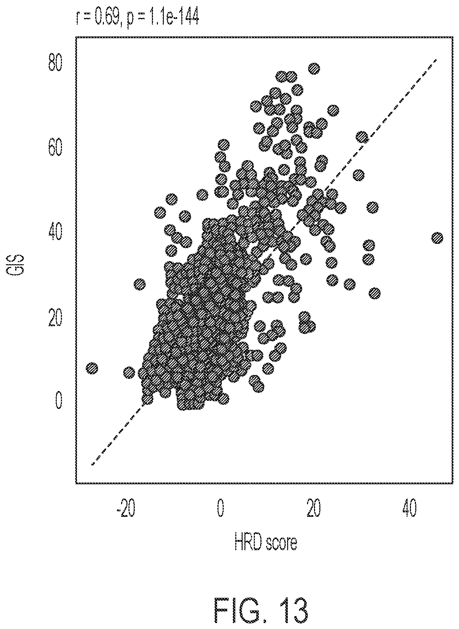

**FIG. 13** shows the Pearson correlation between HRD scores determined according to embodiments of the technology described herein and GIS scores determined according to the existing GIS techniques. The correlation is positive and significantly different from zero (r =0.69; p = 1.1e-144).

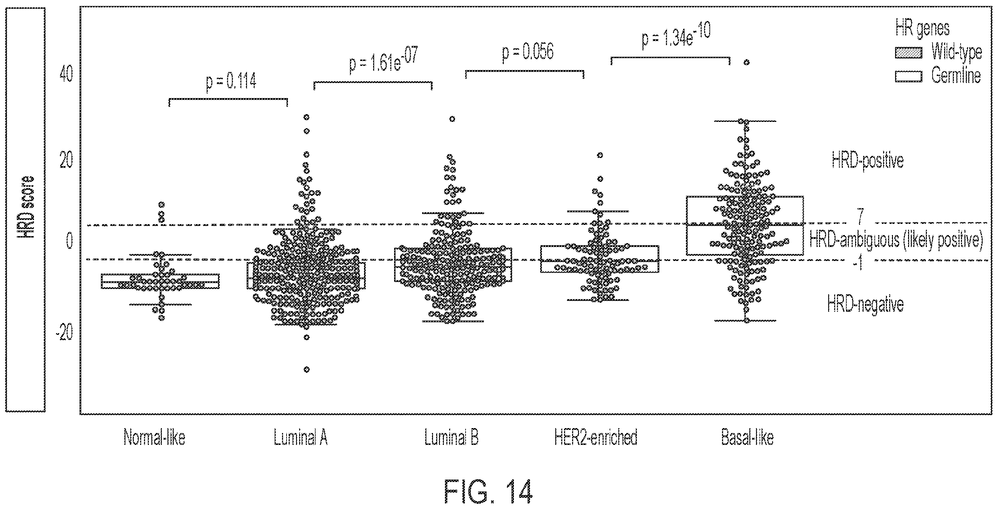

HRD scores determined for the validation dataset were further separated into breast cancer subtype. As shown in **FIG. 14,** many subjects with wildtype HR genes were classified as HRD-positive. For example, many subjects with wildtype HR genes, having Luminal A, Luminal B, HER2-enriched, and basal-like breast cancer, were classified as HRD-positive. This suggests that those subjects may respond well to certain therapies, such as PARPi or platinum-based therapies.

*Example: Detecting HRD in Samples Obtained from Ovarian Cancer Patients*

This example describes use of the techniques described herein as part of a system for determining whether biological samples obtained from ovarian cancer patients include cells having HRD.

An initial multivariate logistic regression analysis was performed to evaluate LF-tCNA and ploidy as potential predictors of HRD in ovarian cancer samples. Samples used to perform the logistic regression were selected from the TCGA-OV cohort. The samples included germline mutated samples (i.e., samples including cells having mutated homologous recombination genes (e.g., BRCA1, BRCA2, PALB2, and/or BARD1 genes)) and wildtype samples (i.e., samples that do not include cells having mutated homologous recombination genes.)

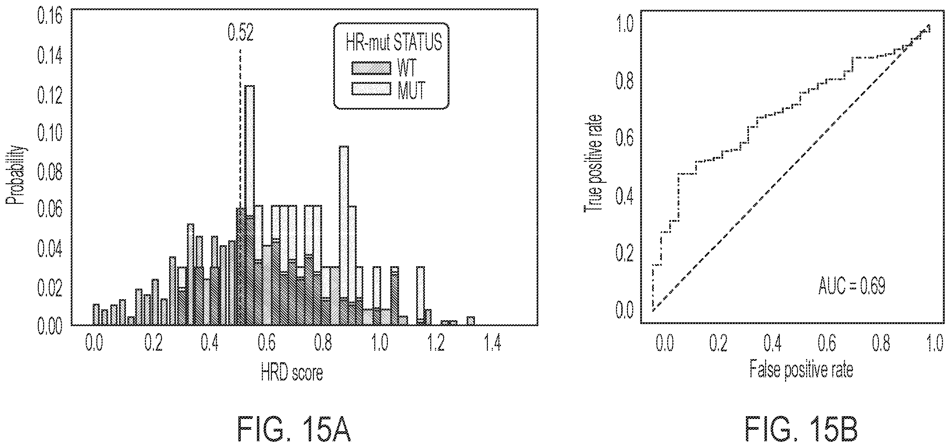

Based on results of the logistic regression analysis, the LF-tCNA proportion alone was selected as a separator between HRD-positive and HRD-negative ovarian cancer samples. Accordingly, in this example, the LF-tCNA proportion itself is used as the HRD score. **FIG. 15A** shows a probability histogram of the HRD scores. The threshold (0.52) for distinguishing between HRD-positive and HRD-negative samples, indicated by the dashed line in FIG. 15A, was selected based on the F1-score. Ovarian cancer samples with HRD scores above 0.52 were classified as HRD-positive and ovarian cancer samples with HRD scores below 0.52 were classified as HRD-negative. The ROC curve in **FIG. 15B** shows the performance of predicting HRD status with LF-tCNA as a predictor.

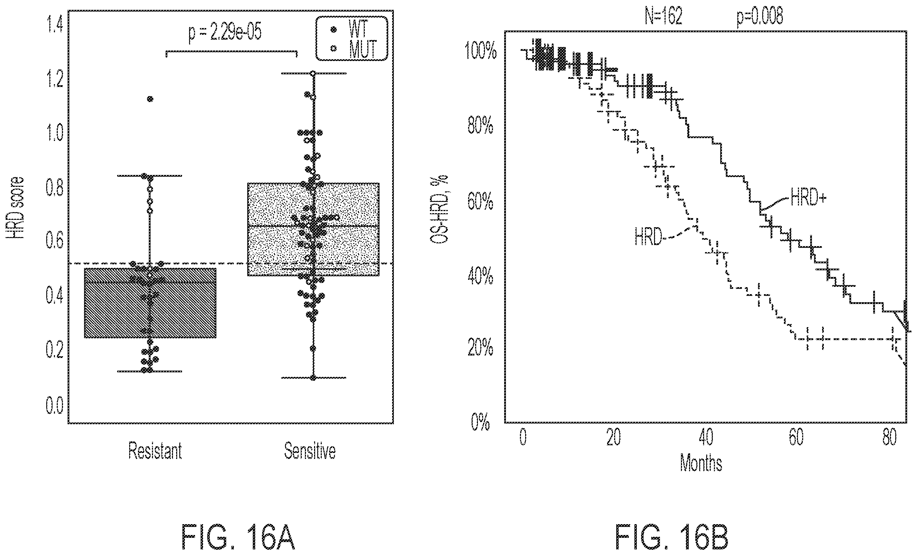

Sensitivity to platinum-based therapy has been shown to correlate with germline mutations in homologous recombination genes. To further validate the HRD scores determined for the ovarian cancer samples, they were evaluated to test how well they separated subjects sensitive and resistant to platinum-based compounds. FIGS. 16A and 16B show results of this analysis. **FIG. 16A** shows the distribution of HRD scores, separated by resistant and sensitive samples. The average HRD score for sensitive subjects was 1.47 times higher than the average HRD score for resistant subjects (p = 2.29e-5), indicating that the HRD score performs well as a predictor for platinum-based therapy sensitivity. Moreover, as shown in **FIG. 16B,** the median overall survival for HRD-positive subjects (42.3 months (95% CI: 31.1-52.0)) was significantly higher than for HRD-negative subjects (31.2 month (95% CI: 23.0-38.2)).

*Computer Implementation*

An illustrative implementation of a computer system 1700 that may be used in connection with any of the embodiments of the technology described herein (e.g., such as the method of FIGS. 3A-3C) is shown in **FIG. 17.** The computer system 1700 includes one or more processors 1710 and one or more articles of manufacture that comprise non-transitory computer-readable storage media (e.g., memory 1720 and one or more non-volatile storage media 1730). The processor 1710 may control writing data to and reading data from the memory 1720 and the non-volatile storage device 1730 in any suitable manner, as the aspects of the technology described herein are not limited to any particular techniques for writing or reading data. To perform any of the functionality described herein, the processor 1710 may execute one or more processor-executable instructions stored in one or more non-transitory computer-readable storage media (e.g., the memory 1720), which may serve as non-transitory computer-readable storage media storing processor-executable instructions for execution by the processor 1710.

Computing device 1700 may include a network input/output (I/O) interface 1740 via which the computing device may communicate with other computing devices. Such computing devices may be interconnected by one or more networks in any suitable form, including a local area network or a wide area network, such as an enterprise network, and intelligent network (IN) or the Internet. Such networks may be based on any suitable technology and may operate according to any suitable protocol and may include wireless networks, wired networks or fiber optic networks.

Computing device 1700 may also include one or more user I/O interfaces 1750, via which the computing device may provide output to and receive input from a user. The user I/O interfaces may include devices such as a keyboard, a mouse, a microphone, a display device (e.g., a monitor or touch screen), speakers, a camera, and/or various other types of I/O devices.

Further, it should be appreciated that a computer may be embodied in any of a number of forms, such as a rack-mounted computer, a desktop computer, a laptop computer, or a tablet computer, as non-limiting examples. Additionally, a computer may be embedded in a device not generally regarded as a computer but with suitable processing capabilities, including a Personal Digital Assistant (PDA), a smartphone, a tablet, or any other suitable portable or fixed electronic device.

The above-described embodiments can be implemented in any of numerous ways. For example, the embodiments may be implemented using hardware, software, or a combination thereof. When implemented in software, the software code can be executed on any suitable processor (e.g., a microprocessor) or collection of processors, whether provided in a single computing device or distributed among multiple computing devices. It should be appreciated that any component or collection of components that perform the functions described above can be generically considered as one or more controllers that control the above-described functions. The one or more controllers can be implemented in numerous ways, such as with dedicated hardware, or with general purpose hardware (e.g., one or more processors) that is programmed using microcode or software to perform the functions recited above.

In this respect, it should be appreciated that one implementation of the embodiments described herein comprises at least one computer-readable storage medium (e.g., RAM, ROM, EEPROM, flash memory or other memory technology, CD-ROM, digital versatile disks (DVD) or other optical disk storage, magnetic cassettes, magnetic tape, magnetic disk storage or other magnetic storage devices, or other tangible, non-transitory computer-readable storage medium) encoded with a computer program (i.e., a plurality of executable instructions) that, when executed on one or more processors, performs the above-described functions of one or more embodiments. The computer-readable medium may be transportable such that the program stored thereon can be loaded onto any computing device to implement aspects of the techniques described herein. In addition, it should be appreciated that the reference to a computer program which, when executed, performs any of the above-described functions, is not limited to an application program running on a host computer. Rather, the terms computer program and software are used herein in a generic sense to reference any type of computer code (e.g., application software, firmware, microcode, or any other form of computer instruction) that can be employed to program one or more processors to implement aspects of the techniques described herein.

The terms "program" or "software" are used herein in a generic sense to refer to any type of computer code or set of computer-executable instructions that can be employed to program a computer or other processor to implement various aspects as described above. Additionally, it should be appreciated that according to one aspect, one or more computer programs that when executed perform methods of the present disclosure need not reside on a single computer or processor but may be distributed in a modular fashion among a number of different computers or processors to implement various aspects of the present disclosure.

Computer-executable instructions may be in many forms, such as program modules, executed by one or more computers or other devices. Generally, program modules include routines, programs, objects, components, data structures, etc. that perform particular tasks or implement particular abstract data types. Typically, the functionality of the program modules may be combined or distributed as desired in various embodiments.

Also, data structures may be stored in computer-readable media in any suitable form. For simplicity of illustration, data structures may be shown to have fields that are related through location in the data structure. Such relationships may likewise be achieved by assigning storage for the fields with locations in a computer-readable medium that convey relationship between the fields. However, any suitable mechanism may be used to establish a relationship between information in fields of a data structure, including through the use of pointers, tags or other mechanisms that establish relationship between data elements.

When implemented in software, the software code can be executed on any suitable processor or collection of processors, whether provided in a single computer or distributed among multiple computers.

The foregoing description of implementations provides illustration and description but is not intended to be exhaustive or to limit the implementations to the precise form disclosed. Modifications and variations are possible in light of the above teachings or may be acquired from practice of the implementations. In other implementations the methods depicted in these figures may include fewer operations, different operations, differently ordered operations, and/or additional operations. Further, non-dependent blocks may be performed in parallel. It will be apparent that example aspects, as described above, may be implemented in many different forms of software, firmware, and hardware in the implementations illustrated in the figures.

*Biological Samples*

Any of the methods, systems, or other claimed elements may use or be used to analyze a biological sample from a subject. In some embodiments, a biological sample has been obtained from a subject having, suspected of having cancer, or at risk of having cancer. The biological sample may be any type of biological sample including, for example, a biological sample of a bodily fluid (e.g., blood, urine or cerebrospinal fluid), one or more cells (e.g., from a scraping or brushing such as a cheek swab or tracheal brushing), a piece of tissue (cheek tissue, muscle tissue, lung tissue, heart tissue, brain tissue, or skin tissue), or some or all of an organ (e.g., brain, lung, liver, bladder, kidney, pancreas, intestines, or muscle), or other types of biological samples (e.g., feces or hair).

In some embodiments, the biological sample is a sample of a tumor from a subject. In some embodiments, the biological sample is a sample of blood from a subject. In some embodiments, the biological sample is a sample of tissue from a subject.

A sample of a tumor, in some embodiments, refers to a sample comprising cells from a tumor. In some embodiments, the sample of the tumor comprises cells from a benign tumor, e.g., non-cancerous cells. In some embodiments, the sample of the tumor comprises cells from a premalignant tumor, e.g., precancerous cells. In some embodiments, the sample of the tumor comprises cells from a malignant tumor, e.g., cancerous cells.

Examples of tumors include, but are not limited to, adenomas, fibromas, hemangiomas, lipomas, cervical dysplasia, metaplasia of the lung, leukoplakia, carcinoma, sarcoma, germ cell tumors, sex cord-stromal tumors, neuroendocrine tumors, gastrointestinal stromal tumors, and blastoma.

A sample of blood, in some embodiments, refers to a sample comprising cells, e.g., cells from a blood sample. In some embodiments, the sample of blood comprises non-cancerous cells. In some embodiments, the sample of blood comprises precancerous cells. In some embodiments, the sample of blood comprises cancerous cells. In some embodiments, the sample of blood comprises blood cells. In some embodiments, the sample of blood comprises red blood cells. In some embodiments, the sample of blood comprises white blood cells. In some embodiments, the sample of blood comprises platelets. Examples of cancerous blood cells include, but are not limited to, leukemia, lymphoma, and myeloma. In some embodiments, a sample of blood is collected to obtain the cell-free nucleic acid (e.g., cell-free DNA) in the blood.

A sample of blood may be a sample of whole blood or a sample of fractionated blood. In some embodiments, the sample of blood comprises whole blood. In some embodiments, the sample of blood comprises fractionated blood. In some embodiments, the sample of blood comprises buffy coat. In some embodiments, the sample of blood comprises serum. In some embodiments, the sample of blood comprises plasma. In some embodiments, the sample of blood comprises a blood clot.

A sample of a tissue, in some embodiments, refers to a sample comprising cells from a tissue. In some embodiments, the sample of the tumor comprises non-cancerous cells from a tissue. In some embodiments, the sample of the tumor comprises precancerous cells from a tissue.

Methods of the present disclosure encompass a variety of tissue including organ tissue or non-organ tissue, including but not limited to, muscle tissue, brain tissue, lung tissue, liver tissue, epithelial tissue, connective tissue, and nervous tissue. In some embodiments, the tissue may be normal tissue, or it may be diseased tissue, or it may be tissue suspected of being diseased. In some embodiments, the tissue may be sectioned tissue or whole intact tissue. In some embodiments, the tissue may be animal tissue or human tissue. Animal tissue includes, but is not limited to, tissues previously obtained from rodents (e.g., rats or mice), primates (e.g., monkeys), dogs, cats, and farm animals.

The biological sample may be from any source in the subject's body including, but not limited to, any fluid [such as blood (e.g., whole blood, blood serum, or blood plasma), saliva, tears, synovial fluid, cerebrospinal fluid, pleural fluid, pericardial fluid, ascitic fluid, and/or urine], hair, skin (including portions of the epidermis, dermis, and/or hypodermis), oropharynx, laryngopharynx, esophagus, stomach, bronchus, salivary gland, tongue, oral cavity, nasal cavity, vaginal cavity, anal cavity, bone, bone marrow, brain, thymus, spleen, small intestine, appendix, colon, rectum, anus, liver, biliary tract, pancreas, kidney, ureter, bladder, urethra, uterus, vagina, vulva, ovary, cervix, scrotum, penis, prostate, testicle, seminal vesicles, breast, and/or any type of tissue (e.g., muscle tissue, epithelial tissue, connective tissue, or nervous tissue).

Any of the biological samples described herein may have been obtained from the subject using any known technique. See, for example, the following publications on collecting, processing, and storing biological samples: Biospecimens and biorepositories: from afterthought to science by Vaught et al. (Cancer Epidemiol Biomarkers Prev. 2012 Feb;21(2):253-5), and Biological sample collection, processing, storage and information management by Vaught and Henderson (IARC Sci Publ. 2011;(163):23-42).

In some embodiments, the biological sample may have been obtained from a surgical procedure (e.g., laparoscopic surgery, microscopically controlled surgery, or endoscopy), bone marrow biopsy, punch biopsy, endoscopic biopsy, or needle biopsy (e.g., a fine-needle aspiration, core needle biopsy, vacuum-assisted biopsy, or image-guided biopsy).

In some embodiments, one or more than one cell (i.e., a cell biological sample) may have been obtained from a subject using a scrape or brush method. The cell biological sample may have been obtained from any area in or from the body of a subject including, for example, from one or more of the following areas: the cervix, esophagus, stomach, bronchus, or oral cavity. In some embodiments, one or more than one piece of tissue (e.g., a tissue biopsy) from a subject may be used. In certain embodiments, the tissue biopsy may comprise one or more than one (e.g., 2, 3, 4, 5, 6, 7, 8, 9, 10, or more than 10) biological samples from one or more tumors or tissues known or suspected of having cancerous cells.

Any of the biological samples from a subject described herein may be stored using any method that preserves stability of the biological sample. In some embodiments, preserving the stability of the biological sample means inhibiting components (e.g., DNA, RNA, protein, or tissue structure or morphology) of the biological sample from degrading until they are measured so that when measured, the measurements represent the state of the sample at the time of obtaining it from the subject. In some embodiments, a biological sample is stored in a composition that is able to penetrate the same and protect components (e.g., DNA, RNA, protein, or tissue structure or morphology) of the biological sample from degrading. As used herein, degradation is the transformation of a component from one from to another such that the first form is no longer detected at the same level as before degradation.

In some embodiments, a biological sample (e.g., tissue sample) is fixed. As used herein, a "fixed" sample relates to a sample that has been treated with one or more agents or processes in order to prevent or reduce decay or degradation, such as autolysis or putrefaction, of the sample. Examples of fixative processes include but are not limited to heat fixation, immersion fixation, and perfusion. In some embodiments a fixed sample is treated with one or more fixative agents. Examples of fixative agents include but are not limited to cross-linking agents (e.g., aldehydes, such as formaldehyde, formalin, glutaraldehyde, etc.), precipitating agents (e.g., alcohols, such as ethanol, methanol, acetone, xylene, etc.), mercurials (e.g., B-5, Zenker's fixative, etc.), picrates, and Hepes-glutamic acid buffer-mediated organic solvent protection effect (HOPE) fixative. In some embodiments, a biological sample (e.g., tissue sample) is treated with a cross-linking agent. In some embodiments, the cross-linking agent comprises formalin. In some embodiments, a formalin-fixed biological sample is embedded in a solid substrate, for example paraffin wax. In some embodiments, the biological sample is a formalin-fixed paraffin-embedded (FFPE) sample. Methods of preparing FFPE samples are known, for example as described by Li et al. JCO Precis Oncol. 2018; 2: PO.17.00091.

In some embodiments, the biological sample is stored using cryopreservation. Non-limiting examples of cryopreservation include, but are not limited to, step-down freezing, blast freezing, direct plunge freezing, snap freezing, slow freezing using a programmable freezer, and vitrification. In some embodiments, the biological sample is stored using lyophilization. In some embodiments, a biological sample is placed into a container that already contains a preservant (e.g., RNALater to preserve RNA) and then frozen (e.g., by snap-freezing), after the collection of the biological sample from the subject. In some embodiments, such storage in frozen state is done immediately after collection of the biological sample. In some embodiments, a biological sample may be kept at either room temperature or 4oC for some time (e.g., up to an hour, up to 8 h, or up to 1 day, or a few days) in a preservant or in a buffer without a preservant, before being frozen.

Non-limiting examples of preservants include formalin solutions, formaldehyde solutions, RNALater or other equivalent solutions, TriZol or other equivalent solutions, DNA/RNA Shield or equivalent solutions, EDTA (e.g., Buffer AE (10 mM Tris·Cl; 0.5 mM EDTA, pH 9.0)) and other coagulants, and Acids Citrate Dextronse (e.g., for blood specimens). In some embodiments, special containers may be used for collecting and/or storing a biological sample. For example, a vacutainer may be used to store blood. In some embodiments, a vacutainer may comprise a preservant (e.g., a coagulant, or an anticoagulant). In some embodiments, a container in which a biological sample is preserved may be contained in a secondary container, for the purpose of better preservation, or for the purpose of avoid contamination.

Any of the biological samples from a subject described herein may be stored under any condition that preserves stability of the biological sample. In some embodiments, the biological sample is stored at a temperature that preserves stability of the biological sample. In some embodiments, the sample is stored at room temperature (e.g., 25 °C). In some embodiments, the sample is stored under refrigeration (e.g., 4 °C). In some embodiments, the sample is stored under freezing conditions (e.g., -20 °C). In some embodiments, the sample is stored under ultralow temperature conditions (e.g., -50 °C to -800 °C). In some embodiments, the sample is stored under liquid nitrogen (e.g., -1700 °C). In some embodiments, a biological sample is stored at -60°C to -80°C (e.g., -70°C) for up to 5 years (e.g., up to 1 month, up to 2 months, up to 3 months, up to 4 months, up to 5 months, up to 6 months, up to 7 months, up to 8 months, up to 9 months, up to 10 months, up to 11 months, up to 1 year, up to 2 years, up to 3 years, up to 4 years, or up to 5 years). In some embodiments, a biological sample is stored as described by any of the methods described herein for up to 20 years (e.g., up to 5 years, up to 10 years, up to 15 years, or up to 20 years).

Methods of the present disclosure encompass having previously obtained one or more biological samples from a subject for analysis. In some embodiments, one biological sample has been collected from a subject for analysis. In some embodiments, more than one (e.g., 2, 3, 4, 5, 6, 7, 8, 9, 10, 11, 12, 13, 14, 15, 16, 17, 18, 19, 20, or more) biological samples have been collected from a subject for analysis. In some embodiments, one biological sample from a subject will be analyzed. In some embodiments, more than one (e.g., 2, 3, 4, 5, 6, 7, 8, 9, 10, 11, 12, 13, 14, 15, 16, 17, 18, 19, 20, or more) biological samples may be analyzed. If more than one biological sample from a subject is analyzed, the biological samples may be procured at the same time (e.g., more than one biological sample may be taken in the same procedure), or the biological samples may be taken at different times (e.g., during a different procedure including a procedure 1, 2, 3, 4, 5, 6, 7, 8, 9, 10 days; 1, 2, 3, 4, 5, 6, 7, 8, 9, 10 weeks; 1, 2, 3, 4, 5, 6, 7, 8, 9, 10 months, 1, 2, 3, 4, 5, 6, 7, 8, 9, 10 years, or 1, 2, 3, 4, 5, 6, 7, 8, 9, 10 decades after a first procedure).

A second or subsequent biological sample may have been taken or obtained from the same region (e.g., from the same tumor or area of tissue) or a different region (including, e.g., a different tumor). A second or subsequent biological sample may have been taken or obtained from the subject after one or more treatments and may have been taken from the same region or a different region. As a non-limiting example, the second or subsequent biological sample may be useful in determining whether the cancer in each biological sample has different characteristics (e.g., in the case of biological samples taken from two physically separate tumors in a subject) or whether the cancer has responded to one or more treatments (e.g., in the case of two or more biological samples from the same tumor or different tumors prior to and subsequent to a treatment). In some embodiments, each of the at least one biological sample is a bodily fluid sample, a cell sample, or a tissue biopsy sample.

In some embodiments, one or more biological specimens are combined (e.g., placed in the same container for preservation) before further processing. For example, a first sample of a first tumor previously obtained from a subject may be combined with a second sample of a second tumor previously obtained from the subject, wherein the first and second tumors may or may not be the same tumor. In some embodiments, a first tumor and a second tumor are similar but not the same (e.g., two tumors in the brain of a subject). In some embodiments, a first biological sample and a second biological sample from a subject are sample of different types of tumors (e.g., a tumor in muscle tissue and brain tissue).

In some embodiments, a sample from which RNA and/or DNA is extracted (e.g., a sample of tumor, or a blood sample) is sufficiently large such that at least 2 $\mu$g (e.g., at least 2 $\mu$g, at least 2.5 $\mu$g, at least 3 $\mu$g, at least 3.5 $\mu$g or more) of DNA can be extracted from it. In some embodiments, the sample from which RNA and/or DNA is extracted can be peripheral blood mononuclear cells (PBMCs). In some embodiments, the sample from which RNA and/or DNA is extracted can be any type of cell suspension. In some embodiments, a sample from which RNA and/or DNA is extracted (e.g., a sample of tumor, or a blood sample) is sufficiently large such that at least 1.8 $\mu$g DNA can be extracted from it. In some embodiments, at least 50 mg (e.g., at least 1 mg, at least 2 mg, at least 3 mg, at least 4 mg, at least 5 mg, at least 10 mg, at least 12 mg, at least 15 mg, at least 18 mg, at least 20 mg, at least 22 mg, at least 25 mg, at least 30 mg, at least 35 mg, at least 40 mg, at least 45 mg, or at least 50 mg) of tissue sample is collected from which RNA and/or DNA is extracted. In some embodiments, at least 20 mg of tissue sample is collected from which RNA and/or DNA is extracted. In some embodiments, at least 30 mg of tissue sample is collected. In some embodiments, at least 10-50 mg (e.g., 10-50 mg, 10-15 mg, 10-30 mg, 10-40 mg, 20-30 mg, 20-40 mg, 20-50 mg, or 30-50 mg) of tissue sample is collected from which RNA and/or DNA is extracted. In some embodiments, at least 30 mg of tissue sample is collected. In some embodiments, at least 20-30 mg of tissue sample is collected from which RNA and/or DNA is extracted. In some embodiments, a sample from which RNA and/or DNA is extracted (e.g., a sample of tumor, or a blood sample) is sufficiently large such that at least 0.2 μg (e.g., at least 200 ng, at least 300 ng, at least 400 ng, at least 500 ng, at least 600 ng, at least 700 ng, at least 800 ng, at least 900 ng, at least 1 μg, at least 1.1 μg, at least 1.2 μg, at least 1.3 μg, at least 1.4 μg, at least 1.5 μg, at least 1.6 μg, at least 1.7 μg, at least 1.8 μg, at least 1.9 μg, or at least 2 μg) of DNA can be extracted from it. In some embodiments, a sample from which RNA and/or DNA is extracted (e.g., a sample of tumor, or a blood sample) is sufficiently large such that at least 0.1 μg (e.g., at least 100 ng, at least 200 ng, at least 300 ng, at least 400 ng, at least 500 ng, at least 600 ng, at least 700 ng, at least 800 ng, at least 900 ng, at least 1 μg, at least 1.1 μg, at least 1.2 μg, at least 1.3 μg, at least 1.4 μg, at least 1.5 μg, at least 1.6 μg, at least 1.7 μg, at least 1.8 μg, at least 1.9 μg, or at least 2 μg) of DNA can be extracted from it.

*Subjects*

Aspects of this disclosure relate to a biological sample that has been previously obtained from a subject. In some embodiments, a subject is a mammal (e.g., a human, a mouse, a cat, a dog, a horse, a hamster, a cow, a pig, or other domesticated animal, a farm animal (e.g., livestock), a sport animal, a laboratory animal, a pet, and a primate). In some embodiments, a subject is a human. In some embodiments, a subject is an adult human (e.g., of 18 years of age or older). In some embodiments, a subject is a child (e.g., less than 18 years of age).

In some embodiments, a human subject is one who has or has been diagnosed with at least one form of cancer. In some embodiments, a cancer from which a subject suffers is a carcinoma, a sarcoma, a myeloma, a leukemia, a lymphoma, or a mixed type of cancer that comprises more than one of a carcinoma, a sarcoma, a myeloma, a leukemia, and a lymphoma. Carcinoma refers to a malignant neoplasm of epithelial origin or cancer of the internal or external lining of the body. Sarcoma refers to cancer that originates in supportive and connective tissues such as bones, tendons, cartilage, muscle, and fat. Myeloma is cancer that originates in the plasma cells of bone marrow. Leukemias ("liquid cancers" or "blood cancers") are cancers of the bone marrow (the site of blood cell production). Lymphomas develop in the glands or nodes of the lymphatic system, a network of vessels, nodes, and organs (specifically the spleen, tonsils, and thymus) that purify bodily fluids and produce infection-fighting white blood cells, or lymphocytes. Non-limiting examples of a mixed type of cancer include adenosquamous carcinoma, mixed mesodermal tumor, carcinosarcoma, and teratocarcinoma. In some embodiments, a subject has a tumor. A tumor may be benign or malignant. In some embodiments, a cancer is any one of the following: melanoma, lung cancer, brain cancer, breast cancer, colorectal cancer, pancreatic cancer, liver cancer, skin cancer, kidney cancer, bladder cancer, ovarian cancer, cervical cancer, or prostate cancer. In some embodiments, a subject is at risk for developing cancer, e.g., because the subject has one or more genetic risk factors, or has been exposed to or is being exposed to one or more carcinogens (e.g., cigarette smoke, or chewing tobacco).

*Sequencing Data*

Aspects of the disclosure relate to methods for determining whether cells in a biological sample have HRD using sequencing data obtained from a biological sample from a subject.

In some embodiments, sequencing data may be generated using a nucleic acid from a sample from a subject. In some embodiments, the sequencing data may indicate a nucleotide sequence of DNA from a previously obtained biological sample of a subject having, suspected of having, or at risk of having a disease. In some embodiments, the nucleic acid is deoxyribonucleic acid (DNA). In some embodiments, the nucleic acid is prepared such that the whole genome is present in the nucleic acid. When nucleic acids are prepared such that the whole genome is sequenced, it is referred to as whole genome sequencing (WGS). In some embodiment, the nucleic acid is prepared such that fragmented DNA and/or RNA is present in the nucleic acid. In some embodiments, the nucleic acid is processed such that only the protein coding regions of the genome remain (e.g., exomes). When nucleic acids are prepared such that only the exomes are sequenced, it is referred to as whole exome sequencing (WES). A variety of methods are known in the art to isolate the exomes for sequencing, for example, solution-based isolation wherein tagged probes are used to hybridize the targeted regions (e.g., exomes) which can then be further separated from the other regions (e.g., unbound oligonucleotides). These tagged fragments can then be prepared and sequenced.

In some embodiments, the sequencing data may include DNA sequencing data, DNA exome sequencing data (e.g., from whole exome sequencing (WES)), DNA genome sequencing data (e.g., from whole genome sequencing (WGS), shallow whole genome sequencing (sWGS), etc.), gene sequencing data, bias-corrected gene sequencing data, or any other suitable type of sequencing data comprising data obtained from a sequencing platform and/or comprising data derived from data obtained from a sequencing platform.

DNA sequencing data, in some embodiments, includes a level of DNA (e.g., copy number of a chromosome, gene, or other genomic region) in a sample from a subject. The level of DNA in a sample from a subject having cancer may be elevated compared to the level of DNA in a sample from a subject not having cancer, e.g., a gene duplication in a cancer subject's sample. The level of DNA in a sample from a subject having cancer may be reduced compared to the level of DNA in a sample from a subject not having cancer, e.g., a gene deletion in a cancer subject's sample.

DNA sequencing data, in some embodiments, refers to DNA sequence reads and/or information derived from DNA sequence reads. A DNA sequence read refers to an inferred sequence of base pairs corresponding to all or part of a DNA fragment. In some embodiments, the sequence reads may be aligned to a reference sequence (e.g., a reference genome such as a human reference genome) to obtained mapped reads. The sequence reads may be aligned using any suitable sequence alignments techniques, as aspects of the technology are not limited in this respect. In some embodiments, information may be derived from one or more mapped sequence reads. For example, the mapped reads may be used to determine a copy number for each of one or more regions of the genome (e.g., a copy number profile). DNA sequencing data, in some embodiments, includes data obtained by processing a biological sample (e.g., DNA (e.g., coding or non-coding genomic DNA) present in a biological sample) using a sequencing apparatus. DNA that is present in a sample may or may not be transcribed, but it may be sequenced using DNA sequencing platforms. Such data may be useful, in some embodiments, to determine whether the subject has one or more mutations associated with a particular cancer.

Sequencing data may include data generated by the nucleic acid sequencing protocol (e.g., the series of nucleotides in a nucleic acid molecule identified by any suitable generation of sequencing (Sanger sequencing, Illumina®, next-generation sequencing (NGS) etc.), as well as information contained therein (e.g., information indicative of source, tissue type, etc.) which may also be considered information that can be inferred or determined from the sequencing data.

DNA sequencing data may be acquired using any method known in the art including any known method of DNA sequencing. For example, DNA sequencing may be used to identify one or more mutations in the DNA of a subject. Any technique used in the art to sequence DNA may be used with the methods and compositions described herein. As a set of non-limiting examples, the DNA may be sequenced through single-molecule real-time sequencing, ion torrent sequencing, pyrosequencing, sequencing by synthesis, sequencing by ligation (SOLiD sequencing), nanopore sequencing, or Sanger sequencing (chain termination sequencing).

In some embodiments, the sequencing data may be obtained using a sequencing platform such as a next generation sequencing platform (e.g., Illumina®, Roche®, Ion Torrent®, etc.), or any high-throughput or massively parallel sequencing platform. In some embodiments, these methods may be automated, in some embodiments, there may be manual intervention. In some embodiments, the sequencing data may be the result of non-next generation sequencing (e.g., Sanger sequencing).

In some embodiments, sequencing data comprises more than 5 kilobases (kb). In some embodiments, the size of the obtained sequencing data is at least 10 kb. In some embodiments, the size of the obtained sequencing data is at least 100 kb. In some embodiments, the size of the obtained sequencing data is at least 500 kb. In some embodiments, the size of the obtained sequencing data is at least 1 megabase (Mb). In some embodiments, the size of the obtained sequencing data is at least 10 Mb. In some embodiments, the size of the obtained sequencing data is at least 100 Mb. In some embodiments, the size of the obtained sequencing data is at least 500 Mb. In some embodiments, the size of the obtained sequencing data is at least 1 gigabase (Gb). In some embodiments, the size of the obtained sequencing data is at least 10 Gb. In some embodiments, the size of the obtained sequencing data is at least 100 Gb. In some embodiments, the size of the obtained sequencing data is at least 500 Gb.

*Therapies*

Aspects of the disclosure relate to methods of identifying or selecting a therapeutic agent for a subject based upon a determination of the subject's HRD status. The disclosure is based, in part, on the recognition that subjects having HRD have an increased likelihood of responding to certain therapies (e.g., platinum-based agents, poly ADP ribose polymerase (PARP) inhibitors, etc.) relative to subjects that do not have HRD.

In some embodiments, the therapeutic agents are platinum-based therapeutic agents. Examples of platinum-based therapeutic agents include but are not limited to cisplatin, carboplatin, and oxaliplatin.

In some embodiments, the therapeutic agents are PARP inhibitors. Examples of PARP inhibitors include but are not limited to veliparib, fluzoparib, talazoparib, Olaparib, rucaparib, and niraparib.

In some embodiments, methods described by the disclosure further comprise a step of administering one or more therapeutic agents to the subject based upon the determination of the subject's HRD status. In some embodiments, a subject is administered one or more (e.g., 1, 2, 3, 4, 5, or more) platinum-based agents. In some embodiments, a subject is administered one or more (e.g., 1, 2, 3, 4, 5 or more) PARP inhibitors.

Aspects of the disclosure relate to methods of treating a subject having (or suspected or at risk of having) cancer based upon a determination that the subject has HRD. In some embodiments, the methods comprise administering one or more (e.g., 1, 2, 3, 4, 5, or more) therapeutic agents to the subject.

The subject to be treated by the methods described herein may be a human subject having, suspected of having, or at risk for a cancer. Examples of a cancer include, but are not limited to, melanoma, lung cancer, brain cancer, breast cancer, colorectal cancer, pancreatic cancer, liver cancer, skin cancer, kidney cancer, bladder cancer, ovarian cancer, cervical cancer, or prostate cancer. At the time of diagnosis, the cancer may be cancer of unknown primary. The subject to be treated by the methods described herein may be a mammal (e.g., may be a human). Mammals include but are not limited to: a human, a mouse, a cat, a dog, a horse, a hamster, a cow, a pig, or other domesticated animal, a farm animal (e.g., livestock), a sport animal, a laboratory animal, a pet, and a primate.

A subject having a cancer may be identified by routine medical examination, e.g., laboratory tests, biopsy, PET scans, CT scans, or ultrasounds. A subject suspected of having a cancer might show one or more symptoms of the disorder, e.g., unexplained weight loss, fever, fatigue, cough, pain, skin changes, unusual bleeding or discharge, and/or thickening or lumps in parts of the body. A subject at risk for a cancer may be a subject having one or more of the risk factors for that disorder. For example, risk factors associated with cancer include, but are not limited to, (a) viral infection (e.g., herpes virus infection), (b) age, (c) family history, (d) heavy alcohol consumption, (e) obesity, and (f) tobacco use.

"An effective amount" as used herein refers to the amount of each active agent required to confer therapeutic effect on the subject, either alone or in combination with one or more other active agents. Effective amounts vary, as recognized by those skilled in the art, depending on the particular condition being treated, the severity of the condition, the individual subject parameters including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a subject may insist upon a lower dose or tolerable dose for medical reasons, psychological reasons, or for virtually any other reasons.

Empirical considerations, such as the half-life of a therapeutic compound, generally contribute to the determination of the dosage. For example, antibodies that are compatible with the human immune system, such as humanized antibodies or fully human antibodies, may be used to prolong half-life of the antibody and to prevent the antibody being attacked by the host's immune system. Frequency of administration may be determined and adjusted over the course of therapy and is generally (but not necessarily) based on treatment, and/or suppression, and/or amelioration, and/or delay of a cancer. Alternatively, sustained continuous release formulations of an anti-cancer therapeutic agent may be appropriate. Various formulations and devices for achieving sustained release are known in the art.

In some embodiments, dosages for an anti-cancer therapeutic agent as described herein may be determined empirically in individuals who have been administered one or more doses of the anti-cancer therapeutic agent. Individuals may be administered incremental dosages of the anti-cancer therapeutic agent. To assess efficacy of an administered anti-cancer therapeutic agent, one or more aspects of a cancer (e.g., tumor formation, tumor growth, molecular category identified for the cancer using the techniques described herein) may be analyzed.

Generally, for administration of any of the anti-cancer antibodies described herein, an initial candidate dosage may be about 2 mg/kg. For the purpose of the present disclosure, a typical daily dosage might range from about any of 0.1 μg/kg to 3 μg/kg to 30 μg/kg to 300 μg/kg to 3 mg/kg, to 30 mg/kg to 100 mg/kg or more, depending on the factors mentioned above. For repeated administrations over several days or longer, depending on the condition, the treatment is sustained until a desired suppression or amelioration of symptoms occurs or until sufficient therapeutic levels are achieved to alleviate a cancer, or one or more symptoms thereof. An exemplary dosing regimen comprises administering an initial dose of about 2 mg/kg, followed by a weekly maintenance dose of about 1 mg/kg of the antibody, or followed by a maintenance dose of about 1 mg/kg every other week. However, other dosage regimens may be useful, depending on the pattern of pharmacokinetic decay that the practitioner (e.g., a medical doctor) wishes to achieve. For example, dosing from one-four times a week is contemplated. In some embodiments, dosing ranging from about 3 μg/mg to about 2 mg/kg (such as about 3 μg/mg, about 10 μg/mg, about 30 μg/mg, about 100 μg/mg, about 300 μg/mg, about 1 mg/kg, and about 2 mg/kg) may be used. In some embodiments, dosing frequency is once every week, every 2 weeks, every 4 weeks, every 5 weeks, every 6 weeks, every 7 weeks, every 8 weeks, every 9 weeks, or every 10 weeks; or once every month, every 2 months, or every 3 months, or longer. The progress of this therapy may be monitored by conventional techniques and assays. The dosing regimen (including the therapeutic used) may vary over time.

When the anti-cancer therapeutic agent is not an antibody, it may be administered at the rate of about 0.1 to 300 mg/kg of the weight of the subject divided into one to three doses, or as disclosed herein. In some embodiments, for an adult subject of normal weight, doses ranging from about 0.3 to 5.00 mg/kg may be administered. The particular dosage regimen, e.g.., dose, timing, and/or repetition, will depend on the particular subject and that individual's medical history, as well as the properties of the individual agents (such as the half-life of the agent, and other considerations well known in the art).

For the purpose of the present disclosure, the appropriate dosage of an anti-cancer therapeutic agent will depend on the specific anti-cancer therapeutic agent(s) (or compositions thereof) employed, the type and severity of cancer, whether the anti-cancer therapeutic agent is administered for preventive or therapeutic purposes, previous therapy, the subject's clinical history and response to the anti-cancer therapeutic agent, and the discretion of the attending physician.

Typically, the clinician will administer an anti-cancer therapeutic agent, such as an antibody, until a dosage is reached that achieves the desired result.

Administration of an anti-cancer therapeutic agent can be continuous or intermittent, depending, for example, upon the recipient's physiological condition, whether the purpose of the administration is therapeutic or prophylactic, and other factors known to skilled practitioners. The administration of an anti-cancer therapeutic agent may be essentially continuous over a preselected period of time or may be in a series of spaced dose, e.g., either before, during, or after developing cancer.

As used herein, the term "treating" refers to the application or administration of a composition including one or more active agents to a subject, who has a cancer, a symptom of a cancer, or a predisposition toward a cancer, with the purpose to cure, heal, alleviate, relieve, alter, remedy, ameliorate, improve, or affect the cancer or one or more symptoms of the cancer, or the predisposition toward a cancer.

Alleviating a cancer includes delaying the development or progression of the disease or reducing disease severity. Alleviating the disease does not necessarily require curative results. As used therein, "delaying" the development of a disease (e.g., a cancer) means to defer, hinder, slow, retard, stabilize, and/or postpone progression of the disease. This delay can be of varying lengths of time, depending on the history of the disease and/or individuals being treated. A method that "delays" or alleviates the development of a disease, or delays the onset of the disease, is a method that reduces probability of developing one or more symptoms of the disease in a given period and/or reduces extent of the symptoms in a given time frame, when compared to not using the method. Such comparisons are typically based on clinical studies, using a number of subjects sufficient to give a statistically significant result.

"Development" or "progression" of a disease means initial manifestations and/or ensuing progression of the disease. Development of the disease can be detected and assessed using clinical techniques known in the art. Alternatively, or in addition to the clinical techniques known in the art, development of the disease may be detectable and assessed based on other criteria. However, development also refers to progression that may be undetectable. For purpose of this disclosure, development or progression refers to the biological course of the symptoms. "Development" includes occurrence, recurrence, and onset. As used herein "onset" or "occurrence" of a cancer includes initial onset and/or recurrence.

In some embodiments, the anti-cancer therapeutic agent described herein is administered to a subject in need of the treatment at an amount sufficient to reduce cancer (e.g., tumor) growth by at least 10% (e.g., 20%, 30%, 40%, 50%, 60%, 70%, 80%, 90% or greater). In some embodiments, the anti-cancer therapeutic agent described herein is administered to a subject in need of the treatment at an amount sufficient to reduce cancer cell number or tumor size by at least 10% (e.g., 20%, 30%, 40%, 50%, 60%, 70%, 80%, 90% or more). In other embodiments, the anti-cancer therapeutic agent is administered in an amount effective in altering cancer type. Alternatively, the anti-cancer therapeutic agent is administered in an amount effective in reducing tumor formation or metastasis.

Conventional methods, known to those of ordinary skill in the art of medicine, may be used to administer the anti-cancer therapeutic agent to the subject, depending upon the type of disease to be treated or the site of the disease. The anti-cancer therapeutic agent can also be administered via other conventional routes, e.g., administered orally, parenterally, by inhalation spray, topically, rectally, nasally, buccally, vaginally or via an implanted reservoir. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques. In addition, an anti-cancer therapeutic agent may be administered to the subject via injectable depot routes of administration such as using 1-, 3-, or 6-month depot injectable or biodegradable materials and methods.

Injectable compositions may contain various carriers such as vegetable oils, dimethylactamide, dimethyformamide, ethyl lactate, ethyl carbonate, isopropyl myristate, ethanol, and polyols (e.g., glycerol, propylene glycol, liquid polyethylene glycol, and the like). For intravenous injection, water soluble anti-cancer therapeutic agents can be administered by the drip method, whereby a pharmaceutical formulation containing the antibody and a physiologically acceptable excipients is infused. Physiologically acceptable excipients may include, for example, 5% dextrose, 0.9% saline, Ringer's solution, and/or other suitable excipients. Intramuscular preparations, e.g., a sterile formulation of a suitable soluble salt form of the anti-cancer therapeutic agent, can be dissolved and administered in a pharmaceutical excipient such as Water-for-Injection, 0.9% saline, and/or 5% glucose solution.

In one embodiment, an anti-cancer therapeutic agent is administered via site-specific or targeted local delivery techniques. Examples of site-specific or targeted local delivery techniques include various implantable depot sources of the agent or local delivery catheters, such as infusion catheters, an indwelling catheter, or a needle catheter, synthetic grafts, adventitial wraps, shunts and stents or other implantable devices, site specific carriers, direct injection, or direct application. See, e.g., PCT Publication No. WO 00/53211 and U.S. Pat. No. 5,981,568.

Targeted delivery of therapeutic compositions containing an antisense polynucleotide, expression vector, or subgenomic polynucleotides can also be used. Receptor-mediated DNA delivery techniques are described in, for example, Findeis et al., Trends Biotechnol. (1993) 11:202; Chiou et al., Gene Therapeutics: Methods and Applications of Direct Gene Transfer (J. A. Wolff, ed.) (1994); Wu et al., J. Biol. Chem. (1988) 263:621; Wu et al., J. Biol. Chem. (1994) 269:542; Zenke et al., Proc. Natl. Acad. Sci. USA (1990) 87:3655; Wu et al., J. Biol. Chem. (1991) 266:338.

Therapeutic compositions containing a polynucleotide may be administered in a range of about 100 ng to about 200 mg of DNA for local administration in a gene therapy protocol. In some embodiments, concentration ranges of about 500 ng to about 50 mg, about 1 $\mu$g to about 2 mg, about 5 $\mu$g to about 500 $\mu$g, and about 20 $\mu$g to about 100 $\mu$g of DNA or more can also be used during a gene therapy protocol.

Therapeutic polynucleotides and polypeptides can be delivered using gene delivery vehicles. The gene delivery vehicle can be of viral or non-viral origin (e.g., Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters and/or enhancers. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (see, e.g., PCT Publication Nos. WO 90/07936; WO 94/03622; WO 93/25698; WO 93/25234; WO 93/11230; WO 93/10218; WO 91/02805; U.S. Pat. Nos. 5,219,740 and 4,777,127; GB Patent No. 2,200,651; and EP Patent No. 0 345 242), alphavirus-based vectors (e.g., Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532)), and adeno-associated virus (AAV) vectors (see, e.g., PCT Publication Nos. WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed.

Non-viral delivery vehicles and methods can also be employed, including, but not limited to, polycationic condensed DNA linked or unlinked to killed adenovirus alone (see, e.g., Curiel, Hum. Gene Ther. (1992) 3:147); ligand-linked DNA (see, e.g., Wu, J. Biol. Chem. (1989) 264:16985); eukaryotic cell delivery vehicles cells (see, e.g., U.S. Pat. No. 5,814,482; PCT Publication Nos. WO 95/07994; WO 96/17072; WO 95/30763; and WO 97/42338) and nucleic charge neutralization or fusion with cell membranes. Naked DNA can also be employed. Exemplary naked DNA introduction methods are described in PCT Publication No. WO 90/11092 and U.S. Pat. No. 5,580,859. Liposomes that can act as gene delivery vehicles are described in U.S. Pat. No. 5,422,120; PCT Publication Nos. WO 95/13796; WO 94/23697; WO 91/14445; and EP Patent No. 0524968. Additional approaches are described in Philip, Mol. Cell. Biol. (1994) 14:2411, and in Woffendin, Proc. Natl. Acad. Sci. (1994) 91:1581.

It is also apparent that an expression vector can be used to direct expression of any of the protein-based anti-cancer therapeutic agents (e.g., anti-cancer antibody). For example, peptide inhibitors that are capable of blocking (from partial to complete blocking) a cancer-causing biological activity are known in the art.

In some embodiments, more than one anti-cancer therapeutic agent, such as an antibody and a small molecule inhibitory compound, may be administered to a subject in need of the treatment. The agents may be of the same type or different types from each other. At least one, at least two, at least three, at least four, or at least five different agents may be co-administered. Generally anti-cancer agents for administration have complementary activities that do not adversely affect each other. Anti-cancer therapeutic agents may also be used in conjunction with other agents that serve to enhance and/or complement the effectiveness of the agents.

Treatment efficacy can be assessed by methods well-known in the art, *e.g.,* monitoring tumor growth or formation in a subject subjected to the treatment. Alternatively, or in addition to, treatment efficacy can be assessed by monitoring tumor type over the course of treatment (*e.g.*, before, during, and after treatment).

A subject having cancer may be treated using any combination of anti-cancer therapeutic agents or one or more anti-cancer therapeutic agents and one or more additional therapies (*e.g.*, surgery and/or radiotherapy). The term combination therapy, as used herein, embraces administration of more than one treatment (*e.g.*, an antibody and a small molecule or an antibody and radiotherapy) in a sequential manner, that is, wherein each therapeutic agent is administered at a different time, as well as administration of these therapeutic agents, or at least two of the agents or therapies, in a substantially simultaneous manner.

Sequential or substantially simultaneous administration of each agent or therapy can be affected by any appropriate route including, but not limited to, oral routes, intravenous routes, intramuscular, subcutaneous routes, and direct absorption through mucous membrane tissues. The agents or therapies can be administered by the same route or by different routes. For example, a first agent (*e.g.,* a small molecule) can be administered orally, and a second agent (*e.g.*, an antibody) can be administered intravenously.

As used herein, the term "sequential" means, unless otherwise specified, characterized by a regular sequence or order, *e.g.,* if a dosage regimen includes the administration of an antibody and a small molecule, a sequential dosage regimen could include administration of the antibody before, simultaneously, substantially simultaneously, or after administration of the small molecule, but both agents will be administered in a regular sequence or order. The term "separate" means, unless otherwise specified, to keep apart one from the other. The term "simultaneously" means, unless otherwise specified, happening or done at the same time, *i.e.,* the agents are administered at the same time. The term "substantially simultaneously" means that the agents are administered within minutes of each other (*e.g.,* within 10 minutes of each other) and intends to embrace joint administration as well as consecutive administration, but if the administration is consecutive it is separated in time for only a short period (*e.g.,* the time it would take a medical practitioner to administer two agents separately). As used herein, concurrent administration and substantially simultaneous administration are used interchangeably. Sequential administration refers to temporally separated administration of the agents or therapies described herein.

Combination therapy can also embrace the administration of the anti-cancer therapeutic agent (*e.g.*, an antibody) in further combination with other biologically active ingredients (*e.g.,* a vitamin) and non-drug therapies (*e.g.,* surgery or radiotherapy).

It should be appreciated that any combination of anti-cancer therapeutic agents may be used in any sequence for treating a cancer. The combinations described herein may be selected on the basis of a number of factors, which include but are not limited to reducing tumor formation or tumor growth, and/or alleviating at least one symptom associated with the cancer, or the effectiveness for mitigating the side effects of another agent of the combination. For example, a combined therapy as provided herein may reduce any of the side effects associated with each individual members of the combination, for example, a side effect associated with an administered anti-cancer agent.

In some embodiments, an anti-cancer therapeutic agent is an antibody, an immunotherapy, a radiation therapy, a surgical therapy, and/or a chemotherapy.

Examples of the antibody anti-cancer agents include, but are not limited to, alemtuzumab (Campath), trastuzumab (Herceptin), Ibritumomab tiuxetan (Zevalin), Brentuximab vedotin (Adcetris), Ado-trastuzumab emtansine (Kadcyla), blinatumomab (Blincyto), Bevacizumab (Avastin), Cetuximab (Erbitux), ipilimumab (Yervoy), nivolumab (Opdivo), pembrolizumab (Keytruda), atezolizumab (Tecentriq), avelumab (Bavencio), durvalumab (Imfinzi), and panitumumab (Vectibix).

Examples of an immunotherapy include, but are not limited to, a PD-1 inhibitor or a PD-L1 inhibitor, a CTLA-4 inhibitor, adoptive cell transfer, therapeutic cancer vaccines, oncolytic virus therapy, T-cell therapy, and immune checkpoint inhibitors.

Examples of radiation therapy include, but are not limited to, ionizing radiation, gamma-radiation, neutron beam radiotherapy, electron beam radiotherapy, proton therapy, brachytherapy, systemic radioactive isotopes, and radiosensitizers.

Examples of a surgical therapy include, but are not limited to, a curative surgery (*e.g.*, tumor removal surgery), a preventive surgery, a laparoscopic surgery, and a laser surgery.

Examples of the chemotherapeutic agents include, but are not limited to, Carboplatin or Cisplatin, Docetaxel, Gemcitabine, Nab-Paclitaxel, Paclitaxel, Pemetrexed, and Vinorelbine.

Additional examples of chemotherapy include, but are not limited to, Platinating agents, such as Carboplatin, Oxaliplatin, Cisplatin, Nedaplatin, Satraplatin, Lobaplatin, Triplatin, Tetranitrate, Picoplatin, Prolindac, Aroplatin and other derivatives; Topoisomerase I inhibitors, such as Camptothecin, Topotecan, irinotecan/SN38, rubitecan, Belotecan, and other derivatives; Topoisomerase II inhibitors, such as Etoposide (VP-16), Daunorubicin, a doxorubicin agent (*e.g.*, doxorubicin, doxorubicin hydrochloride, doxorubicin analogs, or doxorubicin and salts or analogs thereof in liposomes), Mitoxantrone, Aclarubicin, Epirubicin, Idarubicin, Amrubicin, Amsacrine, Pirarubicin, Valrubicin, Zorubicin, Teniposide and other derivatives; Antimetabolites, such as Folic family (Methotrexate, Pemetrexed, Raltitrexed, Aminopterin, and relatives or derivatives thereof); Purine antagonists (Thioguanine, Fludarabine, Cladribine, 6-Mercaptopurine, Pentostatin, clofarabine, and relatives or derivatives thereof) and Pyrimidine antagonists (Cytarabine, Floxuridine, Azacitidine, Tegafur, Carmofur, Capacitabine, Gemcitabine, hydroxyurea, 5-Fluorouracil (5FU), and relatives or derivatives thereof); Alkylating agents, such as Nitrogen mustards (*e.g.*, Cyclophosphamide, Melphalan, Chlorambucil, mechlorethamine, Ifosfamide, mechlorethamine, Trofosfamide, Prednimustine, Bendamustine, Uramustine, Estramustine, and relatives or derivatives thereof); nitrosoureas (*e.g.*, Carmustine, Lomustine, Semustine, Fotemustine, Nimustine, Ranimustine, Streptozocin, and relatives or derivatives thereof); Triazenes (*e.g.*, Dacarbazine, Altretamine, Temozolomide, and relatives or derivatives thereof); Alkyl sulphonates (*e.g.*, Busulfan, Mannosulfan, Treosulfan, and relatives or derivatives thereof); Procarbazine; Mitobronitol, and Aziridines (*e.g.*, Carboquone, Triaziquone, ThioTEPA, triethylenemalamine, and relatives or derivatives thereof) ; Antibiotics, such as Hydroxyurea, Anthracyclines (*e.g.*, doxorubicin agent, daunorubicin, epirubicin and relatives or derivatives thereof); Anthracenediones (*e.g.,* Mitoxantrone and relatives or derivatives thereof); Streptomyces family antibiotics (*e.g.,* Bleomycin, Mitomycin C, Actinomycin, and Plicamycin); and ultraviolet light.

Having thus described several aspects and embodiments of the technology set forth in the disclosure, it is to be appreciated that various alterations, modifications, and improvements will readily occur to those skilled in the art. For example, those of ordinary skill in the art will readily envision a variety of other means and/or structures for performing the function and/or obtaining the results and/or one or more of the advantages described herein, and each of such variations and/or modifications is deemed to be within the scope of the embodiments described herein. Those skilled in the art will recognize or be able to ascertain using no more than routine experimentation many equivalents to the specific embodiments described herein. It is, therefore, to be understood that the foregoing embodiments are presented by way of example only. In addition, any combination of two or more features, systems, articles, materials, kits, and/or methods described herein, if such features, systems, articles, materials, kits, and/or methods are not mutually inconsistent, is included within the scope of the present disclosure.

Also, as described, some aspects may be embodied as one or more methods. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

The terms "approximately," "substantially," and "about" may be used to mean within $\pm 20\%$ of a target value in some embodiments, within $\pm 10\%$ of a target value in some embodiments, within $\pm 5\%$ of a target value in some embodiments, within $\pm 2\%$ of a target value in some embodiments. The terms "approximately," "substantially," and "about" may include the target value.

## Claims

1. A method for determining whether a biological sample obtained from a subject includes cells having homologous recombination deficiency (HRD), the method comprising:
   using at least one computer hardware processor to perform:

   obtaining data about segments of the subject's genome, the data including, for each of at least some of the segments, a respective copy number and a respective length;
   identifying a first subset of the segments, the first subset including segments associated with at least one chromosome arm of the genome, wherein each of the segments of the first subset has a common copy number, wherein the segments include a first segment, and wherein identifying the first subset of the segments comprises, for the first segment:

   determining whether a length of the first segment is greater than or equal to a first threshold length, wherein the first threshold length is between 30% and 70% of a length of the chromosome arm associated with the first segment;
   determining whether a copy number of the first segment equals ploidy of tumor cells in the biological sample; and
   including the first segment in the first subset when it is determined that (i) the length of the first segment is greater than or equal to the first threshold length, and that (ii) the copy number of the first segment equals the ploidy of tumor cells in the biological sample;

identifying a second subset of the segments, wherein each of the segments of the second subset has (i) a respective copy number different from the common copy number and (ii) a respective length that is between 1 megabase and 30% of a length of the chromosome arm associated with the segment;

determining a proportion of a number of segments in the second subset to a number of chromosome arms of the at least one chromosome arm associated with the segments included in the first subset;

determining, based on the determined proportion, whether the biological sample includes cells having HRD, wherein determining, based on the determined proportion, whether the biological sample includes cells having HRD comprises processing the determined proportion with a statistical model trained to predict an output indicating whether the biological sample includes cells having HRD.

2. The method of claim 1, further comprising: identifying a therapy for the subject based on a result of determining whether the biological sample includes cells having HRD.

3. The method of claim 2, wherein identifying the therapy for the subject comprises:
when the output indicates that the biological sample includes cells having HRD, identifying a polyADPribose polymerase inhibitor, PARPi, therapy or a platinum-based chemotherapy for the subject.

4. The method of any preceding claim, wherein the first threshold length is between 35% and 65%, or at least 40% of a length of the chromosome arm associated with the first segment; and/or wherein each of the segments of the second subset has a respective length that is between 3 megabases and 10% of a length of the chromosome arm associated with the segment.

5. The method of any preceding claim, wherein the segments include one or more other segments, and wherein identifying the first subset of the segments further comprises:

determining whether a sum of the length of the first segment and lengths of the one or more other segments is greater than or equal to a second threshold length; and

including the first segment and the one or more other segments in the first subset when it is determined that the sum is greater than or equal to the second threshold length,

optionally wherein the second threshold length is at least 50% of a length of the chromosome arm associated with the first segment and the one or more segments; and/or wherein the one or more other segments consist of three or fewer segments.

6. The method of any preceding claim, wherein identifying the second subset of the segments comprises, for a particular segment:

determining whether a copy number of the particular segment differs from the common copy number;

determining whether a length of the particular segment is between 1 megabase and 30% of a length of the chromosome arm associated with the particular segment; and

including the particular segment in the second subset of segments when it is determined that (i) the copy number of the particular segment differs from the common copy number and (ii) that the length of the particular segment is between 1 megabase and 30% of the length of the chromosome arm associated with the particular segment.

7. The method of any preceding claim, wherein:

(i) the output indicating whether the biological sample includes cells having HRD is a probability that the biological sample includes cells having HRD; and/or

(ii) the output indicating whether the biological sample includes cells having HRD is a score indicative of whether the biological sample includes cells having HRD, and a score greater than or equal to threshold value indicates that the biological sample includes cells having HRD; and/or

(iii) the statistical model is a generalized linear model; and/or the statistical model is a logistic regression model; and/or

(iv) the statistical model is trained to predict the output using training data comprising, for each of a plurality of subjects with a known HRD status, data about segments of the subject's genome.

8. The method of any preceding claim,

   wherein the subject has, is suspected of having, or is at risk of having cancer of a first cancer type,
   wherein processing the determined proportion with the statistical model comprises processing the determined proportion with a first statistical model trained to predict whether cells of the first cancer type have HRD, and wherein the method further comprises:

   using a second statistical model different from the first statistical model to determine whether a second biological sample obtained from a second subject includes cells having HRD,
   wherein the second subject has, is suspected of having, or is at risk of having cancer of a second cancer type different from the first cancer type, and
   wherein the second statistical model is trained to predict whether cells of the second cancer type have HRD;
   optionally wherein the first cancer type is breast cancer and wherein the second cancer type is ovarian cancer.

9. The method of any preceding claim, wherein processing the determined proportion with the statistical model comprises processing the determined proportion with a statistical model trained to predict whether breast cancer cells have HRD, or trained to predict whether ovarian cancer cells have HRD.

10. The method of any preceding claim, further comprising:

    (i) determining ploidy of tumor cells in the biological sample,
    wherein determining whether the biological sample includes cells having HRD is based on the determined proportion and the determined ploidy; and/or
    (ii) obtaining sequencing data, the sequencing data having been previously obtained by sequencing the biological sample from the subject; and

    processing the sequencing data to obtain the data about the segments of the subject's genome.

11. The method of claim 10, wherein obtaining the sequencing data comprises obtaining sequencing data for at least a chromosome of the subject's genome, or for the subject's whole genome.

12. The method of any preceding claim, wherein the subject:

    (i) has, is suspected of having or is at risk of having breast cancer, ovarian cancer, pancreatic cancer, prostate cancer, bladder cancer, colorectal cancer, or lymphoma; or
    (ii) has, is suspected of having, or is at risk of having breast cancer.

13. A method for identifying whether a subject is likely to be sensitive to treatment with PolyADPribose polymerase inhibitor (PARPi) therapy or a platinum-based chemotherapy, the method comprising:

    determining whether a biological sample obtained from the subject includes cells having HRD using the method of any preceding claim; and
    when it is determined that the biological sample includes cells having HRD,
    determining that the subject is likely to be sensitive to treatment with the PARPi therapy or the platinum-based chemotherapy.

14. A system, comprising:

    at least one computer hardware processor; and
    at least one non-transitory computer-readable storage medium storing processor-executable instructions that, when executed by the at least one computer hardware processor, cause the at least one computer hardware processor to perform a method according to any preceding claim.

15. At least one non-transitory computer-readable storage medium storing processor-executable instructions that, when executed by at least one computer hardware processor, cause the at least one computer hardware processor to perform a method according to any of claims 1-13.

**Patentansprüche**

1. Verfahren zum Bestimmen, ob eine von einem Individuum erhaltene biologische Probe Zellen mit homologer Rekombinationsdefizienz (HRD) umfasst, wobei das Verfahren Folgendes umfasst:
Verwenden zumindest eines Computerhardwareprozessors, um Folgendes auszuführen:

Ermitteln von Daten über Segmente des Genoms des Individuums, wobei die Daten für jedes zumindest einiger der Segmente eine entsprechende Kopienzahl und eine entsprechende Länge umfassen;
Identifizieren eines ersten Teilsatzes der Segmente, wobei der erste Teilsatz Segmente umfasst, die zumindest einem Chromosomenarm des Genoms zugeordnet sind, wobei jedes der Segmente des ersten Teilsatzes eine gemeinsame Kopienzahl hat, wobei die Segmente ein erstes Segment umfassen, und wobei das Identifizieren des ersten Teilsatzes der Segmente für das erste Segment Folgendes umfasst:

Bestimmen, ob die Länge des ersten Segments größer als oder gleich wie eine erste Schwellenlänge ist, wobei die erste Schwellenlänge zwischen 30 % und 70 % der Länge des Chromosomenarms beträgt, der dem ersten Segment zugeordnet ist;
Bestimmen, ob die Kopienzahl des ersten Segments gleich der Ploidie von Tumorzellen in der biologischen Probe ist; und
Aufnehmen des ersten Segments in den ersten Teilsatz, wenn bestimmt wird, dass (i) die Länge des ersten Segments größer als oder gleich wie die erste Schwellenlänge ist und (ii) die Kopienzahl des ersten Segments gleich der Ploidie von Tumorzellen in der biologischen Probe ist;

Identifizieren eines zweiten Teilsatzes der Segmente, wobei jedes der Segmente des zweiten Teilsatzes (i) eine entsprechende Kopienzahl aufweist, die sich von der gemeinsamen Kopienzahl unterscheidet, und (ii) eine entsprechende Länge aufweist, die zwischen 1 Megabase und 30 % der Länge des Chromosomenarms beträgt, der dem Segment zugeordnet ist;
Bestimmen des Verhältnisses zwischen der Anzahl von Segmenten im zweiten Teilsatz und der Anzahl von Chromosomenarmen des zumindest einen Chromosomenarms, der den Segmenten zugeordnet ist, die im ersten Teilsatz enthalten sind;
Bestimmen, basierend auf dem bestimmten Verhältnis, ob die biologische Probe Zellen mit HRD umfasst, wobei das Bestimmen basierend auf dem bestimmten Verhältnis, ob die biologische Probe Zellen mit HRD umfasst, das Verarbeiten des bestimmten Verhältnisses mit einem statistischen Modell umfasst, das trainiert wurde, um ein Ergebnis vorherzusagen, das angibt, ob die biologische Probe Zellen mit HRD umfasst.

2. Verfahren nach Anspruch 1, das weiters Folgendes umfasst: Identifizieren einer Therapie für das Individuum basierend auf dem Ergebnis einer Bestimmung, ob die biologische Probe Zellen mit HRD umfasst.

3. Verfahren nach Anspruch 2, wobei das Identifizieren der Therapie für das Individuum Folgendes umfasst:
wenn das Ergebnis angibt, dass die biologische Probe Zellen mit HRD umfasst, Identifizieren einer Poly-ADP-Ribose-Polymerase-Inhibitor-, PARPi-, Therapie oder einer Chemotherapie auf Platinbasis für das Individuum.

4. Verfahren nach einem der vorangegangenen Ansprüche, wobei die erste Schwellenlänge zwischen 35 % und 65 % liegt oder zumindest 40 % der Länge des Chromosomenarms beträgt, der dem ersten Segment zugeordnet ist; und/oder wobei jedes der Segmente des zweiten Teilsatzes eine entsprechende Länge aufweist, die zwischen 3 Megabasen und 10 % der Länge des Chromosomenarms beträgt, der dem Segment zugeordnet ist.

5. Verfahren nach einem der vorangegangenen Ansprüche, wobei die Segmente ein oder mehrere weitere Segmente umfassen und wobei das Identifizieren des ersten Teilsatzes der Segmente weiters Folgendes umfasst:

Bestimmen, ob die Summe aus der Länge des ersten Segments und der Länge des einen oder der mehreren weiteren Segmente größer als oder gleich wie eine zweite Schwellenlänge ist; und
Aufnehmen des ersten Segments und des einen oder der mehreren weiteren Segmente in den ersten Teilsatz, wenn bestimmt wird, dass die Summe größer als oder gleich wie die zweite Schwellenlänge ist, wobei gegebenenfalls die zweite Schwellenlänge zumindest 50 % der Länge des Chromosomenarms beträgt, der dem ersten Segment und dem einen oder den mehreren Segmenten zugeordnet ist; und/oder wobei das eine oder die mehreren weiteren Segmente aus drei oder weniger Segmenten bestehen.

6. Verfahren nach einem der vorangegangenen Ansprüche, wobei das Identifizieren des zweiten Teilsatzes der Segmente für ein jeweiliges Segment Folgendes umfasst:

Bestimmen, ob sich die Kopienzahl des jeweiligen Segments von der gemeinsamen Kopienzahl unterscheidet;
Bestimmen, ob die Länge des jeweiligen Segments zwischen 1 Megabase und 30 % der Länge des Chromosomenarms liegt, der dem jeweiligen Segment zugeordnet ist; und
Aufnehmen des jeweiligen Segments in den zweiten Teilsatz von Segmenten, wenn bestimmt wird, dass (i) sich die Kopienzahl des jeweiligen Segments von der gemeinsamen Kopienzahl unterschiedet und (ii) die Länge des jeweiligen Segments zwischen 1 Megabase und 30 % der Länge des Chromosomenarms liegt, der dem jeweiligen Segment zugeordnet ist.

7. Verfahren nach einem der vorangegangenen Ansprüche, wobei:

(i) das Ergebnis, das angibt, ob die biologische Probe Zellen mit HRD umfasst, eine Wahrscheinlichkeit ist, dass die biologische Probe Zellen mit HRD umfasst; und/oder
(ii) das Ergebnis, das angibt, ob die biologische Probe Zellen mit HRD umfasst, ein Wert ist, der angibt, ob die biologische Probe Zellen mit HRD umfasst, wobei ein Wert größer als oder gleich wie ein Schwellenwert angibt, dass die biologische Probe Zellen mit HRD umfasst; und/oder
(iii) das statistische Modell ein generalisiertes lineares Modell ist; und/oder das statistische Modell ein logistisches Regressionsmodell ist; und/oder
(iv) das statistische Modell trainiert wurde, um das Ergebnis unter Verwendung von Trainingsdaten vorherzusagen, die für jedes aus einer Vielzahl von Individuen mit einem bekannten HRD-Status Daten über Segmente des Genoms des Individuums umfassen.

8. Verfahren nach einem der vorangegangenen Ansprüche,

wobei das Individuum an Krebs eines ersten Krebstyps leidet, ein Verdacht darauf besteht oder ein Risiko dafür vorliegt,
wobei das Verarbeiten des bestimmten Verhältnisses mit dem statistischen Modell das Verarbeiten des bestimmten Verhältnisses mit einem ersten statistischen Modell umfasst, das trainiert wurde, um vorherzusagen, ob Zellen des ersten Krebstyps HRD aufweisen, und
wobei das Verfahren weiters Folgendes umfasst:

Verwenden eines zweiten statistischen Modells, das sich vom ersten statistischen Modell unterscheidet, um zu bestimmen, ob eine zweite biologische Probe von einem zweiten Individuum Zellen mit HRD umfasst, wobei das zweite Individuum an Krebs eines zweiten Krebstyps, der sich vom ersten Krebstyp unterscheidet, leidet, ein Verdacht darauf besteht oder ein Risiko dafür vorliegt und
wobei das zweite statistische Modell trainiert wurde, um vorherzusagen, ob Zellen des zweiten Krebstyps HRD aufweisen; wobei gegebenenfalls der erste Krebstyp Brustkrebs ist und der zweite Krebstyp Eierstockkrebs ist.

9. Verfahren nach einem der vorangegangenen Ansprüche, wobei das Verarbeiten des bestimmten Verhältnisses mit dem statistischen Modell das Verarbeiten des bestimmten Verhältnisses mit einem statistischen Modell umfasst, das trainiert wurde, um vorherzusagen, ob Brustkrebszellen HRD aufweisen, oder trainiert wurde, um vorherzusagen, ob Eierstockkrebszellen HRD aufweisen.

10. Verfahren nach einem der vorangegangenen Ansprüche, das weiters Folgendes umfasst:

(i) Bestimmen der Ploidie von Tumorzellen in der biologischen Probe, wobei das Bestimmen, ob die biologische Probe Zellen mit HRD umfasst, auf dem bestimmten Verhältnis und der bestimmten Ploidie basiert; und/oder
(ii) Ermitteln von Sequenzierungsdaten, wobei die Sequenzierungsdaten zuvor durch Sequenzieren der biologischen Probe vom Individuum erhalten wurden; und

Verarbeiten der Sequenzierungsdaten, um Daten über die Segmente des Genoms des Individuums zu erhalten.

11. Verfahren nach Anspruch 10, wobei das Ermitteln von Sequenzierungsdaten das Ermitteln von Sequenzierungsdaten für zumindest ein Chromosom des Genoms des Individuums oder für das gesamte Genom des Individuums umfasst.

12. Verfahren nach einem der vorangegangenen Ansprüche, wobei das Individuum:

(i) an Brustkrebs, Eierstockkrebs, Pankreaskrebs, Prostatakrebs, Blasenkrebs, Kolorektalkrebs oder einem Lymphom leidet, ein Verdacht darauf besteht oder ein Risiko dafür vorliegt; oder

(ii) an Brustkrebs leidet, ein Verdacht darauf besteht oder ein Risiko dafür vorliegt.

13. Verfahren zum Identifizieren, ob ein Individuum voraussichtlich auf eine Behandlung mit einer Poly-ADP-Ribose-Polymerase-Inhibitor- (PARPi-) Therapie oder eine Chemotherapie auf Platinbasis anspricht, wobei das Verfahren Folgendes umfasst:

Bestimmen, ob eine biologische Probe, die von dem Individuum erhalten wurde, Zellen mit HRD umfasst, unter Verwendung eines Verfahrens nach einem der vorangegangenen Ansprüche; und

wenn bestimmt wird, dass die biologische Probe Zellen mit HRD umfasst, Bestimmen, dass das Individuum voraussichtlich auf eine Behandlung mit der PARPi-Therapie oder der Chemotherapie auf Platinbasis anspricht.

14. System, das Folgendes umfasst:

zumindest einen Computerhardwareprozessor; und

zumindest ein nicht-transitorisches, computerlesbares Speichermedium, auf dem durch einen Prozessor ausführbare Anweisungen gespeichert sind, die bei Ausführung durch den zumindest einen Computerhardwareprozessor bewirken, dass der zumindest eine Computerhardwareprozessor ein Verfahren nach einem der vorangegangenen Ansprüche ausführt.

15. Zumindest ein nicht-transitorisches, computerlesbares Speichermedium, auf dem durch einen Prozessor ausführbare Anweisungen gespeichert sind, die bei Ausführung durch zumindest einen Computerhardwareprozessor bewirken, dass der zumindest eine Computerhardwareprozessor ein Verfahren nach einem der Ansprüche 1 bis 13 ausführt.

## Revendications

1. Procédé pour déterminer si un échantillon biologique obtenu à partir d'un sujet inclut des cellules présentant une déficience de recombinaison homologue (HRD), le procédé comprenant :
l'utilisation d'au moins un processeur matériel informatique pour effectuer les étapes consistant à :

obtenir des données sur des segments du génome du sujet, les données comprenant, pour chacun d'au moins certains des segments, un nombre de copie respectifs et une longueur respective ;
identifier un premier sous-ensemble des segments, le premier sous-ensemble comprenant des segments associés à au moins un bras chromosomique du génome, dans lequel chacun des segments du premier sous-ensemble présente un nombre de copies commun, dans lequel les segments comprennent un premier segment, et dans lequel l'identification du premier sous-ensemble des segments comprend, pour le premier segment, les étapes consistant à :

déterminer si une longueur du premier segment est supérieure ou égale à une première longueur de seuil, dans lequel la première longueur de seuil est comprise entre 30 % et 70 % d'une longueur du bras chromosomique associé au premier segment ;
déterminer si un nombre de copies du premier segment est égal à la ploïdie des cellules tumorales dans l'échantillon biologique ; et
inclure le premier segment dans le premier sous-ensemble lorsqu'il est déterminé que (i) la longueur du premier segment est supérieure ou égale à la première longueur de seuil, et que (ii) le nombre de copies du premier segment est égal à la ploïdie des cellules tumorales dans l'échantillon biologique ;

identifier un second sous-ensemble des segments, dans lequel chacun des segments du second sous-ensemble présente (i) un nombre de copies respectif différent du nombre de copies commun et (ii) une longueur respective qui est comprise entre 1 mégabase et 30 % d'une longueur du bras chromosomique associé au segment ;
déterminer une proportion d'un certain nombre de segments dans le second sous-ensemble par rapport à un certain nombre de bras chromosomiques du au moins un bras chromosomique associé aux segments inclus dans le premier sous-ensemble ;

déterminer, sur la base de la proportion déterminée, si l'échantillon biologique inclut des cellules présentant une HRD, dans lequel la détermination, sur la base de la proportion déterminée, que l'échantillon biologique inclut ou non des cellules présentant une HRD comprend un traitement de la proportion déterminée avec un modèle statistique entraîné pour prédire une sortie indiquant si l'échantillon biologique inclut des cellules présentant une HRD.

2. Procédé selon la revendication 1, comprenant en outre l'étape consistant à :
identifier une thérapie pour le sujet sur la base d'un résultat de détermination que l'échantillon biologique comprend ou non des cellules présentant une HRD.

3. Procédé selon la revendication 2, dans lequel l'identification de la thérapie pour le sujet comprend l'étape consistant à :
lorsque la sortie indique que l'échantillon biologique inclut des cellules présentant une HRD, identifier un inhibiteur de polyADPribose polymérase, PARPi, une thérapie ou une chimiothérapie à base de platine pour le sujet.

4. Procédé selon l'une quelconque des revendications précédentes, dans lequel la première longueur de seuil est comprise entre 35 % et 65 %, ou au moins 40 % d'une longueur du bras chromosomique associé au premier segment ; et/ou
dans lequel chacun des segments du second sous-ensemble présente une longueur respective qui est comprise entre 3 mégabases et 10 % d'une longueur du bras chromosomique associé au segment.

5. Procédé selon l'une quelconque des revendications précédentes, dans lequel les segments incluent un ou plusieurs autres segments, et dans lequel l'identification du premier sous-ensemble des segments comprend en outre les étapes consistant à :

déterminer si une somme de la longueur du premier segment et des longueurs des un ou plusieurs autres segments est supérieure ou égale à une seconde longueur de seuil ; et
inclure le premier segment et les un ou plusieurs autres segments dans le premier sous-ensemble lorsqu'il est déterminé que la somme est supérieure ou égale à la seconde longueur de seuil,
facultativement dans lequel la seconde longueur de seuil est d'au moins 50 % d'une longueur du bras chromosomique associé au premier segment et aux un ou plusieurs segments ; et/ou
dans lequel les un ou plusieurs autres segments consistent en trois segments ou moins.

6. Procédé selon l'une quelconque des revendications précédentes, dans lequel l'identification du second sous-ensemble des segments comprend, pour un segment particulier, les étapes consistant à :

déterminer si un nombre de copies du segment particulier diffère du nombre de copies commun ;
déterminer si une longueur du segment particulier est comprise entre 1 mégabase et 30 % d'une longueur du bras chromosomique associé au segment particulier ; et
inclure le segment particulier dans le second sous-ensemble de segments lorsqu'il est déterminé que (i) le nombre de copies du segment particulier diffère du nombre de copies commun et (ii) que la longueur du segment particulier est comprise entre 1 mégabase et 30 % de la longueur du bras chromosomique associé au segment particulier.

7. Procédé selon l'une quelconque des revendications précédentes, dans lequel :

(i) la sortie indiquant si l'échantillon biologique inclut des cellules présentant une HRD est une probabilité que l'échantillon biologique inclut des cellules présentant une HRD ; et/ou
(ii) la sortie indiquant si l'échantillon biologique inclut des cellules présentant une HRD est un score indiquant si l'échantillon biologique comprend des cellules présentant une HRD, et un score supérieur ou égal à la valeur de seuil indique que l'échantillon biologique inclut des cellules présentant une HRD ; et/ou
(iii) le modèle statistique est un modèle linéaire généralisé ; et/ou le modèle statistique est un modèle de régression logistique ; et/ou
(iv) le modèle statistique est entraîné pour prédire la sortie à l'aide de données d'entraînement comprenant, pour chacun d'une pluralité de sujets présentant un statut de HRD connu, des données sur des segments du génome du sujet.

8. Procédé selon l'une quelconque des revendications précédentes, dans lequel le sujet présente, est suspecté de présenter ou risque de présenter un cancer d'un premier type de cancer,

dans lequel le traitement de la proportion déterminée avec le modèle statistique comprend le traitement de la proportion déterminée avec un premier modèle statistique entraîné pour prédire si des cellules du premier type de cancer présentent une DRH, et
dans lequel le procédé comprend en outre l'étape consistant à :

utiliser un second modèle statistique différent du premier modèle statistique pour déterminer si un second échantillon biologique obtenu auprès d'un second sujet inclut des cellules présentant une HRD,
dans lequel le second sujet présente, est suspecté de présenter ou risque de présenter un cancer d'un second type de cancer différent du premier type de cancer, et
dans lequel le second modèle statistique est formé pour prédire si des cellules du second type de cancer présentent une HRD ;
facultativement dans lequel le premier type de cancer est le cancer du sein et dans lequel le second type de cancer est le cancer de l'ovaire.

9. Procédé selon l'une quelconque des revendications précédentes, dans lequel le traitement de la proportion déterminée avec le modèle statistique comprend un traitement de la proportion déterminée avec un modèle statistique entraîné pour prédire si des cellules de cancer du sein présentent une HRD, ou entraîné pour prédire si des cellules de cancer de l'ovaire présentent une HRD.

10. Procédé selon l'une quelconque des revendications précédentes, comprenant en outre les étapes consistant à :

(i) déterminer la ploïdie des cellules tumorales dans l'échantillon biologique, dans lequel la détermination que l'échantillon biologique comprend ou non des cellules présentant une HRD est basée sur la proportion déterminée et la ploïdie déterminée ; et/ou
(ii) obtenir des données de séquençage, les données de séquençage ayant été précédemment obtenues par séquençage de l'échantillon biologique provenant du sujet ; et

traiter les données de séquençage pour obtenir les données sur les segments du génome du sujet.

11. Procédé selon la revendication 10, dans lequel l'obtention des données de séquençage comprend l'obtention de données de séquençage pour au moins un chromosome du génome du sujet, ou pour le génome entier du sujet.

12. Procédé selon l'une quelconque des revendications précédentes, dans lequel le sujet :

(i) présente, est suspecté de présenter ou risque de présenter un cancer du sein, un cancer de l'ovaire, un cancer du pancréas, un cancer de la prostate, un cancer de la vessie, un cancer colorectal ou un lymphome ; ou
(ii) présente, est suspecté de présenter ou risque de présenter un cancer du sein.

13. Procédé pour identifier si un sujet est susceptible d'être sensible à un traitement par thérapie d'inhibiteur de polyADPribose polymérase (PARPi) ou une chimiothérapie à base de platine, le procédé comprenant les étapes consistant à :

déterminer si un échantillon biologique obtenu auprès du sujet inclut des cellules présentant une HRD en utilisant le procédé selon l'une quelconque des revendications précédentes ; et
lorsqu'il est déterminé que l'échantillon biologique inclut des cellules présentant une HRD,
déterminer que le sujet est susceptible d'être sensible au traitement avec la thérapie PARPi ou la chimiothérapie à base de platine.

14. Système, comprenant :

au moins un processeur matériel informatique ; et
au moins un support de stockage non transitoire lisible par ordinateur stockant des instructions, qui, lorsqu'elles sont exécutées par le au moins un processeur informatique, amènent les un ou plusieurs processeurs informatiques à exécuter un procédé selon l'une quelconque des revendications.

15. Au moins un support de stockage non transitoire lisible par ordinateur stockant des instructions exécutables par processeur qui, lorsqu'elles sont exécutées par au moins un processeur informatique, amènent le au moins un processeur informatique à exécuter un procédé selon l'une quelconque des revendications 1 à 13.

100

104

Biological Sample

Sequencing Data 106

108

Computing Device(s)

Output 110

110-1 Homologous Recombination Deficiency (HRD) Status

Therapy 110-2

102 Subject

FIG. 1A

FIG. 1B

FIG. 2

300

START

Obtain Data About Segments Of A Subject's Genome, The Data Including, For Each Of At Least Some Of The Segments, A Respective Copy Number And A Respective Length — 302

Identify A First Subset Of The Segments, The First Subset Including Segments Associated With At Least One Chromosome Arm Of The Genome, Each Of The Segments In The First Subset Having A First Copy Number — 304

Identify A Second Subset Of The Segments, Each Of The Segments In The Second Subset Having (i) A Respective Copy Number Different From The First Copy Number And (ii) A Respective Length That Satisfies A Respective Length Criterion — 306

Determine A Proportion Of A Number Of Segments In The Second Subset To A Number Of Chromosome Arms Of The At Least One Chromosome Arm Associated With The Segments In The First Subset — 308

Determine, Based On The Determined Proportion, Whether The Biological Sample Includes Cells Having Homologous Recombination Deficiency (HRD) — 310

Identify A Therapy For The Subject Based On The Output Indicating Whether The Biological Sample Includes Cells Having HRD — 312

Administer The Therapy To The Subject — 314

END

FIG. 3A

340

START

Obtain Segment Data For A First Segment ⎯ 342

344

Length Of First Segment ≥ First Threshold Length? —— No

Yes  346

Copy Number = Ploidy? —— No

Yes

348

First Segment One Of Group Of Segments Including ≤ Threshold Number Of Segments ? —— No

Yes

350

Yes —— Summary Length ≥ Second Threshold Length?

Include First Segment In First Subset Of Segments ⎯ 352    No

Yes —— Another Segment? ⎯ 354

No

END

# FIG. 3B

FIG. 3C

FIG. 4

FIG. 5

FIG. 6A

FIG. 6B

FIG. 7A

FIG. 7B

FIG. 7C

FIG. 8

FIG. 9A

FIG. 9B

FIG. 10A

FIG. 10B

FIG. 11A

FIG. 11B

FIG. 12

FIG. 13

FIG. 14

FIG. 15A

FIG. 15B

FIG. 16A

FIG. 16B

EP 4 555 107 B1

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63389640
- WO 2016135478 A1
- WO 0053211 A
- US 5981568 A
- WO 9007936 A
- WO 9403622 A
- WO 9325698 A
- WO 9325234 A
- WO 9311230 A
- WO 9310218 A
- WO 9102805 A
- US 5219740 A
- US 4777127 A
- GB 2200651 A
- EP 0345242 A
- WO 9412649 A
- WO 9303769 A
- WO 9319191 A
- WO 9428938 A
- WO 9511984 A
- WO 9500655 A
- US 5814482 A
- WO 9507994 A
- WO 9617072 A
- WO 9530763 A
- WO 9742338 A
- WO 9011092 A
- US 5580859 A
- US 5422120 A
- WO 9513796 A
- WO 9423697 A
- WO 9114445 A
- EP 0524968 A

### Non-patent literature cited in the description

- **WANG et al.** Copy number signature analysis tool and its application in prostate cancer reveals distinct mutational processes and clinical outcomes. *PLOS GENETICS*, 04 May 2021, vol. 17 (5)
- **KENDALL, J ; KRASNITZ, A**. Computational methods for DNA copy-number analysis of tumors. *Cancer Genomics and Proteomics: Methods and Protocols*, 2014, 243-259
- **FAVERO F et al.** Sequenza: allele-specific copy number and mutation profiles from tumor sequencing data. *Annals of Oncology*, 2015, vol. 26 (1), 64-70
- **CARTER, S et al.** Absolute quantification of somatic DNA alterations in human cancer. *Nat Biotechnol*, 2012, vol. 30 (5), 413-421
- **BAO, L**. AbsCN-seq: a statistical method to estimate tumor purity, ploidy and absolute copy numbers from next-generation sequencing data. *Bioinformatics*, 2014, vol. 30 (8), 1056-1063
- **VAN DER AUWERA GA ; O'CONNOR BD**. Genomics in the Cloud: Using Docker, GATK. O'Reilly Media, 2020
- **VAUGHT et al.** *Cancer Epidemiol Biomarkers Prev*, February 2012, vol. 21 (2), 253-5
- **VAUGHT ; HENDERSON**. *Biological sample collection, processing, storage and information management*, 2011, vol. 163, 23-42
- **LI et al.** *JCO Precis Oncol*, 2018, vol. 2
- **FINDEIS et al.** *Trends Biotechnol*, 1993, vol. 11, 202
- **CHIOU et al.** Gene Therapeutics: Methods and Applications of Direct Gene Transfe. 1994
- **WU et al.** *J. Biol. Chem.*, 1988, vol. 263, 621
- **WU et al.** *J. Biol. Chem*, 1994, vol. 269, 542
- **ZENKE et al.** *Proc. Natl. Acad. Sci. USA*, 1990, vol. 87, 3655
- **WU et al.** *J. Biol. Chem.*, 1991, vol. 266, 338
- **JOLLY**. *Cancer Gene Therapy*, 1994, vol. 1, 51
- **KIMURA**. *Human Gene Therapy*, 1994, vol. 5, 845
- **CONNELLY**. *Human Gene Therapy*, 1995, vol. 1, 185
- **KAPLITT**. *Nature Genetics*, 1994, vol. 6, 148
- Administration of DNA linked to killed adenovirus as described in Curiel. *Hum. Gene Ther*, 1992, vol. 3, 147
- *Curiel, Hum. Gene Ther*, 1992, vol. 3, 147
- **WU**. *J. Biol. Chem.*, 1989, vol. 264, 16985
- *Philip, Mol. Cell. Biol*, 1994, vol. 14, 2411
- **WOFFENDIN**. *Proc. Natl. Acad. Sci.*, 1994, vol. 91, 1581